# EUROPÄISCHE PATENTANMELDUNG

(11) **EP 1 939 185 A1**
(43) Veröffentlichungstag der Anmeldung: **02.07.2008**
(21) Anmeldenummer: 06077236.5
(22) Anmeldetag: 20.12.2006
(51) Int. Cl.: C07D 239/47, C07D 239/48, C07D 401/12, C07D 403/12, C07D 407/12, C07D 417/12, A61K 31/506, A61P 35/00

(54) **Neuartige Hetaryl-Phenylendiamin-Pyrimidine als Proteinkinaseinhibitoren zur Behandlung von Krebs**

(71) Anmelder: Bayer Schering Pharma Aktiengesellschaft, 13353 Berlin (DE)
(72) Erfinder: Jautelat, Rolf, 16548 Glienicke/Nordbahn (DE); Lücking, Ulrich, 10245 Berlin (DE); Siemeister, Gerhard, 13503 Berlin (DE)

(57) **Zusammenfassung**

Die Erfindung betrifft neuartige Hetaryl-Phenylendiamin-Pyrimidine sowie deren strukturell verwandte Sauerstoff- und Schwefelanaloga der allgemeinen Formel I, Verfahren zu deren Herstellung, sowie deren Verwendung als Arzneimittel in der Behandlung von Krebs.

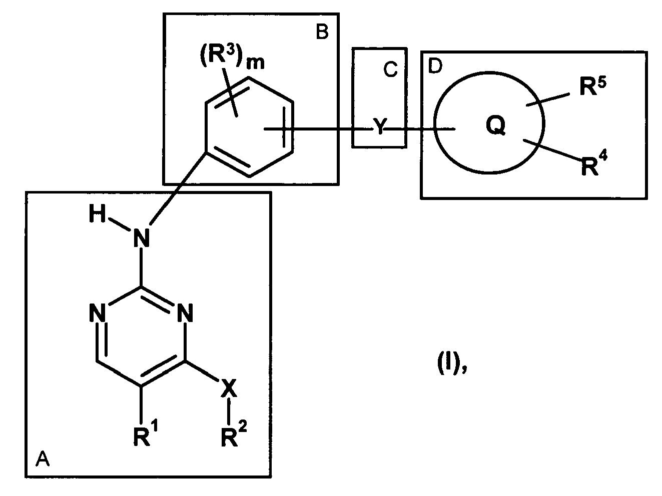

## Beschreibung

Die Erfindung betrifft neuartige Hetaryl-Phenylendiamin-Pyrimidine sowie deren strukturell verwandte Sauerstoff- und Schwefelanaloga als Proteinkinaseinhibitoren.

In eukaryontischen Zellen werden viele biologische Prozesse wie z.B. DNA-Replikation, Energiestoffwechsel, Zellwachstum oder -differenzierung durch reversible Phosphorylierung von Proteinen reguliert. Der Phosphorylierungsgrad eines Proteins hat unter anderem Einfluss auf die Funktion, Lokalisation oder Stabilität von Proteinen. Die Enzymfamilien der Proteinkinasen und Proteinphosphatasen sind verantwortlich für die Phosphorylierung bzw. Dephosphorylierung von Proteinen.

Über eine Inhibition von speziellen Proteinkinasen oder Proteinphosphatasen erhofft man sich in biologische Prozesse so eingreifen zu können, dass Krankheiten des menschlichen oder tierischen Körpers ursächlich oder symptomatisch behandelt werden können.

Im besonderen Interesse sind dabei Proteinkinasen, deren Inhibition die Behandlung von Krebs ermöglicht.

Folgende Proteinkinasefamilien kommen beispielsweise als Targets für inhibitorische Moleküle in Frage:
a) Zellzykluskinasen d.h. Kinasen, deren Aktivität den Ablauf des Zellteilungszyklus steuern. Zu den Zellzykluskinasen gehören im wesentlichen die Zyklin-abhängigen Kinasen (cyclin-dependent kinase: cdk), die polo-like Kinasen (Plk), und die Aurora Kinasen.
b) Rezeptortyrosinkinasen, die die Angiogenese regulieren (angiogene Rezeptortyrosinkinasen), wie beispielsweise die Rezeptortytrosinkinasen, die am Vascular Endothelial Growth Factor (VEGF) / VEGF-Rezeptor System, Fibroblast Growth Factor (FGF) / FGF-Rezeptor System, am Eph-Ligand / EphB4 System, und am Tie-Ligand / Tie System beteiligt sind,
c) Rezeptortyrosinkinasen, deren Aktivität zur Proliferation von Zellen beiträgt (proliferative Rezeptortyrosinkinasen), wie beispielsweise Rezeptortyrosinkinasen, die am Platelet-Derived Growth Factor (PDGF) Ligand / PDGF-Rezeptor System, Epithelial Growth Factor (EGF) Ligand / EGF-Rezeptor System, c-Kit Ligand / c-Kit-Rezeptor System und am FMS-like Tyrosinkinase 3 (Flt-3) Ligand / Flt-3 System beteiligt sind,
d) Kontrollpunktkinasen (Checkpoint-Kinasen), die den geordneten Ablauf der Zellteilung überwachen, wie beispielsweise ATM und ATR, Chk1 und Chk2, Mps1, Bub1 und BubR1,
e) Kinasen, deren Aktivität die Zelle vor Apoptose schützt (anti-apoptotische Kinasen, Kinasen in sog. survival pathways, anti-apoptotische Kinasen), wie beispielsweise Akt/PKB, PDK1, IkappaB kinase (IKK), Pim1, und integrin-linked kinase (ILK),
f) Kinasen, die für die Migration von Tumorzellen notwendig sind (migratorische Kinasen), wie beispielsweise focal adhesion kinase (FAK), und Rho kinase (ROCK).

Die Inhibierung einer oder mehrerer dieser Proteinkinasen eröffnet die Möglichkeit, das Tumorwachstum zu hemmen.

Dabei besteht vor allem ein Bedarf an Strukturen, die neben der Inhibierung von Zellzykluskinasen das Tumorwachstum durch die Inhibierung einer oder mehrerer weiterer Kinasen hemmen (Multi-target Tumor Growth Inhibitoren = MTGI). Bevorzugt ist vor allem die zusätzliche Inhibierung von Rezeptortyrosinkinasen, die die Angiogenese regulieren.

Den strukturell naheliegenden Stand der Technik bilden die Strukturen folgender Patentanmeldungen:
WO 2002/096888 offenbart Anilino-Pyrimidinderivate als Inhibitoren der Zyklin-abhängigen Kinasen. Hetaryl-Phenylendiamin-Pyrimidine werden nicht offenbart.
WO 2004/026881 offenbart makrozyklische Anilino-Pyrimidinderivate als Inhibitoren der Zyklin-abhängigen Kinasen Hetaryl-Phenylendiamin-Pyrimidine werden nicht offenbart.
WO 2005/037800 offenbart offene Anilino-Pyrimidinderivate als Inhibitoren der Zyklin-abhängigen Kinasen Hetaryl-Phenylendiamin-Pyrimidine werden nicht offenbart.
WO 2004/0046118 offenbart offene Diphenyl-Amino-Pyrimidine für die Behandlung von Krankheiten, die mit einer Hyperproliferation einhergehen. Hetaryl-Phenylendiamin-Pyrimidine werden nicht offenbart.

Allen diesen Strukturen des Standes der Technik ist gemeinsam, dass sie Zellzykluskinasen inhibieren.

Ausgehend von diesem Stand der Technik ist die Aufgabe der vorliegenden Erfindung, alternative Proteinkinaseinhibitoren bereitzustellen.

Im besonderen besteht die Aufgabe der vorliegenden Erfindung, Inhibitoren von Proteinkinasen bereitzustellen, über die ein Tumorwachstum gehemmt werden kann.

Vor allem besteht ein Bedarf an Verbindungen, die neben Zellzykluskinasen auch Rezeptortyrosinkinsen inhibieren, die die Angiogenese hemmen.

Vor allem besteht ein Bedarf an Verbindungen, die neben der Inhibierung von einer oder mehrerer Proteinkinasen tatsächlich die Proliferation von Krebszellen hemmt.

Die Aufgabe der vorliegenden Anmeldung wird gelöst durch Verbindungen der allgemeinen Formel (I) mit den Bausteinen A, B, C und D, 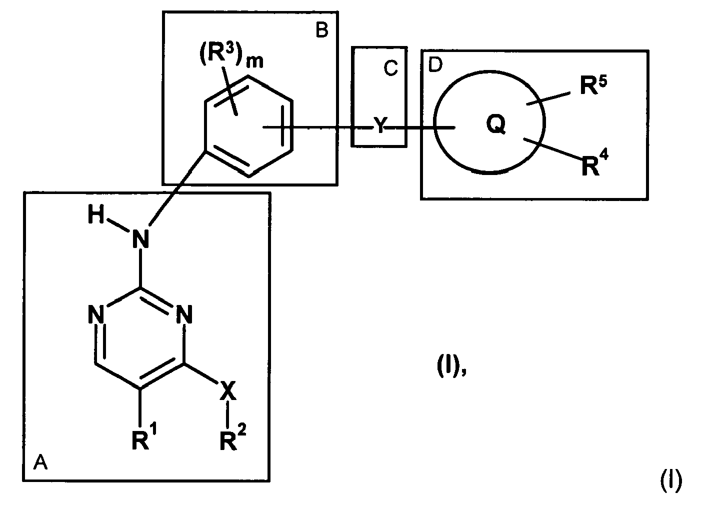 in der
- R¹: für
(i) Wasserstoff, Halogen, Cyano, Nitro, -NR⁸R⁹, -NR⁷-C(O)-R¹², -NR⁷-C(O)-OR¹², -NR⁷-C(O)-NR⁸R⁹, -NR⁷-SO₂-, -CF₃ oder -OCF₃, oder
(ii) einen gegebenenfalls mit Hydroxy, -NR⁸R⁹, -NR⁷-C(O)-R¹², -NR⁷-C(O)-OR¹², -NR⁷-C(O)-NR⁸R⁹, -NR⁷-SO₂-R¹², Cyano, Halogen, C₁-C₆-Alkoxy, -CF₃ und/oder -OCF₃ ein- oder mehrfach, gleich oder verschieden substituierten C₁-C₆-Alkyl-, C₂-C₆-Alkenyl-, C₁-C₆-Alkoxy- oder C₂-C₆-Alkinylrest, oder
(iii) einen gegebenenfalls mit Hydroxy, -NR⁸R⁹, -NR⁷-C(O)-R¹²,
-NR⁷-C(O)-OR¹², -NR⁷-C(O)-NR⁸R⁹, -NR⁷-SO₂-R¹², Cyano, Halogen, -CF₃, C₁-C₆-Alkoxy, -OCF₃ und/oder C₁-C₆-Alkyl ein- oder mehrfach, gleich oder verschieden substituierten Phenyl- oder monocyclischen Heteroarylring steht,
- R²: für
(i) Wasserstoff oder
(ii) einen C₁-C₁₀-Alkyl-,-C₂-C₁₀-Alkenyl- oder C₂-C₁₀-Alkinylrest, einen C₃-C₇-Cycloalkyl- oder einen Heterocyclylring mit 3 bis 8 Ringatomen steht,
   jeweils gegebenenfalls ein- oder mehrfach, gleich oder verschieden substituiert mit
   a) Halogen, Hydroxy, -NR⁸R⁹, -NR⁷-C(O)-R¹², -NR⁷-C(O)-OR¹², -NR⁷-C(O)-NR⁸R⁹, -NR⁷-SO₂-R¹², Cyano, -C(O)R⁶,-O(CO)-R¹², -SO₂NR⁸R⁹, -SO₂-R¹² -S(O)(NR⁸)R¹², -(N)S(O)R¹³R¹⁴, -CF₃, -OCF₃, -N[(CO)-(C₁-C₆-Alkyl)]₂ und/oder
   b) C₁-C₆-Alkoxy, C₁-C₆-Alkyl, C₂-C₆-Alkenyl, C₂-C₆-Alkinyl, C₃-C₈-Cycloalkyl, Heterocyclyl mit 3 bis 8 Ringatomen und/oder einem monocyclischen oder bicyclischen Heteroaryl, jeweils gegebenenfalls selbst ein- oder mehrfach, gleich oder verschieden mit Halogen, Hydroxy, einem C₁-C₆-Alkyl, C₁-C₆-Alkoxy, -NR⁸R⁹, -C(O)OR¹⁶, -SO₂NR⁸R⁹, -CF₃ oder -OCF₃ substituiert,
- R³: für
(i) Hydroxy, Halogen, Cyano, Nitro, -CF₃, -OCF₃, -C(O)NR⁸R⁹, -C(S)NR⁸R⁹, -NR⁸R⁹, -NR⁷-C(O)-R¹², -NR⁷-C(O)-OR¹², -NR⁷-C(O)-NR⁸R⁹, -NR⁷-SO₂-R¹², und/oder
(ii) einen gegebenenfalls mit Halogen, Hydroxy, C₁-C₆-Alkoxy, -CF₃, -OCF₃ oder -NR⁸R⁹ ein- oder mehrfach, gleich oder verschieden substituierten C₁-C₆-Alkyl- und/oder C₁-C₆-Alkoxyrest, und/oder
(iii) einen gegebenenfalls mit Halogen, Hydroxy, C₁-C₆-Alkoxy, -CF₃, -OCF₃, -NR⁸R⁹ und/oder C₁-C₆-Alkyl ein- oder mehrfach, gleich oder verschieden substituierten C₃-C₇-Cycloalkylring
steht,
- m: für 0-3 steht,

- R⁴ und R⁵: unabhängig voneinander für
(i) Wasserstoff, -NR⁸R⁹, -OR⁸, Halogen, -(CO)-NR⁸R⁹ und/oder
(ii) einen C₁-C₆-Alkyl-, C₂-C₆-Alkenyl-, C₂-C₆-Alkinylrest, einen C₃-C₇-Cycloalkyl- oder Phenylring, einen Heterocyclylring mit 3 bis 8 Ringatomen oder einen monocyclischen Heteroarylring stehen, jeweils gegebenenfalls selbst mit Hydroxy, -NR⁸R⁹, Cyano, Halogen, -CF₃, C₁-C₆-Alkoxy, -OCF₃ und/oder C₁-C₆-Alkyl ein- oder mehrfach, gleich oder verschieden substituiert,
- X und Y: unabhängig voneinander für -O-, -S-, -S(O)-, -S(O)₂- oder -NR¹⁵- stehen,
wobei,
R¹⁵ für
(i) Wasserstoff oder
(ii) einen C₁-C₆-Alkylrest, C₃-C₈-Cycloalkyl- oder einen Heterocyclylring mit 3 bis 8 Ringatomen oder
(iii) -C(O)-(C₁-C₆)-Alkyl, -C(O)-Phenyl, oder -C(O)-Benzyl steht,
   und (ii) und (iii) gegebenenfalls mit Hydroxy, -NR¹⁰R¹¹-, Cyano, Halogen, -CF₃, C₁-C₆-Alkoxy und/oder -OCF₃ ein- oder mehrfach, gleich oder verschieden substituiert sind,
oder
wenn X für -NR¹⁵- steht, alternativ
-NR¹⁵- und R² gemeinsam einen 3 bis 8 gliedrigen Ring bilden, der gegebenenfalls zusätzlich zum Stickstoffatom ein oder mehrere weitere Heteroatome enthält, gegebenenfalls ein- oder mehrfach, gleich oder verschieden mit Hydroxy, C₁-C₆-Alkyl, C₁-C₆-Alkoxy, -C(O)R¹², -SO₂R¹², Halogen oder der Gruppe -NR⁸R⁹ substituiert ist, gegebenenfalls 1 bis 3 Doppelbindungen enthält und/oder gegebenenfalls durch eine oder mehrere -C(O)-Gruppen unterbrochen ist,
- Q: für einen monocyclischen oder bicyclischen Heteroarylring steht,
- R⁶: für
(i) Wasserstoff oder Hydroxy, oder
(ii) einen C₁-C₆-Alkyl-, C₃-C₆-Alkenyl-, C₃-C₆-Alkinyl- oder C₁-C₆-Alkoxyrest, einen C₃-C₇-Cycloalkyl- oder Phenylring, einen Heterocyclylring mit 3 bis 8 Ringatomen oder einen monocyclischen Heteroarylring steht, jeweils gegebenenfalls selbst mit Hydroxy, -NR⁸R⁹, Cyano, Halogen, -CF₃, C₁-C₆-Alkoxy und/oder -OCF₃ ein- oder mehrfach, gleich oder verschieden substituiert,
- R⁷: für Wasserstoff oder einen C₁-C₆-Alkylrest steht,
- R⁸ und R⁹: unabhängig voneinander für
(i) Wasserstoff und/oder
(ii) einen C₁-C₆-Alkylrest, C₂-C₆-Alkenyl-, C₃-C₈-Cycloalkyl- und/oder Phenylring, einen Heterocyclylring mit 3 bis 8 Ringatomen und/oder einen monocyclischen Heteroarylring, gegebenenfalls mit Hydroxy, -NR¹⁰R¹¹, -NR⁷-C(O)-R¹², Cyano, Halogen, -CF₃, C₁-C₆-Alkoxy und/oder -OCF₃ ein- oder mehrfach, gleich oder verschieden substituiert sind,
oder
- R⁸ und R⁹: gemeinsam mit dem Stickstoffatom einen 3- bis 8-gliedrigen Ring bilden, der gegebenenfalls zusätzlich zum Stickstoffatom 1 oder 2 weitere Heteroatome enthält und der mit Hydroxy, -NR¹⁰R¹¹, Cyano, Halogen, -CF₃, C₁-C₆-Alkoxy und/oder -OCF₃ ein- oder mehrfach, gleich oder verschieden substituiert sein kann,
- R¹⁰ und R¹¹: unabhängig voneinander für Wasserstoff oder einen C₁-C₆-Alkylrest stehen, der gegebenenfalls mit Hydroxy, Cyano, Halogen, -CF₃, C₁-C₆-Alkoxy und/oder -OCF₃ ein- oder mehrfach, gleich oder verschieden substituiert ist.
- R¹² , R¹³ , R¹⁴: unabhängig voneinander für einen C₁-C₆-Alkyl, C₂-C₆-Alkenyl- und/oder C₂-C₆-Alkinylrest, einen C₃-C₇-Cycloalkyl- oder Phenylring , einen Heterocyclylring mit 3 bis 8 Ringatomen oder einen monocyclischen Heteroarylring steht, gegebenenfalls jeweils selbst mit Hydroxy, Nitro, -NR⁸R⁹, Cyano, Halogen, -CF₃, C₁-C₆-Alkyl, C₁-C₆-Alkoxy und/oder -OCF₃ ein- oder mehrfach, gleich oder verschieden substituiert,
- R¹⁶: für
(i) Wasserstoff oder
(ii) einen C₁-C₆-Alkyl-, C₃-C₆-Alkenyl-, C₃-C₆-Alkinylrest, einen C₃-C₇-Cycloalkyl- oder Phenylring, einen Heterocyclylring mit 3 bis 8 Ringatomen oder einen monocyclischen Heteroarylring steht, jeweils gegebenenfalls selbst mit Hydroxy, -NR⁸R⁹, Cyano, Halogen, -CF₃, C₁-C₆-Alkoxy und/oder -OCF₃ ein- oder mehrfach, gleich oder verschieden substituiert,
sowie deren Salze, Diastereomere und Enantiomere.

Kein Dokument des Standes der Technik offenbart die erfindungsgemäßen Verbindungen oder legt diese nahe.

Der Erfindung liegen folgende Definitionen zu Grunde:

### Cₙ-Alkyl:

Monovalenter, geradkettiger oder verzweigter, gesättigter Kohlenwasserstoffrest mit n Kohlenstoffatomen.

### Ein C₁-C₆ Alkylrest umfasst unter anderem beispielsweise:

Methyl-, Ethyl-, Propyl-, Butyl-, Pentyl-,Hexyl-, iso-Propyl-, iso-Butyl-, sec-Butyl, *tert-*Butyl-, iso-Pentyl-, 2-Methylbutyl-, 1-Methylbutyl-, 1-Ethylpropyl-, 1,2-Dimethylpropyl, neo-Pentyl-, 1,1'-Dimethylpropyl-, 4-Methylpentyl-, 3-Methylpentyl-, 2-Methylpentyl-, 1-Methylpentyl-, 2-Ethylbutyl-, 1-Ethylbutyl-, 3,3-Dimethylbutyl-, 2,2-Dimethylbutyl-, 1,1-Dimethylbutyl-, 2,3-Dimethylbutyl-, 1,3-Dimethylbutyl- 1,2-Dimethylbutyl-.

Bevorzugt ist ein Methyl-, Ethyl-, Propyl- oder Isopropylrest.

### Cₙ-Alkenyl:

monovalenter, geradkettiger oder verzweigter Kohlenwasserstoffrest mit n Kohlenstoffatomen und mindestens einer Doppelbindung.

Ein C₂-C₁₀ Alkenylrest umfasst unter anderem beispielsweise: Vinyl-, Allyl-, (E)-2-Methylvinyl-, (Z)-2-Methylvinyl-, Homoallyl-, (E)-But-2-enyl-, (Z)-But-2-enyl-, (E)-But-1-enyl-, (Z)-But-1-enyl-, Pent-4-enyl-, (E)-Pent-3-enyl-, (Z)-Pent-3-enyl-, (E)-Pent-2-enyl-, (Z)-Pent-2-enyl-, (E)-Pent-1-enyl-, (Z)-Pent-1-enyl-, Hex-5-enyl-, (E)-Hex-4-enyl-, (Z)-Hex-4-enyl-, (E)-Hex-3-enyl-, (Z)-Hex-3-enyl-, (E)-Hex-2-enyl-, (Z)-Hex-2-enyl-, (E)-Hex-1-enyl-, (Z)-Hex-1-enyl-, Isopropenyl-, 2-Methylprop-2-enyl-, 1-Methylprop-2-enyl-, 2-Methylprop-1-enyl-, (E)-1-Methylprop-1-enyl-, (Z)-1-Methylprop-1-enyl-, 3-Methylbut-3-enyl-, 2-Methylbut-3-enyl-, 1-Methylbut-3-enyl-, 3-Methylbut-2-enyl-, (E)-2-Methylbut-2-enyl-, (Z)-2-Methylbut-2-enyl-, (E)-1-Methylbut-2-enyl-, (Z)-1-Methylbut-2-enyl-, (E)-3-Methylbut-1-enyl-, (Z)-3-Methylbut-1-enyl-, (E)-2-Methylbut-1-enyl-, (Z)-2-Methylbut-1-enyl-, (E)-1-Methylbut-1-enyl-, (Z)-1-Methylbut-1-enyl-, 1,1-Dimethylprop-2-enyl-, 1-Ethylprop-1-enyl-, 1-Propylvinyl-, 1-Isopropylvinyl-, 4-Methylpent-4-enyl-, 3-Methylpent-4-enyl-, 2-Methylpent-4-enyl-, 1-Methylpent-4-enyl-, 4-Methylpent-3-enyl-, (E)-3-Methylpent-3-enyl-, (Z)-3-Methylpent-3-enyl-, (E)-2-Methylpent-3-enyl-, (Z)-2-Methylpent-3-enyl-, (E)-1-Methylpent-3-enyl-, (Z)-1-Methylpent-3-enyl-, (E)-4-Methylpent-2-enyl-, (Z)-4-Methylpent-2-enyl-, (E)-3-Methylpent-2-enyl-, (Z)-3-Methylpent-2-enyl-, (E)-2-Methylpent-2-enyl-, (Z)-2-Methylpent-2-enyl-, (E)-1-Methylpent-2-enyl-, (Z)-1-Methylpent-2-enyl-, (E)-4-Methylpent-1-enyl-, (Z)-4-Methylpent-1-enyl-, (E)-3-Methylpent-1-enyl-, (Z)-3-Methylpent-1-enyl-, (E)-2-Methylpent-1-enyl-, (Z)-2-Methylpent-1-enyl-, (E)-1-Methylpent-1-enyl-, (Z)-1-Methylpent-1-enyl-, 3-Ethylbut-3-enyl-, 2-Ethylbut-3-enyl-, 1-Ethylbut-3-enyl-, (E)-3-Ethylbut-2-enyl-, (Z)-3-Ethylbut-2-enyl-, (E)-2-Ethylbut-2-enyl-, (Z)-2-Ethylbut-2-enyl-, (E)-1-Ethylbut-2-enyl-, (Z)-1-Ethylbut-2-enyl-, (E)-3-Ethylbut-1-enyl-, (Z)-3-Ethylbut-1-enyl-, 2-Ethylbut-1-enyl-, (E)-1-Ethylbut-1-enyl-, (Z)-1-Ethylbut-1-enyl-, 2-Propylprop-2-enyl-, 1-Propylprop-2-enyl-, 2-Isopropylprop-2-enyl-, 1-Isopropylprop-2-enyl-, (E)-2-Propylprop-1-enyl-, (Z)-2-Propylprop-1-enyl-, (E)-1-Propylprop-1-enyl-, (Z)-1-Propylprop-1-enyl-, (E)-2-Isopropylprop-1-enyl-, (Z)-2-Isopropylprop-1-enyl-, (E)-1-Isopropylprop-1-enyl-, (Z)-1-Isopropylprop-1-enyl-, (E)-3,3-Dimethylprop-1-enyl-, (Z)-3,3-Dimethylprop-1-enyl-, 1-(1,1-Dimethylethyl)ethenyl.

Bevorzugt ist ein Vinyl- oder Allylrest.

### Cn-AIkinVl:

Monovalenter, geradkettiger oder verzweigter Kohlenwasserstoffrest mit n Kohlenstoffatomen und mindestens einer Dreifachbindung.

Ein C₂-C₁₀ Alkinylrest umfasst unter anderem beispielsweise: Ethinyl-, Prop-1-inyl-, Prop-2-inyl-, But-1-inyl-, But-2-inyl-, But-3-inyl-, Pent-1-inyl-, Pent-2-inyl-, Pent-3-inyl-, Pent-4-inyl-, Hex-1-inyl-, Hex-2-inyl-, Hex-3-inyl-, Hex-4-inyl-, Hex-5-inyl-, 1-Methylprop-2-inyl-, 2-Methylbut-3-inyl-, 1-Methylbut-3-inyl-, 1-Methylbut-2-inyl-, 3-Methylbut-1-inyl-, 1-Ethylprop-2-inyl-, 3-Methylpent-4-inyl, 2-Methylpent-4-inyl, 1-Methylpent-4-inyl, 2-Methylpent-3-inyl, 1-Methylpent-3-inyl, 4-Methylpent-2-inyl, 1-Methylpent-2-inyl, 4-Methylpent-1-inyl, 3-Methylpent-1-inyl, 2-Ethylbut-3-inyl-, 1-Ethylbut-3-inyl-, 1-Ethylbut-2-inyl-, 1-Propylprop-2-inyl-, 1-lsopropylprop-2-inyl-, 2,2-Dimethylbut-3-inyl-, 1,1-Dimethylbut-3-inyl-, 1,1-Dimethylbut-2-inyl- oder eine 3,3-Dimethylbut-1-inyl-.

Bevorzugt ist ein Ethinyl-, Prop-1-inyl- oder Prop-2-inyl-rest.

### Cₙ-Cycloalkyl:

Monovalenter, cyclischer Kohlenwasserstoffring mit n Kohlenstoffatomen.

### C₃-C₇-Cycklolalkylring umfasst:

Cyclopropyl, Cyclobutyl, Cyclopentyl, Cyclohexyl- und Cycloheptyl.

Bevorzugt ist ein Cyclopropyl-, Cylopentyl- oder ein Cyclohexylring.

### Cₙ-Alkoxy:

Geradkettiger oder verzweigter Cₙ-Alkyletherrest der Formel -OR mit R=Alkyl.

### Cₙ-Aryl

Cₙ-Aryl ist ein monovalentes, aromatisches Ringsystem ohne Heteroatom mit n Kohlenwasserstoffatomen.
C₆-Aryl ist gleich Phenyl. C₁₀-Aryl ist gleich Naphthyl.

Bevorzugt ist Phenyl.

### Heteroatome

Unter Heteroatomen sind Sauerstoff-, Stickstoff- oder Schwefel-Atome zu verstehen.

### Heteroaryl

Heteroaryl ist ein monovalentes, aromatisches Ringsystem mit mindestens einem von einem Kohlenstoff verschiedenen Heteroatom. Als Heteroatome können Stickstoffatome, Sauerstoffatome und/oder Schwefelatome vorkommen. Die Bindungsvalenz kann an einem beliebigen aromatischen Kohlenstoffatom oder an einem Stickstoffatom sein. Ein Stickstoffatom als Heteroatom kann oxidiert als N-oxid vorliegen.

Ein monocyclischer Heteroarylring gemäß der vorliegenden Erfindung hat 5 oder 6 Ringatome.

Heteroarylringe mit 5 Ringatomen umfassen beispielsweise die Ringe: Thienyl, Thiazolyl, Furanyl, Pyrrolyl, Oxazolyl, Imidazolyl, Pyrazolyl, Isoxazolyl, Isothiazolyl, Oxadiazolyl, Triazolyl, Tetrazolyl- und Thiadiazolyl.

Heteroarylringe mit 6 Ringatomen umfassen beispielsweise die Ringe: Pyridyl, Pyridazinyl, Pyrimidinyl, Pyrazinyl und Triazinyl.

Ein bicyclischer Heteroarylring gemäß der vorliegenden Erfindung hat 9 oder 10 Ringatome.

Heteroarylringe mit 9 Ringatomen umfassen beispielsweise die Ringe: Phthalidyl-, Thiophthalidyl-, Indolyl-, Isoindolyl-, Indazolyl-, Benzothiazolyl-, Indolonyl-, Isoindolonyl-, Benzofuranyl, Benzothienyl, Benzimidazolyl, Benzoxazolyl, Azocinyl, Indolizinyl, Purinyl.

Heteroarylringe mit 10 Ringatomen umfassen beispielsweise die Ringe: lsoquinolinyl-, Quinolinyl-, Benzoxazinonyl-, Phthalazinonyl, Quinolonyl-, Isoquinolonyl-, Quinazolinyl-, Quinoxalinyl-, Cinnolinyl-, Phthalazinyl-, 1,7- or 1,8-Naphthyridinyl-, Quinolinyl-, Isoquinolinyl-, Quinazolinyl- oder Quinoxalinyl-

Monocyclische Heteroarylringe mit 5 oder 6 Ringatomen sind bevorzugt.

### Heterocyclyl

Heterocyclyl im Sinne der Erfindung ist ein vollständig hydriertes Heteroaryl (vollständig hydriertes Heteroaryl = gesättigtes Heterocyclyl) d.h. ein nicht aromatisches Ringsystem mit mindestens einem von einem Kohlenstoff verschiedenen Heteroatom. Als Heteroatome können Stickstoffatome, Sauerstoffatome und/oder Schwefelatome vorkommen. Die Bindungsvalenz kann an einem beliebigen Kohlenstoffatom oder an einem Stickstoffatom sein. Heterocycylring mit 3 Ringatomen umfasst beispielsweise:
Aziridinyl.
Heterocycylring mit 4 Ringatomen umfasst beispielsweise:
Azetidinyl, Oxetanyl.
Heterocycylringe mit 5 Ringatomen umfassen beispielsweise die Ringe:
Pyrrolidinyl, Imidazolidinyl , Pyrazolidinyl und Tetrahydrofuranyl.
Heterocyclylringe mit 6 Ringatomen umfassen beispielsweise die Ringe:
Piperidinyl, Piperazinyl, Morpholinyl, Tetrahydropyranyl und Thiomorpholinyl Heterocycylring mit 7 Ringatomen umfasst beispielsweise:
Azepanyl, Oxepanyl, [1,3]-Diazepanly, [1,4]-Diazepanyl.
Heterocycylring mit 8 Ringatomen umfasst beispielsweise:
Oxocanyl, Azocanyl

### Halogen

Die Bezeichnung Halogen umfasst Fluor, Chlor, Brom und lod. Bevorzugt ist Brom.

### Baustein A

In der allgemeinen Formel (I) kann R¹ stehen für:
(i) Wasserstoff, Halogen, Cyano, Nitro, -NR⁸R⁹, -NR⁷-C(O)-R¹², -NR⁷-C(O)-OR¹², -NR⁷-C(O)-NR⁸R⁹, -NR⁷-SO₂-R¹², -CF₃ oder -OCF₃, oder
(ii) einen gegebenenfalls mit Hydroxy, -NR⁸R⁹, -NR⁷-C(O)-R¹², -NR⁷-C(O)-OR¹², -NR⁷-C(O)-NR⁸R⁹, -NR⁷-SO₂-R¹², Cyano, Halogen, C₁-C₆-Alkoxy, -CF₃ und/oder -OCF₃ ein- oder mehrfach, gleich oder verschieden substituierten C₁-C₆-Alkyl-, C₂-C₆-Alkenyl-, C₁-C₆-Alkoxy- oder C₂-Cₑ-Alkinylrest, oder
(iii) einen gegebenenfalls mit Hydroxy, -NR⁸R⁹, -NR⁷-C(O)-R¹², -NR⁷-C(O)-OR¹², -NR⁷-C(O)-NR⁸R⁹, -NR⁷-SO₂-R¹², Cyano, Halogen, -CF₃, C₁-C₆-Alkoxy, -OCF₃ und/oder C₁-C₆-Alkyl ein- oder mehrfach, gleich oder verschieden substituierten Phenyl- oder monocyclischen Heteroarylring.

Bevorzugt steht R¹ für:
Halogen, -CF₃, -OCF₃, C₁-C₄-Alkyl oder Nitro.
Mehr bevorzugt steht R¹ für Halogen, -CF₃ oder C₁-C₂-Alkyl.
Noch mehr bevorzugt steht R¹ für Halogen oder -CF₃.Besonders bevorzugt steht R¹ für Halogen, insbesondere Brom.

In der allgemeinen Formel (I) kann R² stehen für:
(i) Wasserstoff oder
(ii) einen C₁-C₁₀-Alkyl-, C₂-C₁₀-Alkenyl- oder C₂-C₁₀-Alkinylrest, einen C₃-C₇-Cycloalkyl- oder einen Heterocyclylring mit 3 bis 8 Ringatomen, jeweils gegebenenfalls ein- oder mehrfach, gleich oder verschieden substituiert mit
   a) Halogen, Hydroxy, -NR⁸R⁹, -NR⁷-C(O)-R¹², -NR⁷-C(O)-OR¹², -NR⁷-C(O)-NR⁸R⁹, -NR⁷-SO₂-R¹², Cyano, -C(O)R⁶ -O(CO)-R¹², -SO₂NR⁸R⁹, -SO₂-R¹², -S(O)(NR⁸)R¹², -(N)S(O)R¹³R¹⁴, -CF₃, -OCF₃, -N[(CO)-(C₁-C₆-Alkyl)]₂ und/oder
   b) C₁-C₆-Alkoxy, C₁-C₆-Alkyl, C₂-C₆-Alkenyl, C₂-C₆-Alkinyl, C₃-C₈-Cycloalkyl, Heterocyclyl mit 3 bis 8 Ringatomen und/oder einem monocyclischen oder bicyclischen Heteroaryl, jeweils gegebenenfalls selbst ein- oder mehrfach, gleich oder verschieden mit Halogen, Hydroxy, einem C₁-C₆-Alkyl, C₁-C₆-Alkoxy, -NR⁸R⁹,
      -C(O)OR¹⁶, -SO₂NR⁸R⁹, -CF₃ oder -OCF₃ substituiert.

Bevorzugt steht R² für:
einen C₁-C₁₀-Alkyl-, C₂-C₁₀-Alkenyl- oder C₂-C₁₀-Alkinylrest, einen C₃-C₇-Cycloalkyl- oder einen Heterocyclylring mit 3 bis 7 Ringatomen, jeweils gegebenenfalls ein oder mehrfach, gleich oder verschieden substituiert mit Hydroxy, -NR⁸R⁹, -NR⁷-C(O)-R¹², -NR⁷-C(O)-OR¹², -NR⁷-C(O)-NR⁸R⁹ oder einem monocyclischen oder bicyclischen Heteroaryl, der gegebenenfalls selbst ein- oder mehrfach substituiert ist mit Hydroxy oder einem C₁-C₆-Alkylrest.

Mehr bevorzugt steht R² für:
einen C₁-C₈-Alkyl-, C₂-C₈-Alkenyl- oder C₂-C₈-Alkinylrest, einen C₃-C₆-Cycloalkyl- oder einen Heterocyclylring mit 3 bis 5 Ringatomen, jeweils gegebenenfalls ein oder mehrfach, gleich oder verschieden substituiert mit Hydroxy, -NR⁸R⁹, -NR⁷-C(O)-R¹², -NR⁷-C(O)-OR¹², -NR⁷-C(O)-NR⁸R⁹ oder einem monocyclischen Heteroaryl, der gegebenenfalls selbst ein- oder mehrfach substituiert ist mit Hydroxy oder einem C₁-C₅-Alkylrest.

Besonders bevorzugt steht R² für:
einen C₁-C₆-Alkyl-, gleich oder verschieden substituiert mit Hydroxy, -NR⁸R⁹, -NR⁷-C(O)-R¹² oder einem monocyclischen Heteroaryl, der gegebenenfalls selbst ein- oder mehrfach substituiert ist mit einem C₁-C₅-Alkylrest.

Ganz besonders bevorzugt steht R² für:
einen C₁-C₆-Alkyl-, gleich oder verschieden substituiert mit Hydroxy .

In der allgemeinen Formel (I) kann X stehen für:
-O-, -S-, -S(O)-, -S(O)₂- oder -NR¹⁵-.
wobei,
R¹⁵ steht für
(i) Wasserstoff oder
(ii) einen C₁-C₆-Alkylrest, C₃-C₈-Cycloalkyl- oder einen Heterocyclylring mit 3 bis 8 Ringatomen oder
(iii) -C(O)-(C₁-C₆)-Alkyl, -C(O)-Phenyl, oder -C(O)-Benzyl,
   wobei (ii) und (iii) gegebenenfalls mit Hydroxy, -NR¹⁰R¹¹, Cyano, Halogen, -CF₃, C₁-C₆-Alkoxy und/oder -OCF₃ ein- oder mehrfach, gleich oder verschieden substituiert sind,
oder
wenn X für -NR¹⁵- steht, alternativ
-NR¹⁵- und R² gemeinsam einen 3 bis 8 gliedrigen Ring bilden, der gegebenenfalls zusätzlich zum Stickstoffatom ein oder mehrere weitere Heteroatome enthält, gegebenenfalls ein- oder mehrfach, gleich oder verschieden mit Hydroxy, C₁-C₆-Alkyl, C₁-C₆-Alkoxy, -C(O)R¹², -SO₂R¹², Halogen oder der Gruppe -NR⁸R⁹ substituiert ist, gegebenenfalls 1 bis 3 Doppelbindungen enthält und/oder gegebenenfalls durch eine oder mehrere -C(O)-Gruppen unterbrochen ist.

Bevorzugt steht X für :
-O-, -S-, -S(O)- , -S(O)₂- oder -NR¹⁵-, wobei
R¹⁵ für Wasserstoff oder einen C₁-C₆-Alkylrest, C₃-C₈-Cycloalkyl- oder einen Heterocyclylring mit 3 bis 8 Ringatomen steht, jeweils gegebenenfalls ein- oder mehrfach, gleich oder verschieden substituiert mit Hydroxy, -NR¹⁰R¹¹, Cyano, Halogen, -CF₃, C₁-C₆-Alkoxy und/oder -OCF₃, -
oder
wenn X für -NR¹⁵- steht,
-NR¹⁵- und R² bevorzugt alternativ gemeinsam einen 3 bis 6 gliedrigen Ring bilden, der gegebenenfalls zusätzlich zum Stickstoffatom ein weiteres Heteroatom enthält, gegebenenfalls ein- oder mehrfach, gleich oder verschieden mit Hydroxy, C₁-C₆-Alkyl, C₁-C₆-Alkoxy, -C(O)R¹², -SO₂R¹², Halogen oder der Gruppe -NR⁸R⁹ substituiert ist, gegebenenfalls 1 oder 2 Doppelbindungen enthält und/oder durch eine -C(O)-Gruppe unterbrochen ist.

Mehr bevorzugt steht X für -O- oder -NR¹⁵-,
wobei,
R¹⁵ für Wasserstoff oder einen C₃-C₆-Alkylrest, C₃-C₇-Cycloalkyl- oder einen Heterocyclylring mit 3 bis 6 Ringatomen steht, jeweils gegebenenfalls ein- oder mehrfach, gleich oder verschieden substituiert mit Hydroxy, -NR¹⁰R¹¹, Cyano, Halogen, -CF₃, C₁-C₆-Alkoxy und/oder -OCF₃,
oder
wenn X für -NR¹⁵- steht,
-NR¹⁵- und R² mehr bevorzugt alternativ gemeinsam einen 5 oder 6 gliedrigen Ring, der gegebenenfalls zusätzlich zum Stickstoffatom ein weiteres Heteroatom enthält und der gegebenenfalls ein- oder mehrfach, gleich oder verschieden mit Hydroxy, C₁-C₆-Alkyl, C₁-C₆-Alkoxy, -C(O)R¹², -SO₂R¹², Halogen oder der Gruppe -NR⁸R⁹ substituiert ist.

Besonders bevorzugt steht X für -O- oder -NR¹⁵- , wobei R¹⁵ für Wasserstoff steht.

Ganz besonders bevorzugt steht X für -O-

### Baustein B

In der allgemeinen Formel (I) kann R³ stehen für:
(i) Hydroxy, Halogen, Cyano, Nitro, -CF₃, -OCF₃, -C(O)NR⁸R⁹, -C(S)NR⁸R⁹, - NR⁸R⁹, -NR⁷-C(O)-R¹², -NR⁷-C(O)-OR¹², -NR⁷-C(O)-NR⁸R⁹, -NR⁷-SO₂-R¹², und/oder
(ii) einen gegebenenfalls mit Halogen, Hydroxy, C₁-C₆-Alkoxy, -CF₃, -OCF₃ oder -NR⁸R⁹ ein- oder mehrfach, gleich oder verschieden substituierten C₁-C₆-Alkyl- und/oder C₁-C₆-Alkoxyrest, und/oder
(iii) einen gegebenenfalls mit Halogen, Hydroxy, C₁-C₆-Alkoxy, -CF₃, -OCF₃, -NR⁸R⁹ und/oder C₁-C₆-Alkyl ein- oder mehrfach, gleich oder verschieden substituierten C₃-C₇-Cycloalkylring

Bevorzugt steht R³ für:
(i) Hydroxy, Halogen, Cyano, Nitro, -CF₃, -OCF₃, -C(O)NR⁸R⁹, -C(S)NR⁸R⁹, -NR⁸R⁹, -NR⁷-C(O)-R¹², -NR⁷-C(O)-OR¹², -NR⁷-C(O)-NR⁸R⁹, -NR⁷-SO₂-R¹², und/oder
(ii) einen gegebenenfalls mit Halogen, Hydroxy, C₁-C₆-Alkoxy, -CF₃, -OCF₃ oder -NR⁸R⁹ ein- oder mehrfach, gleich oder verschieden substituierten C₁-C₅-Alkyl- und/oder C₁-C₅-Alkoxyrest,

Mehr bevorzugt steht R³ für
(i) Hydroxy, Halogen, Cyano, Nitro, -CF₃, -OCF₃, -NR⁸R⁹, -NR⁷-C(O)-R¹², -NR⁷-C(O)-OR¹², -NR⁷-C(O)-NR⁸R⁹, -NR⁷-SO₂-R¹², und/oder
(ii) einen gegebenenfalls mit Halogen, Hydroxy, C₁-C₆-Alkoxy, -CF₃, -OCF₃ oder -NR⁸R⁹ ein- oder mehrfach, gleich oder verschieden substituierten C₁-C₃-Alkyl- und/oder C₁-C₃-Alkoxyrest

Noch mehr bevorzugt steht R³ für:
(i) Hydroxy, Halogen, Cyano, Nitro, -CF₃, -OCF₃,-NR⁸R⁹ und/oder
(ii) einen C₁-C₃-Alkyl- und/oder C₁-C₃-Alkoxyrest

Besonders bevorzugt steht R³ für:
Halogen, für einen C₁-C₃-Alkyl- und/oder C₁-C₃-Alkoxyrest und hier vor allem für Fluor, Chlor, Methyl und/oder Methoxy.

In der allgemeinen Formel (I) kann m stehen für:
0-3, bevorzugt für 0-2 , mehr bevorzugt für 0 oder 1.

### Baustein C

In der allgemeinen Formel (I) kann Y stehen für:
-O-, -S-, -S(O)- , -S(O)₂- oder -NR¹⁵-,
wobei,
R¹⁵ steht für
(i) Wasserstoff oder
(ii) einen C₁-C₆-Alkylrest, C₃-C₈-Cycloalkyl- oder einen Heterocyclylring mit 3 bis 8 Ringatomen, oder
(iii) -C(O)-(C₁-C₆)-Alkyl, -C(O)-Phenyl, oder -C(O)-Benzyl,
wobei (ii) und (iii) gegebenenfalls mit Hydroxy, -NR¹⁰R¹¹, Cyano, Halogen, -CF₃, C₁-C₆-Alkoxy und/oder -OCF₃ ein- oder mehrfach, gleich oder verschieden substituiert sind,

Bevorzugt steht Y für :
-O-, -S- oder -NR¹⁵-, wobei
R¹⁵ für Wasserstoff oder einen C₁-C₆-Alkylrest, C₃-C₈-Cycloalkyl- oder einen Heterocyclylring mit 3 bis 8 Ringatomen steht, jeweils gegebenenfalls ein- oder mehrfach, gleich oder verschieden substituiert mit Hydroxy, -NR¹⁰R¹¹, Cyano, Halogen, -CF₃, C₁-C₆-Alkoxy und/oder -OCF₃,

Mehr bevorzugt steht Y für -O- oder -NR¹⁵-,
wobei,
R¹⁵ für Wasserstoff oder einen C₁-C₃-Alkylrest, C₃-C₇-Cycloalkyl- oder einen Heterocyclylring mit 3 bis 6 Ringatomen steht, jeweils gegebenenfalls ein- oder mehrfach, gleich oder verschieden substituiert mit Hydroxy, -NR¹⁰R¹¹, Cyano, Halogen, -CF₃, C₁-C₆-Alkoxy und/oder -OCF₃,

Besonders bevorzugt steht Y für -O- oder -NR¹⁵- , wobei R¹⁵ für Wasserstoff oder einen C₁-C₃-Alkylrest steht..

Ganz besonders bevorzugt steht Y für -NR¹⁵- , wobei R¹⁵ für Wasserstoff oder einen C₁-C₃-Alkylrest steht.

### Baustein D

In der allgemeinen Formel (I) kann Q stehen für:
für einen monocyclischen oder bicyclischen Heteroarylring.
Bevorzugt steht Q für einen monocyclischen Heteroarylring.

Mehr bevorzugt steht Q für einen monocyclischen Heteroarylring mit 6 Ringatomen.

Wenn Q für einen monocyclischen Heteroarylring mit 6 Ringatomen steht, ist ein Pyrimidinyl-, Pyridyl oder Pyridyl-N-oxidring bevorzugt.
Wenn Q für einen monocyclischen Heteroarylring mit 5 Ringatomen steht, ist ein Tetrazolyl- oder Triazolylring bevorzugt.
Wenn Q für einen bicyclischen Heteroarylring steht, ist ein Indolyl- oder Benzothiazolylring bevorzugt..

In der allgemeinen Formel (I) können R⁴ und R⁵ unabhängig voneinander stehen für:
(i) Wasserstoff, -NR⁸R⁹, -OR⁸ , Halogen, -(CO)-NR⁸R⁹ und/oder
(ii) einen C₁-C₆-Alkyl-, C₂-C₆-Alkenyl-, C₂-C₆-Alkinylrest, einen C₃-C₇-Cycloalkyl- oder Phenylring, einen Heterocyclylring mit 3 bis 8 Ringatomen oder einen monocyclischen Heteroarylring, jeweils gegebenenfalls selbst mit Hydroxy, -NR⁸R⁹, Cyano, Halogen, -CF₃, C₁-C₆-Alkoxy, -OCF₃ und/oder C₁-C₆-Alkyl ein- oder mehrfach, gleich oder verschieden substituiert

Bevorzugt stehen R⁴ und R⁵ unabhängig voneinander für:
(i) Wasserstoff, -NHR⁸, -OR⁸, Halogen, -(CO)-NR⁸R⁹ und/oder
(ii) einen C₁-C₄-Alkyl-, C₃-C₅-Alkenyl-, C₃-C₅-Alkinylrest oder einen C₃-C₆-Cycloalkylring, jeweils gegebenenfalls selbst mit Hydroxy, -NR⁸R⁹, Cyano, Halogen, -CF₃, C₁-C₆-Alkoxy, -OCF₃ und/oder C₁-C₆-Alkyl ein- oder mehrfach, gleich oder verschieden substituiert

Noch mehr bevorzugt stehen R⁴ und R⁵ unabhängig voneinanderfür:
Wasserstoff, einen C₁-C₃-Alkylrest, -NR⁸R⁹, -OR⁸, Halogen, wobei R⁸ und R⁹ unabhängig voneinander für Wasserstoff, einem monocyclischen Heteroarylring oder einen C₁-C₆-Alkylrest stehen, die gegebenenfalls ein- oder mehrfach, gleich oder verschieden substituiert sind mit Hydroxy, -NR¹⁰R¹¹ oder -NR⁷-C(O)-R¹².

R⁴ ist bevorzugt
(i) Wasserstoff oder
(ii) -NR⁸R⁹ oder-OR⁸, wobei R⁸ für einen C₁-C₆-Alkylrest steht, der einfach mit Hydroxy, einem -N(C₁-C₃)-Alkyl oder -NH-(CO)-(C₁-C₃)-Alkylrest substituiert ist und R⁹ Wasserstoff ist, oder
(iii) -NR⁸R⁹, wobei R⁸ und R⁹ für einen C₁-C₆-Alkylrest.

R⁴ ist besonders bevorzugt:
Wasserstoff, -NR⁸R⁹ oder-OR⁸, wobei R⁸ für einen C₁-C₆-Alkylrest steht, der einfach mit Hydroxy substituiert ist und R⁹ Wasserstoff ist.

R⁵ ist bevorzugt Wasserstoff, Halogen oder ein C₁-C₆-Alkylrest.

Bevorzugt ist weiterhin, wenn
(i) R⁴ und R⁵ beide für Wasserstoff stehen oder
(ii) R⁴ für -NR⁸R⁹ oder -OR⁸ steht, wobei R⁸ und R⁹ unabhängig voneinander für Wasserstoff, einen monocyclischen Heteroarylring oder einen C₁-C₆-Alkylrest stehen, die gegebenenfalls ein- oder mehrfach, gleich oder verschieden substituiert sind mit Hydroxy, -NR¹⁰R¹¹, -NR⁷-C(O)-R¹² und R⁵ für Wasserstoff, Halogen oder einen C₁-C₆-Alkylrest steht.

Mehr bevorzugt ist, wenn
(i) R⁴ und R⁵ beide für Wasserstoff stehen oder
(ii) R⁴ für-NHR⁸ oder -OR⁸ steht, wobei R⁸ für einen C₂-C₅-Alkylrest steht, der gegebenenfalls ein- oder mehrfach, gleich oder verschieden mit einem -N(C₁-C₃)-Alkyl- oder-NH-(CO)-(C₁-C₃)-Alkylrest und/oder mit Hydroxy substituiert ist und R⁵ für Wasserstoff, Halogen oder einen C₁-C₄-Alkylrest steht.

### Bausteinübergreifende Definitionen:

In der allgemeinen Formel (I) kann R⁶ stehen für:
(i) Wasserstoff oder Hydroxy, oder
(ii) einen C₁-C₆-Alkyl-, C₃-C₆-Alkenyl-, C₃-C₆-Alkinyl- oder C₁-C₆-Alkoxyrest, einen C₃-C₇-Cycloalkyl- oder Phenylring, einen Heterocyclylring mit 3 bis 8 Ringatomen oder einen monocyclischen Heteroarylring steht, jeweils gegebenenfalls selbst mit Hydroxy, -NR⁸R⁹, Cyano, Halogen, -CF₃, C₁-C₆-Alkoxy und/oder -OCF₃ ein- oder mehrfach, gleich oder verschieden substituiert,

Bevorzugt steht R⁶ für:
(i) Wasserstoff oder
(ii) einen C₁-C₄-Alkyl-, C₃-C₅-Alkenyl-, C₃-C₅-Alkinyl- oder C₁-C₅-Alkoxyrest, einen C₃-C₆-Cycloalkyl- oder Phenylring, einen Heterocyclylring mit 3 bis 6 Ringatomen oder einen monocyclischen Heteroarylring, jeweils gegebenenfalls selbst mit Hydroxy, -NR⁸R⁹, Cyano, Halogen, -CF₃, C₁-C₆-Alkoxy und/oder -OCF₃ ein- oder mehrfach, gleich oder verschieden substituiert.

Mehr bevorzugt steht R⁶ für: einen C₂-C₅-Alkyl-, C₄-C₆-Alkenyl-, C₄-C₆-Alkinyl- oder C₂-C₅-Alkoxyrest, einen C₄-C₆-Cycloalkyl- oder Phenylring, einen Heterocyclylring mit 3 bis 5 Ringatomen oder einen monocyclischen Heteroarylring, jeweils gegebenenfalls selbst mit Hydroxy, -NR⁸R⁹, Cyano, Halogen, -CF₃, C₁-C₆-Alkoxy und/oder -OCF₃ ein- oder mehrfach, gleich oder verschieden substituiert.

Besonders bevorzugt steht R⁶ für:
einen C₁-C₆-Alkyl-, einen C₁-C₆-Alkoxyrest oder einen C₃-C₇-Cycloalkylring, jeweils gegebenenfalls selbst mit Hydroxy, -NR⁸R⁹ und / oder C₁-C₆-Alkoxy ein- oder mehrfach, gleich oder verschieden substituiert.

In der allgemeinen Formel (I) kann R⁷ stehen für Wasserstoff oder einen C₁-C₆-Alkylrest.
Bevorzugt für R⁷ ist Wasserstoff.

In der allgemeinen Formel (I) können R⁸ und R⁹ unabhängig voneinander stehen für:
(i) Wasserstoff und/oder
(ii) einen C₁-C₆-Alkylrest, C₂-C₆-Alkenyl-, C₃-C₈-Cycloalkyl- und/oder Phenylring, einen Heterocyclylring mit 3 bis 8 Ringatomen und/oder einen monocyclischen Heteroarylring, jeweils gegebenenfalls mit Hydroxy, - NR¹⁰R¹¹, -NR⁷-C(O)-R¹², Cyano, Halogen, -CF₃, C₁-C₆-Alkoxy und/oder-OCF₃ ein- oder mehrfach, gleich oder verschieden substituiert,
oder
R⁸ und R⁹ bilden gemeinsam mit dem Stickstoffatom einen 3- bis 8-gliedrigen Ring, der gegebenenfalls zusätzlich zum Stickstoffatom 1 oder 2 weitere Heteroatome enthält und der mit Hydroxy, -NR¹⁰R¹¹, Cyano, Halogen, -CF₃, C₁-C₆-Alkoxy und/oder -OCF₃ ein- oder mehrfach, gleich oder verschieden substituiert sein kann

Bevorzugt stehen R⁸ und R⁹ für::
(i) Wasserstoff und/oder
(ii) einen C₁-C₅-Alkyl-, C₂-C₅-Alkenylrest, einen C₃-C₇-Cycloalkyl- und/oder Phenylring und/oder einen monocyclischen Heteroarylring, jeweils gegebenenfalls mit Hydroxy, -NR¹⁰R¹¹, -NR⁷-C(O)-R¹² und/oder C₁-C₆-Alkoxy ein- oder mehrfach, gleich oder verschieden substituiert,
   oder
   R⁸ und R⁹ bilden gemeinsam mit dem Stickstoffatom einen 5- bis 7-gliedrigen Ring, der gegebenenfalls zusätzlich zum Stickstoffatom 1 weiteres Heteroatom enthält und der mit Hydroxy, -NR¹⁰R¹¹ und/oder C₁-C₆-Alkoxy ein- oder mehrfach, gleich oder verschieden substituiert sein kann.

Mehr bevorzugt stehen R⁸ und R⁹ unabhängig voneinander für: Wasserstoff, einen monocyclischen Heteroarylring oder einen C₁-C₆-Alkylrest, die gegebenenfalls ein- oder mehrfach, gleich oder verschieden substituiert sind mit Hydroxy, -NR¹⁰R¹¹, - NR⁷-C(O)-R¹².

Besonders bevorzugt steht R⁸ für einen C₂-C₅-Alkylrest , der gegebenenfalls ein- oder mehrfach, gleich oder verschieden mit einem -N(C₁-C₃)-Alkyl- oder -NH-(CO)-(C₁-C₃)-Alkylrest und/oder mit Hydroxy substituiert ist.

In der allgemeinen Formel (I) können R¹⁰und R¹¹ unabhängig voneinander stehen für Wasserstoff oder einen C₁-C₆-Alkylrest, der gegebenenfalls mit Hydroxy, Cyano, Halogen, -CF₃, C₁-C₆-Alkoxy und/oder -OCF₃ ein- oder mehrfach, gleich oder verschieden substituiert ist.

Bevorzugt können R¹⁰und R¹¹ unabhängig voneinander stehen für Wasserstoff oder einen C₁-C₆-Alkylrest, der gegebenenfalls mit Hydroxy, Halogen oder C₁-C₆-Alkoxy ein- oder mehrfach, gleich oder verschieden substituiert ist.

Mehr bevorzugt können R¹⁰und R¹¹ unabhängig voneinander stehen für Wasserstoff oder einen C₁-C₄-Alkylrest, der gegebenenfalls mit Hydroxy ein- oder mehrfach, gleich oder verschieden substituiert ist.

Besonders bevorzugt können R¹⁰und R¹¹ unabhängig voneinander stehen für Wasserstoff oder eine Methylgruppe.

In der allgemeinen Formel (I) können R¹² , R¹³ , R¹⁴ unabhängig voneinander stehen für einen C₁-C₆-Alkyl, C₂-C₆-Alkenyl- und/oder C₂-C₆-Alkinylrest, einen C₃-C₇-Cycloalkyl- und/oder Phenylring , einen Heterocyclylring mit 3 bis 8 Ringatomen und/oder einen monocyclischen Heteroarylring stehen,
jeweils gegebenenfalls selbst mit Hydroxy, Nitro, -NR⁸R⁹, Cyano, Halogen, -CF₃, C₁-C₆-Alkyl, C₁-C₆-Alkoxy und/oder -OCF₃ ein- oder mehrfach, gleich oder verschieden substituiert,

Bevorzugt steht R¹² für einen C₁-C₆-Alkyl, C₂-C₆-Alkenyl- oder C₂-C₆-Alkinylrest, einen C₃-C₇-Cycloalkyl- oder Phenylring, einen Heterocyclylring mit 3 bis 8 Ringatomen oder einen monocyclischen Heteroarylring,
jeweils gegebenenfalls selbst mit Hydroxy, Halogen, Nitro, -NR⁸R⁹, C₁-C₆-Alkyl und/oder C₁-C₆-Alkoxy ein- oder mehrfach, gleich oder verschieden substituiert.

Mehr bevorzugt steht R¹² für einen C₁-C₅-Alkyl, C₂-C₅-Alkenyl-, einen C₃-C₆-Cycloalkyl- oder Phenylring, einen Heterocyclylring mit 3 bis 6 Ringatomen oder einen monocyclischen Heteroarylring,
jeweils gegebenenfalls selbst mit Hydroxy, Halogen, Nitro, -NR⁸R⁹, C₁-C₆-Alkyl und/oder C₁-C₆-Alkoxy ein- oder mehrfach, gleich oder verschieden substituiert.

Besonders bevorzugt steht R¹² für einen C₁-C₆-Alkylrest

Bevorzugt stehen R¹³ und R¹⁴ unabhängig voneinander stehen für einen C₁-C₆-Alkyl, C₂-C₆-Alkenyl- und/oder C₂-C₆-Alkinylrest, einen C₃-C₇-Cycloalkyl- und/oder Phenylring, einen Heterocyclylring mit 3 bis 8 Ringatomen und/oder einen monocyclischen Heteroarylring,
jeweils gegebenenfalls selbst mit Hydroxy,-NR⁸R⁹ und/oder C₁-C₆-Alkoxy ein- oder mehrfach, gleich oder verschieden substituiert.

Mehr bevorzugt stehen R¹³ und R¹⁴ unabhängig voneinander stehen für einen C₁-C₅-Alkyl, C₂-C₅-Alkenyl- und/oder C₂-C₅-Alkinylrest, einen C₃-C₆-Cycloalkyl- und/oder Phenylring, einen Heterocyclylring mit 3 bis 6 Ringatomen und/oder einen monocyclischen Heteroarylring.

Besonders bevorzugt stehen R¹³ und R¹⁴ unabhängig voneinander stehen für einen C₁-C₆-Alkylrest.

In der allgemeinen Formel (I) kann R¹⁶ stehen für:
(i) Wasserstoff oder
(ii) einen C₁-C₆-Alkyl-, C₃-C₆-Alkenyl-, C₃-C₆-Alkinylrest, einen C₃-C₇-Cycloalkyl- oder Phenylring, einen Heterocyclylring mit 3 bis 8 Ringatomen oder einen monocyclischen Heteroarylring, jeweils gegebenenfalls selbst mit Hydroxy, -NR⁸R⁹, Cyano, Halogen, -CF₃, C₁-C₆-Alkoxy und/oder -OCF₃ ein- oder mehrfach, gleich oder verschieden substituiert.

Bevorzugt kann R¹⁶ stehen für:
einen C₁-C₆-Alkyl-, C₃-C₆-Alkenyl-, C₃-C₆-Alkinylrest, einen C₃-C₇-Cycloalkyl- oder Phenylring, einen Heterocyclylring mit 3 bis 8 Ringatomen oder einen monocyclischen Heteroarylring, jeweils gegebenenfalls selbst mit Hydroxy, -NR⁸R⁹, Cyano, Halogen, -CF₃, C₁-C₆-Alkoxy und/oder -OCF₃ ein- oder mehrfach, gleich oder verschieden substituiert.

Mehr bevorzugt kann R¹⁶ stehen für:
einen C₁-C₆-Alkylrest, einen C₃-C₇-Cycloalkyl- oder Phenylring, einen Heterocyclylring mit 3 bis 8 Ringatomen oder einen monocyclischen Heteroarylring.

Besonders bevorzugt kann R¹⁶ stehen für einen C₁-C₆-Alkylrest.

Ebenfalls als von der vorliegenden Erfindung als erfasst anzusehen sind alle Verbindungen, die sich ergeben durch jede mögliche Kombination der oben genannten möglichen, bevorzugten und besonders bevorzugten Bedeutungen der Substituenten.

Besondere Ausführungsformen der Erfindung bestehen darüber hinaus in Verbindungen, die sich durch Kombination der direkt in den Beispielen offenbarten Bedeutungen für die Substituenten ergeben.

Eine bevorzugte Untergruppe sind Verbindungen der allgemeinen Formel (I) mit den Bausteinen A, B, C und D, in der
- R¹: für Halogen, -CF₃, -OCF₃, C₁-C₄-Alkyl oder Nitro steht,
- R²: für einen C₁-C₁₀-Alkyl-, C₂-C₁₀-Alkenyl- oder C₂-C₁₀-Alkinylrest, einen C₃-C₇-Cycloalkyl- oder einen Heterocyclylring mit 3 bis 7 Ringatomen steht, jeweils gegebenenfalls ein oder mehrfach, gleich oder verschieden substituiert mit Hydroxy, -NR⁸R⁹, -NR⁷-C(O)-R¹², -NR⁷-C(O)-OR¹², -NR⁷-C(O)-NR⁸R⁹ oder einem monocyclischen oder bicyclischen Heteroaryl, der gegebenenfalls selbst ein- oder mehrfach substituiert ist mit Hydroxy oder einem C₁-C₆-Alkylrest.
- R³: für
(i) Hydroxy, Halogen, Cyano, Nitro, -CF₃, -OCF₃, -C(O)NR⁸R⁹, -C(S)NR⁸R⁹, -NR⁸R⁹, -NR⁷-C(O)-R¹², -NR⁷-C(O)-OR¹², -NR⁷-C(O)-NR⁸R⁹ oder -NR⁷-SO₂-R¹² und/oder
(ii) einen gegebenenfalls mit Halogen, Hydroxy, C₁-C₆-Alkoxy, -CF₃, -OCF₃ oder -NR⁸R⁹ ein- oder mehrfach, gleich oder verschieden substituierten C₁-C₅-Alkyl- und/oder C₁-C₅-Alkoxyrest, steht
- m: für 0-3 steht,
- R⁴ und R⁵: unabhängig voneinander stehen für
(i) Wasserstoff, -NHR⁸, -OR⁸, Halogen, -(CO)-NR⁸R⁹ und/oder
(ii) einen C₁-C₄-Alkyl-, C₃-C₅-Alkenyl-, C₃-C₅-Alkinylrest oder einen C₃-C₆-Cycloalkylring, jeweils gegebenenfalls selbst mit Hydroxy, -NR⁸R⁹, Cyano, Halogen, -CF₃, C₁-C₆-Alkoxy, -OCF₃ und/oder C₁-C₆-Alkyl ein- oder mehrfach, gleich oder verschieden substituiert
- X und Y: unabhängig voneinander stehen für
-O-, -S-, -S(O)- oder -NR¹⁵-, wobei
R¹⁵ für Wasserstoff oder einen C₁-C₆-Alkylrest, C₃-C₈-Cycloalkyl- oder einen Heterocyclylring mit 3 bis 8 Ringatomen steht, jeweils gegebenenfalls ein- oder mehrfach, gleich oder verschieden substituiert mit Hydroxy, -NR¹⁰R¹¹, Cyano, Halogen, -CF₃, C₁-C₆-Alkoxy und/oder-OCF₃, oder
wenn X für -NR¹⁵- steht,
-NR¹⁵- und R² bevorzugt alternativ gemeinsam einen 3 bis 6 gliedrigen Ring bilden, der gegebenenfalls zusätzlich zum Stickstoffatom ein weiteres Heteroatom enthält, gegebenenfalls ein- oder mehrfach, gleich oder verschieden mit Hydroxy, C₁-C₆-Alkyl, C₁-C₆-Alkoxy, -C(O)R¹², -SO₂R¹², Halogen oder der Gruppe-NR⁸R⁹ substituiert ist, gegebenenfalls 1 oder 2 Doppelbindungen enthält und/oder durch eine -C(O)-Gruppe unterbrochen ist.
- Q: für einen monocyclischen oder bicyclischen Heteroarylring steht,
- R⁶: für
(i) Wasserstoff oder
(ii) einen C₁-C₄-Alkyl-, C₃-C₅-Alkenyl-, C₃-C₅-Alkinyl- oder C₁-C₅-Alkoxyrest, einen C₃-C₆-Cycloalkyl- oder Phenylring, einen Heterocyclylring mit 3 bis 6 Ringatomen oder einen monocyclischen Heteroarylring steht, jeweils gegebenenfalls selbst mit Hydroxy, -NR⁸R⁹, Cyano, Halogen, -CF₃, C₁-C₆-Alkoxy und/oder -OCF₃ ein- oder mehrfach, gleich oder verschieden substituiert.
- R⁷: für Wasserstoff steht,
- R⁸ und R⁹: unabhängig voneinander stehen für
Wasserstoff und/oder einen C₁-C₅-Alkyl-, C₂-C₅-Alkenylrest, einen C₃-C₇-Cycloalkyl- und/oder Phenylring und/oder einen monocyclischen Heteroarylring, jeweils gegebenenfalls mit Hydroxy,
-NR¹⁰R¹¹, -NR⁷-C(O)-R¹² und/oder C₁-C₆-Alkoxy ein- oder mehrfach, gleich oder verschieden substituiert,
oder
- R⁸ und R⁹: bilden gemeinsam mit dem Stickstoffatom einen 5- bis 7-gliedrigen Ring, der gegebenenfalls zusätzlich zum Stickstoffatom 1 weiteres Heteroatom enthält und der mit Hydroxy, -NR¹⁰R¹¹und/oder C₁-C₆-Alkoxy ein- oder mehrfach, gleich oder verschieden substituiert sein kann.
- R¹⁰ und R¹¹: unabhängig voneinander stehen für Wasserstoff oder einen C₁-C₆-Alkylrest, der gegebenenfalls mit Hydroxy, Halogen oder C₁-C₆-Alkoxy ein- oder mehrfach, gleich oder verschieden substituiert ist, .
- R¹²: für einen C₁-C₆-Alkyl, C₂-C₆-Alkenyl- oder C₂-C₆-Alkinylrest, einen C₃-C₇-Cycloalkyl- oder Phenylring, einen Heterocyclylring mit 3 bis 8 Ringatomen oder einen monocyclischen Heteroarylring steht jeweils gegebenenfalls selbst mit Hydroxy, Halogen, Nitro, -NR⁸R⁹, C₁-C₆-Alkyl und/oder C₁-C₆-Alkoxy ein- oder mehrfach, gleich oder verschieden substituiert.
- R¹³ und R¹⁴: unabhängig voneinander stehen für einen C₁-C₆-Alkyl, C₂-C₆-Alkenyl- und/oder C₂-C₆-Alkinylrest, einen C₃-C₇-Cycloalkyl- und/oder Phenylring, einen Heterocyclylring mit 3 bis 8 Ringatomen und/oder einen monocyclischen Heteroarylring, jeweils gegebenenfalls selbst mit Hydroxy,-NR⁸R⁹ und/oder C₁-C₆-Alkoxy ein- oder mehrfach, gleich oder verschieden substituiert.
- R¹⁶: für einen C₁-C₆-Alkyl-, C₃-C₆-Alkenyl-, C₃-C₆-Alkinylrest, einen C₃-C₇-Cycloalkyl- oder Phenylring, einen Heterocyclylring mit 3 bis 8 Ringatomen oder einen monocyclischen Heteroarylring steht, jeweils gegebenenfalls selbst mit Hydroxy, -NR⁸R⁹, Cyano, Halogen, -CF₃, C₁-C₆-Alkoxy und/oder -OCF₃ ein- oder mehrfach, gleich oder verschieden substituiert,
sowie deren Salze, Diastereomere und Enantiomere.

Eine mehr bevorzugte Untergruppe sind Verbindungen der allgemeinen Formel (I) mit den Bausteinen A, B, C und D,
in der
- R¹: für Halogen oder -CF₃ steht,
- R²: für einen C₁-C₁₀-Alkylrest steht, der gegebenenfalls ein- oder mehrfach, gleich oder verschieden substituiert ist mit, Hydroxy, -NR⁸R⁹, -NR⁷-C(O)-R¹² und/oder einem monocyclischen Heteroaryl, gegebenenfalls selbst ein- oder mehrfach, mit einem C₁-C₆-Alkyl, substituiert,
- m: für 0 steht,
- R⁴ und R⁵: unabhängig voneinander stehen für Wasserstoff, -NR⁸R⁹, -OR⁸, Halogen, -(CO)-NR⁸R⁹ und/oder einen C₁-C₆-Alkylrest,
- X und Y: unabhängig voneinander stehen für -O-, -S-, -S(O)- oder -NR¹⁵-, wobei R¹⁵ für Wasserstoff oder einen C₁-C₆-Alkylrest steht,
- Q: für einen monocyclischen oder bicyclischen Heteroarylring steht,
- R⁷: für Wasserstoff steht,
- R⁸ und R⁹: unabhängig voneinander für Wasserstoff , einen C₁-C₆-Alkylrest und/oder einen monocyclischen Heteroarylring stehen, die gegebenenfalls mit Hydroxy, -NR¹⁰R¹¹, -NR⁷-C(O)-R¹² substituiert sind,
- R¹⁰und R¹¹: unabhängig voneinander für Wasserstoff oder einen C₁-C₆-Alkylrest stehen,
- R¹²: für einen C₁-C₆-Alkylrest steht,
sowie deren Salze, Diastereomere und Enantiomere.

Eine noch mehr bevorzugte Untergruppe sind Verbindungen der allgemeinen Formel (I) mit den Bausteinen A, B, C und D,in der
- R¹: für Halogen oder -CF₃ steht,
- R²: für einen C₁-C₁₀-Alkylrest steht, der gegebenenfalls ein- oder mehrfach, gleich oder verschieden substituiert ist mit, Hydroxy, -NR⁸R⁹, -NR⁷-C(O)-R¹² und/oder einem monocyclischen Heteroaryl, gegebenenfalls selbst ein- oder mehrfach, mit einem C₁-C₆-Alkyl, substituiert,
- m: für 0 steht,
- R⁴ und R⁵: unabhängig voneinander stehen für Wasserstoff, -NR⁸R⁹, -OR⁸, Halogen, -(CO)-NR⁸R⁹ und/oder einen C₁-C₆-Alkylrest,
- X: steht für-O-, -S-, oder -NR¹⁵-,
wobei R¹⁵ für Wasserstoff oder einen C₁-C₆-Alkylrest steht,
- Y: steht für -NR¹⁵-,wobei R¹⁵ für Wasserstoff oder einen C₁-C₆-Alkylrest steht,
- Q: für einen monocyclischen oder bicyclischen Heteroarylring steht,
- R⁷: für Wasserstoff steht,
- R⁸ und R⁹: unabhängig voneinander für Wasserstoff, einen C₁-C₆-Alkylrest und/oder einen monocyclischen Heteroarylring stehen, die gegebenenfalls mit Hydroxy, -NR¹⁰R¹¹, -NR⁷-C(O)-R¹² substituiert sind,
- R¹⁰und R¹¹: unabhängig voneinander für Wasserstoff oder einen C₁-C₆-Alkylrest stehen,
- R¹²: für einen C₁-C₆-Alkylrest steht,
sowie deren Salze, Diastereomere und Enantiomere.

Eine ganz besonders bevorzugte Untergruppe sind Verbindungen der allgemeinen Formel (I) mit den Bausteinen A, B, C und D,in der
- R¹: für Halogen steht,
- R²: für einen C₁-C₁₀-Alkylrest steht, der gegebenenfalls ein- oder mehrfach, gleich oder verschieden substituiert ist mit, Hydroxy, -NR⁸R⁹, -NR⁷-C(O)-R¹² und/oder einem monocyclischen Heteroaryl, gegebenenfalls selbst ein- oder mehrfach, mit einem C₁-C₆-Alkyl, substituiert,
- m: für 0 steht,
- R⁴ und R⁵: unabhängig voneinander stehen für Wasserstoff, -NR⁸R⁹, -OR⁸, Halogen, -(CO)-NR⁸R⁹ und/oder einen C₁-C₆-Alkylrest,
- X: steht für -O- oder -NR¹⁵-,
wobei R¹⁵ für Wasserstoff oder einen C₁-C₆-Alkylrest steht,
- Y: steht für -NR¹⁵-,wobei R¹⁵ für Wasserstoff oder einen C₁-C₆-Alkylrest steht,
- Q: für einen monocyclischen oder bicyclischen Heteroarylring steht,
- R⁷: für Wasserstoff steht,
- R⁸ und R⁹: unabhängig voneinander für Wasserstoff , einen C₁-C₆-Alkylrest und/oder einen monocyclischen Heteroarylring stehen, die gegebenenfalls mit Hydroxy, -NR¹⁰R¹¹, -NR⁷-C(O)-R¹² substituiert sind,
- R¹⁰und R¹¹: unabhängig voneinander für Wasserstoff oder einen C₁-C₆-Alkylrest stehen,
- R¹²: für einen C₁-C₆-Alkylrest steht,
sowie deren Salze, Diastereomere und Enantiomere.

### A. Herstellung der Intermediate

Die in den folgenden Schemata verwendete Gruppe mit der Bezeichnung RL ist eine Fluchtgruppe. Als Fluchtgruppen geeignet sind:
-F, -Cl, -Br, -I, -OCF₃, -S-CH₃, -SOCH₃, -SO₂-CH₃, -O-SO₂-CF₃ (OTf) und andere Fluchtgruppen mit ähnlicher Reaktivität.
Die einzelnen Schemata führen die für diese Reaktion bevorzugten Fluchtgruppen auf.

### a) Herstellung der Intermediate der Formel (II) :

2,4-Dichlor-pyrimidine der Formel (X) können mit Nucleophilen der Formel (IX) zu Verbindungen der Formel (II) umgesetzt werden (siehe z.B.: a) U. Lücking et al, WO 2005037800; b) J. Bryant et al, WO 2004048343; c) U. Lücking et al, WO 2003076437; d) T. Brumby et al, WO 2002096888).

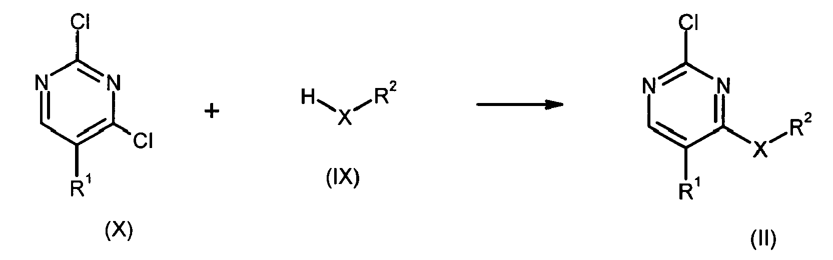

### Schema 1: Umsetzung von 2,4-Dichlorpyrimidinen mit Nucleophilen; Die Substituenten R¹, R² und X haben die in der allgemeinen Formel (I) angegebenen Bedeutungen.

### Intermediat 11.1:

### (2R,3R)-3-(5-Brom-2-chlor-pyrimidin-4-ylamino)-butan-2-ol

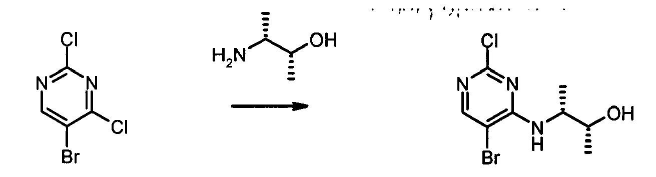

Herstellung nach: Lücking et al, WO 2005/037800, Seite 95.

In Analogie zu Intermediat 1 werden folgende Intermediate hergestellt:

| **Intermediat** | Struktur | Analytical Data | Herstellung |
|---|---|---|---|
| **II.2** | 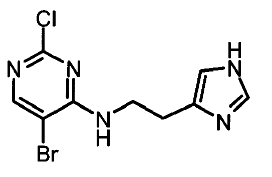 | | WO 2004/048343, Seite 72 |
| **II.3** | 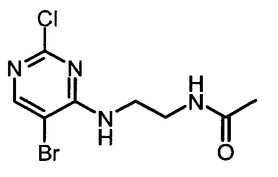 | | |

### Intermediat 11.4:

### (R)-3-(5-Brom-2-chlor-pyrimidin-4-ylamino)-2-methyl-butan-2-ol

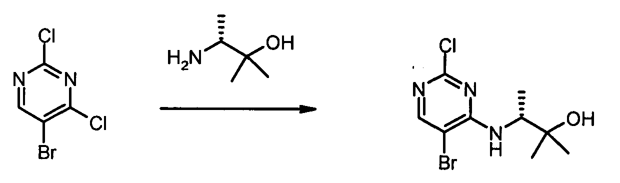

Herstellung nach: Lücking et al, WO 2005/037800, Seite 94.

### Intermediat 11.5:

### (2R,3R)-3-(2-Chloro-5-trifluoromethyl-pyrimidin-4-ylamino)-butan-2-ol

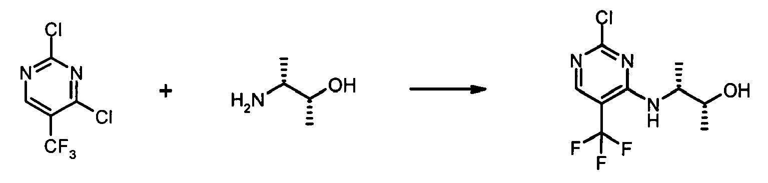

Ein Reaktionsgemisch aus 3,78 g (17,4 mmol) 2,4-Dichlor-5-trifluormethyl-pyrimidin (Frontier Scientific) und 2,19 g (17,4 mmol) (2R,3R)-3-Amino-butan-2-ol Hydrochlorid in 70 ml Acetonitril wird bei 0°C tropfenweise mit 4,83 ml (34,8 mmol) Triethylamin versetzt. Das Eisbad wird entfernt und der Ansatz wird 48 Stunden bei Raumtemperatur gerührt. Der Ansatz wird in halbkonzentrierte NaCl Lösung gegeben und mit Essigester extrahiert (2x). Die vereinten organischen Phasen werden getrocknet (Na₂SO₄), filtriert und eingeengt. Der erhaltene Rückstand wird mittels präperativer HPLC gereinigt. Man erhält 1,45 g (5,36 mmol; 31% Ausbeute) des Produktes.
- Säule:: X Bridge C18 5µ 100x30 mm
- Eluenten:: A:H2O B:Acetonitril
- Puffer:: A / 0,1 % TFA
- Gradient:: 60%A+40%B(2')_40->70%B(10')->99%B(0,5')
- Fluss:: 40,0 mL/min
- Detektion:: DAD (210-500 nm) TAC ; MS-ESI+ (125-800 m/z) TIC
- Temperatur:: Rt
- Retention:: 5,0-6,0 min
¹H-NMR (DMSO): 8.38 (s, 1 H), 6.72 (d, 1 H), 5.00 (d, 1 H), 4.09 (m, 1 H), 3.71 (m, 1 H), 1.12 (d, 3H), 1.01 (d, 3H).

### Intermediat II.6:

### 5-Bromo-2-chloro-4-methylsulfanyl-pyrimidine

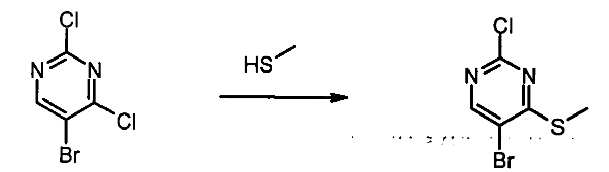

2 g Natriumthiomethylat (30 mmol) und 6.5 g of 5-Brom-2,4-dichloropyrimidin (28,5 mMol) wurden in 50 mL trockenem Acetonitril 24 h bei RT gerührt. Die Mischung wurde auf Eiswasser gegeben, 3x mit Dichlormethan extrahiert, mit Natriumsulfat getrocknet und eingeengt.. Das Produkt wurde aus Hexan umkristallisiert. Ausbeute 4 g (70%) 5-Bromo-2-chloro-4-methylsulfanyl-pyrimidine
MS (ES+) 241

### Intermediate 11.7:

### (2R,3R)-3-(5-Brom-2-chlor-pyrimidin-4-yloxy)-butan-2-ol

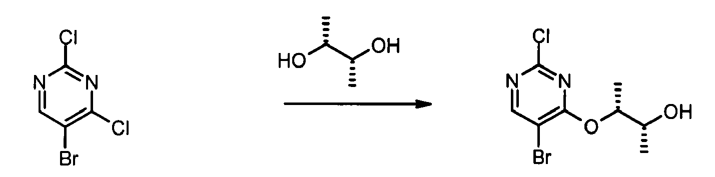

Herstellung nach: Lücking et al, WO 2005/037800, Seite 93.

### Intermediat 11.8

### 2-(2-Chloro-pyrimidin-4-ylamino)-ethanol

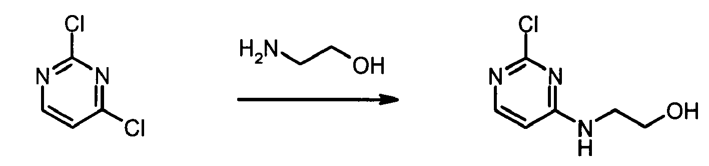

Zu einer Suspension von 1 g (6,7 mmol) 2,4-Dichlorpyrimidin in 15 ml Acetonitril/DMF 2:1 wurden unter Eisbadkühlung 0,93 ml (6,7 mmol) Triethylamin hinzufügt und eine Lösung von 0,4 ml (6,7 mmol) 2-Aminoethanol in 1 ml Acetonitril hinzugetropft. Danach wurden 12 h bei Raumtemperatur nachgerührt. Das Reaktionsgemisch wurde abfiltirert, gut mit Dichlormethan gewaschen und die Mutterlauge einengt. Nach Chromatographie (Hexan/Essigester 1:1-> DCM/MeOH 4:1) ergaben sich 148 mg (13% d.Th.) des Intermediates II.12
¹H-NMR (DMSO):7,92 (d, 1H), 7,83 (d, 1H), 6,44 (m, 1H), 4,76 (d, 1H), 3,46 (m, 2H), 3,31 (m, 2H)
MS: 174 (ES).

### b) Intermediate der Formel (III) :

Intermediate der Formel (III), insbesondere der Formel (IIIa), (IIIb), (IIIc) und (IIId) sind in großem Umfang kommerziell erhältlich oder können nach bekannten Methoden hergestellt werden.

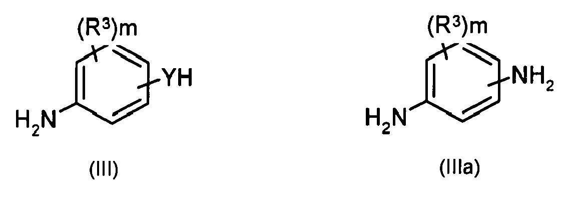

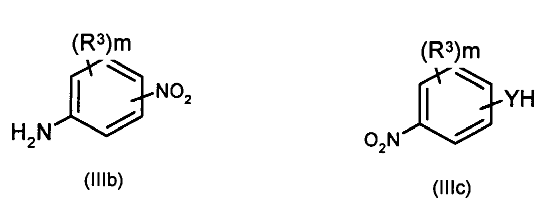

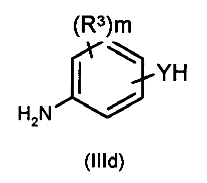

### c) Intermediate der Formel (V) :

Intermediate der Formel (V), insbesondere der Formel (Va), (Vb) und (Vc) sind in großem Umfang kommerziell erhältlich oder können nach verschiedenen bekannten Methoden hergestellt werden.

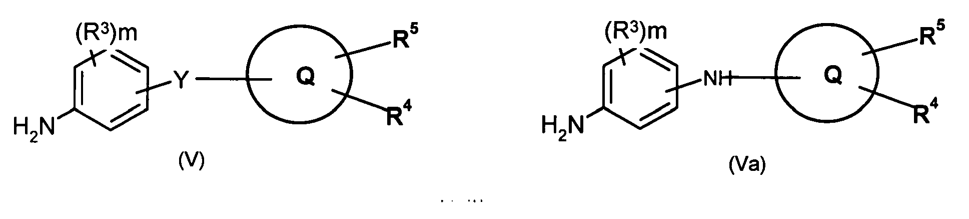

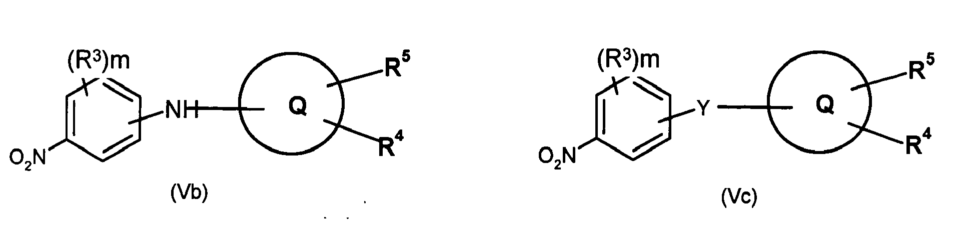

Die Substituenten Q, R³, R⁴, R⁵, Y und m haben die in der allgemeinen Formel (I) angegebenen Bedeutungen.

Folgende Intermediate sind beispielsweise kommerziell erhältlich:

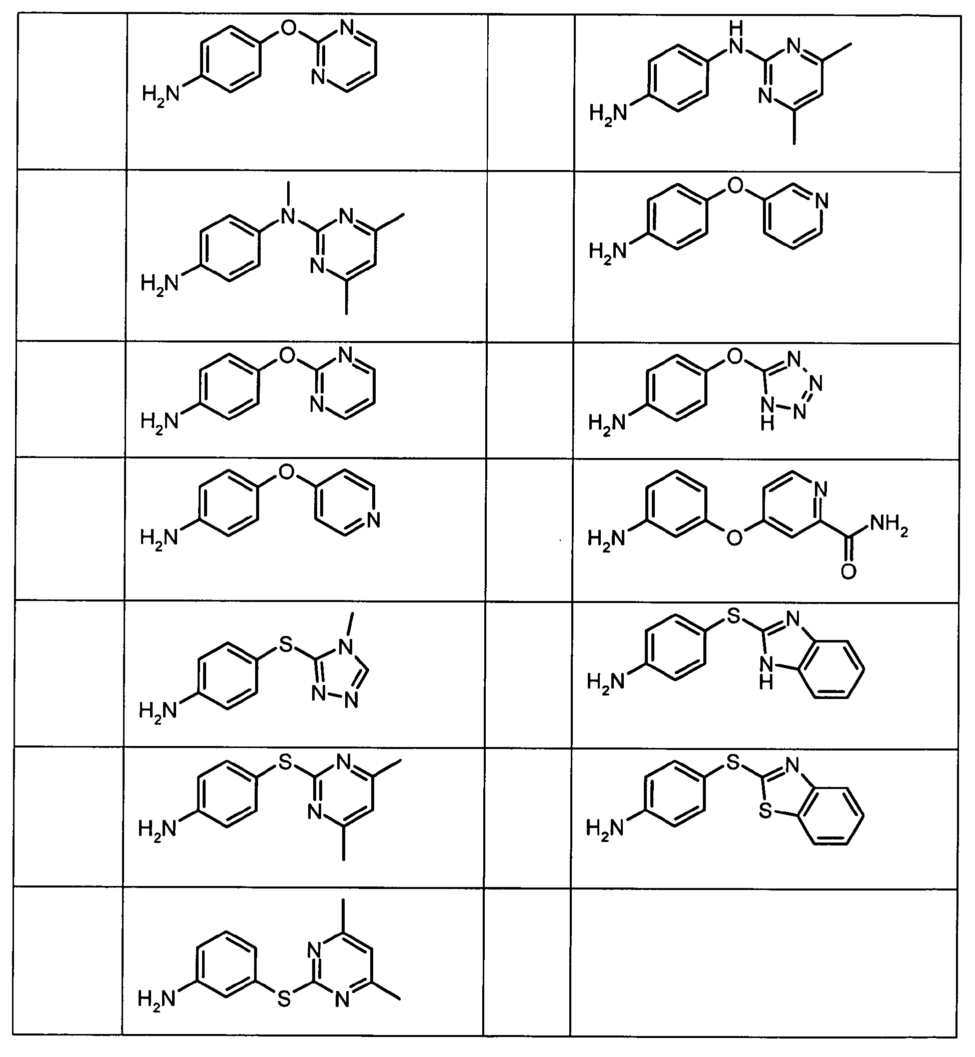

### Synthese von Intermediaten der Formel (V)

### Verfahrensvariante A I

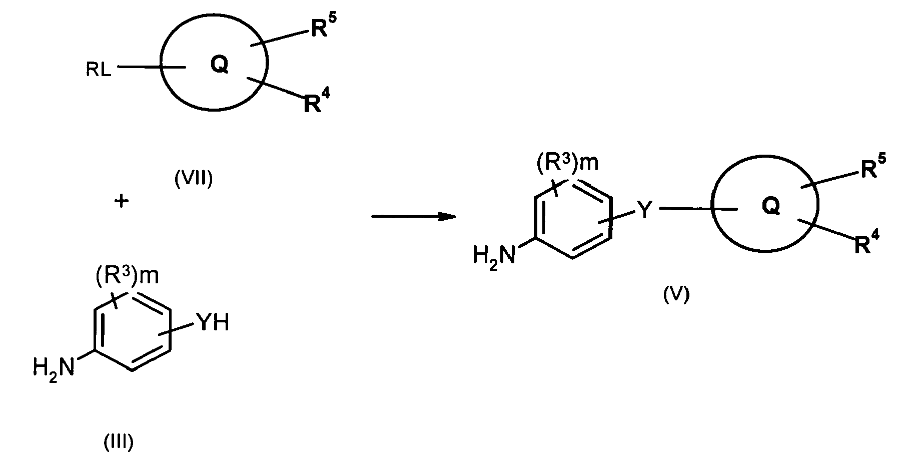

### Schema 2: Direkte Umsetzung von Anilinderivaten der Formel (III) mit Elektrophilen der Formel (VII); Die Substituenten Q, R³, R⁴, R⁵ und m haben die in der allgemeinen Formel (I) angegebenen Bedeutungen. RL als Fluchtgruppe ist insbesondere Br, Cl, I und OTf.

### Verfahrensvariante A II

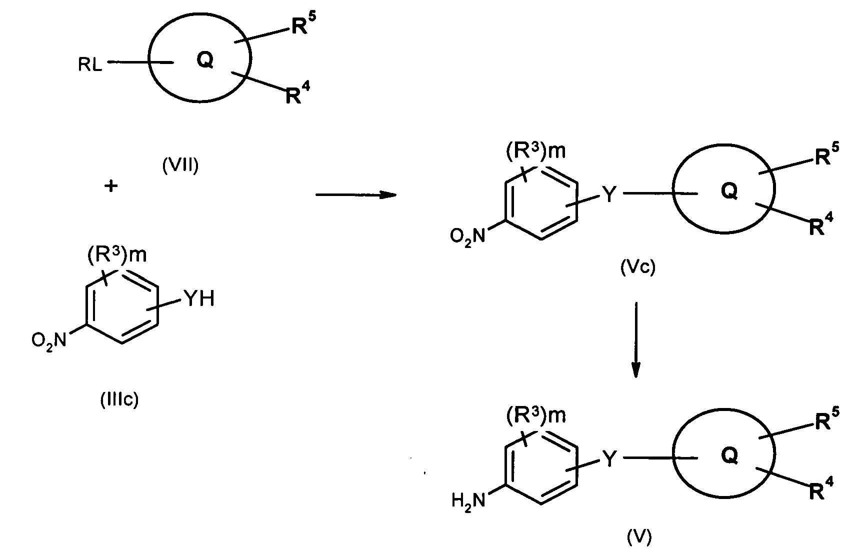

### Schema 3: Umsetzung von Nitroanilinen der Formel (IIIc) mit Elektrophilen der Formel (VII) und nachfolgender Reduktion der Nitrogruppe; Die Substituenten Q, R³, R⁴, R⁵ und m haben die in der allgemeinen Formel (I) angegebenen Bedeutungen. RL als Fluchtgruppe ist insbesondere Br, CI, 1 und OTf.

### Intermediat V.1 (nach Verfahrensvariante A I)

### N-Pyridin-2-yl-benzene-1,4-diamine

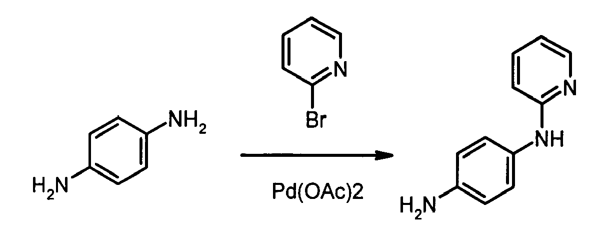

Eine Aufschlemmung von 2,7 g (25 mMol) 1,4-Phenylendiamin , 0,48 ml (5 mMol) 2-Brompyridin, 13,82 g (100 mMol) Kaliumcarbonat, 22,5 mg (0,1mMol) Pd(II)-acetat und 62,5 mg (0,1 mMol) rac-BINAP werden in 50 ml Toluol 8 h unter N2-Atmosphäre am Rückfluss gekocht. Danach wird abgesaugt, die Mutterlauge weitgehend eingeengt, von ausgefallenen Kristallen befreit (abgesaugt; Edukt Diamin)und wieder eingeengt. Nach Chromatographie (Kieselgel; Hexan/Essigester 1:1) der Mutterlauge erhält man 620 mg (67% d.Th.) des Intermediates V.1. N-Pyridin-2-yl-benzene-1,4-diamine.
¹H-NMR (DMSO): 8,32 (s, 1H), 7,98(m, 1H), 7,38(m, 1H), 7,15 (m, 2H), 6,52 (m, 4H), 4,69 (s , 2H)
MS : 186 (ES).

### Intermediat V.1 (nach Verfahrensvariante A II):

### N-Pyrimidin-2-yl-benzene-1,4-diamine

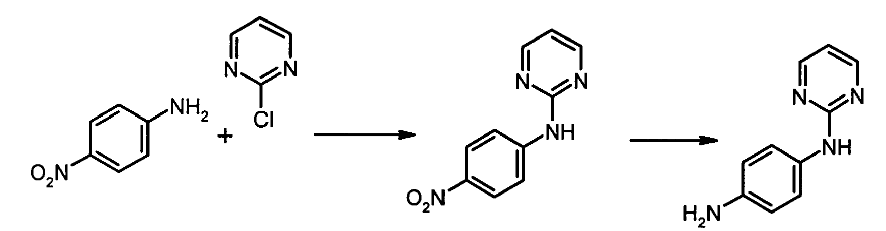

Eine Lösung von 2,76 g (20 mMol) 4-Nitroanilin und 2,29 g (20 mMol) 2-Chlorpyrimidin in 150 ml Acetonitril wird mit 5 ml (20 mMol) 4 molarer HCL in Dioxan und 5 ml Wasser versetzt und 12 h unter Rückfluß gekocht. Nach Abkühlen wird mit 3,33 ml (24mMol) Triethylamin versetzt, die Kristalle abgesaugt, mit Acetonitril und Wasser gewaschen und dann bei +60°C im Vaccuum getrocknet. Man erhält so 1,07 g (25% d.Th.) 4-Nitro-phenyl)-pyrimidin-2-yl-amine.
¹H-NMR (DMSO): 10,45 (s, 1H), 8,59(d, 2H), 8,17(m, 2H), 7,99 (m, 2H), 7,0 (m, 1H)
MS : 217 (ES).

Eine Lösung von 1,07 g (4,95 mmol) (4-Nitro-phenyl)-pyrimidin-2-yl-amine in 80 ml THF/ETOH 1:1 1 wird mit Palladium auf Kohle (10%ig) unter Wasserstoff-Atmosphäre bei Raumtemperatur hydriert .Nach dem Abfiltrieren des Katalysators und Einengen erhält man 0,88 g (95% d.Th.) des Intermediates V.1.
¹H-NMR (DMSO): 9,0 (s, 1H), 8,30(d, 2H), 7,27(m, 2H), 6,62 (m, 1H), 6,46 (m, 2H) 4,70(m , 2H)
MS : 187 (ES).

### d) Intermediate der Formel (VI):

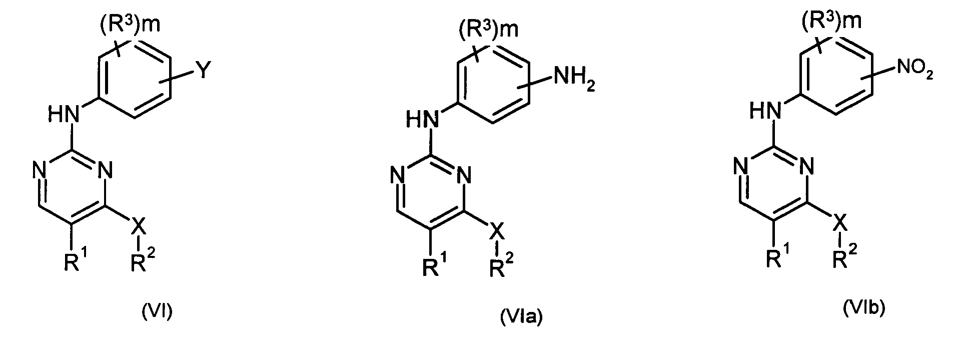

### Verfahrensvariante A III

2-Chlor-pyrimidine der Formel (II) können mit Nucleophilen der Formel (IIId) zu Verbindungen der Formel (VI) umgesetzt werden.

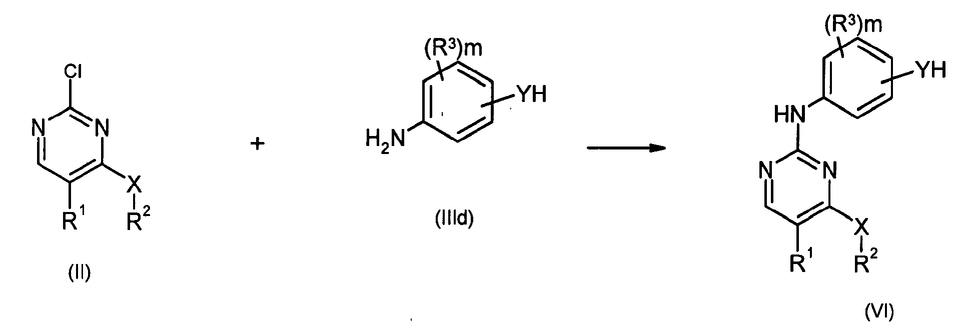

### Schema 4: Darstellung von Intermediaten des Typs (VI); Die Substituenten R¹, R², R³, Y und m haben die in der allgemeinen Formel (I) angegebenen Bedeutungen.

### Verfahrensvariante A IV

Alternativ werden 2-Chlor-Pyrimidine der Formel (II) erst mit Nitro-Anilinen der Formel (IIIc) zu Verbindungen (Vlb) umgesetzt, bei denen dann wiederum die Nitrogruppe reduziert wird unter Erhalt von Intermediaten der Formel (VIa). Für die Reduktion der Nitrogruppe stehen eine Reihe von Methoden zur Verfügung (siehe z.B.: R.C. Larock, Comprehensive Organic Transformations, VCH, New York, 1989, 411-415). Geeignet ist beispielsweise die beschriebene Hydrierung unter Verwendung von Raney Nickel oder die Verwendung von Titan(IIII)chlorid in THF

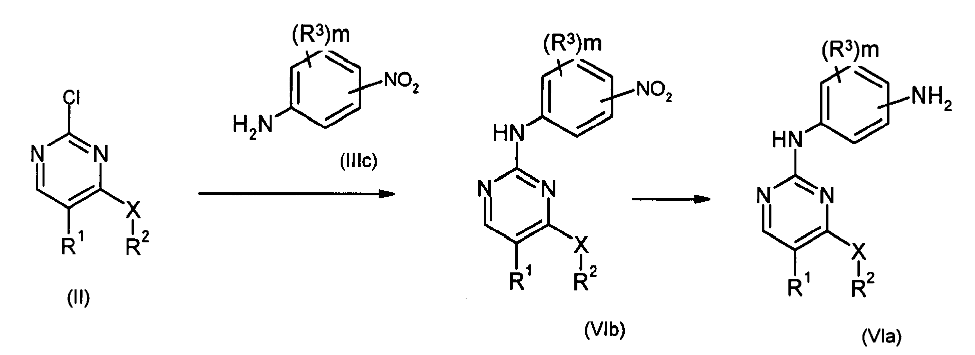

### Schema 5: Darstellung von Intermediaten des Typs (VI) via Nitro-Zwischenstufe; Die Substituenten R¹, R², R³ und m haben die in der allgemeinen Formel (I) angegebenen Bedeutungen.

### Verfahrensvariante A V

Die Mutterlauge der Umsetzung von Verfahrensvariante B I enthält in der Regel Intermediate der Formel (VI), so dass diese durch Einengen und Reinigung der Mutterlauge erhalten werden können.

### Intermediat VI.1

### (2R,3R)-3-[2-(4-Amino-phenylamino)-5-bromo-pyrimidin-4-ylamino]-butan-2-ol

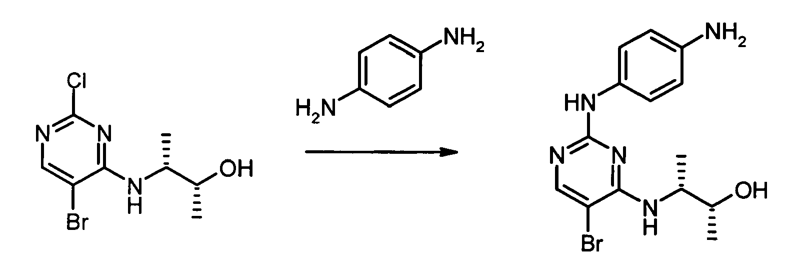

Die Mutterlauge aus der Darstellung von Beispiel 1 wird eingeengt und chromatographisch ( DCM/ MeOH 9:1 ) gereinigt. Man erhält so 73 mg des Intermediates VI.1.
¹H-NMR (DMSO): 8,67 (s, 1 H), 7,88 (s, 1 H), 7.23 (d 2H), 6,43 (d, 2H), 5,82 (d, 1 H), 4,91 (d, 1 H), 4,66 (s, 2H), 3,97 (m, 1 H), 3,71 (m, 1 H), 1,12 (d, 3H), 1,02 (d, 3H)
MS: 352 (ES).

### Intermediate VI.2 und VI.3:

### Intermediate VI.2

### (2R,3R)-3-[5-Bromo-2-(4-nitro-phenylamino)-pyrimidin-4-yloxy]-butan-2-ol und

### Intermediat VI.3

### (2R,3R)-3-[2-(4-Amino-phenylamino)-5-bromo-pyrimidin-4-yloxy]-butan-2-ol

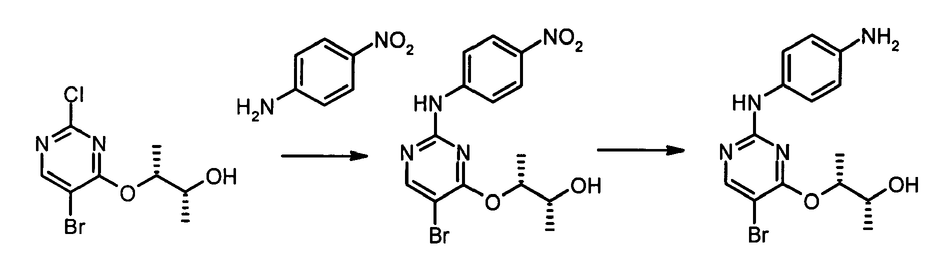

Zu einer Lösung von 1,126 g (4 mmol) (2R,3R)-3-(5-Brom-2-chlor-pyrimidin-4-yloxy)-butan-2-ol und 0,552 g (4 mmol) 4-Nitroanilin in 20 ml Acetonitril werden 1 ml Wasser und 1 ml 4 molarer Salzsäure in Dioxan addiert und dann wird 16 h bei +95°C gerührt. Nach Abkühlen addiert man 1 Äquivalenten Triethylamin und saugt die ausgefallenen Kristalle ab. Man erhält so 990 mg 64,5 % d.Th. (2R,3R)-3-[5-Bromo-2-(4-nitro-phenylamino)-pyrimidin-4-yloxy]-butan-2-ol (Intermediat VI.2).
¹H-NMR (DMSO): 10,41 ( s, 1H), 8,46(s, 1H), 8,16(d, 2H), 7,91 (d, 2H), 5,19 (m, 1H),4,87 (m, 1H), 3,79(m, 1H), 1,26 (d, 3H), 1,10 (d, 3H)
MS : 383 (ES).

56,59 g (25,7 mmol) Eisen( III)-Sulfat werden in 190 ml Wasser gelöst und mit 59 ml 25%igem wässrigem Ammoniak versetzt .Dann wird eine Aufschlemmung von 7,8 g (20,35 mmol) (2R,3R)-3-[5-Bromo-2-(4-nitro-phenylamino)-pyrimidin-4-yloxy]-butan-2-ol (Verbindung 4.2) in 234 ml Methanol bei RT zugetropft. Anschließend wird 3h bei +90°C gerührt. Nach Abkühlen wird über Celite abfiltriert, gut mit MeOH nachgewaschen, die Mutterlauge eingeengt und chromatographisch gereinigt (Dichlormethan/MeOH 4:1). So erhält man 5,68g (79 % d.Th.) des Intermediates VI.3.
¹H-NMR (DMSO): 9,11 (s, 1H), 8,17(s, 1H), 7,21 (d, 2H), 6,47 (d, 2H), 5,1 (m, 1H),4,78 (m, 3H), 3,75(m, 1H), 1,18 (d, 3H), 1,06 (d, 3H)
MS : 353 (ES).

In Analogie zu Intermediat VI.2 und VI.3 wurden folgendes Intermediate hergestellt:

| **Intermediat** | Struktur | Analytik |
|---|---|---|
| **VI.4** | 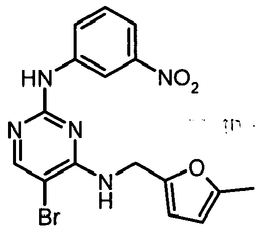 | MS: 404 (ES+) |
| **VI.5** | 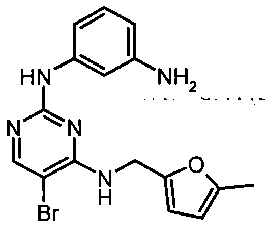 | MS: 374 (ES+) |

### e) Intermediate der Formel (VII):

Intermediate der Formel (VII) sind in großem Umfang kommerziell erhältlich oder können nach verschiedenen bekannten Methoden hergestellt werden.

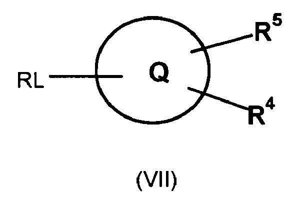

Die Substituenten Q, R⁴ und R⁵ haben die in der allgemeinen Formel (I) angegebenen Bedeutungen.
RL als Fluchtgruppe ist insbesondere -Cl, -Br, -I oder -OTf.

Folgende Intermediate der Formel VII sind beispielsweise kommerziell erhältlich:

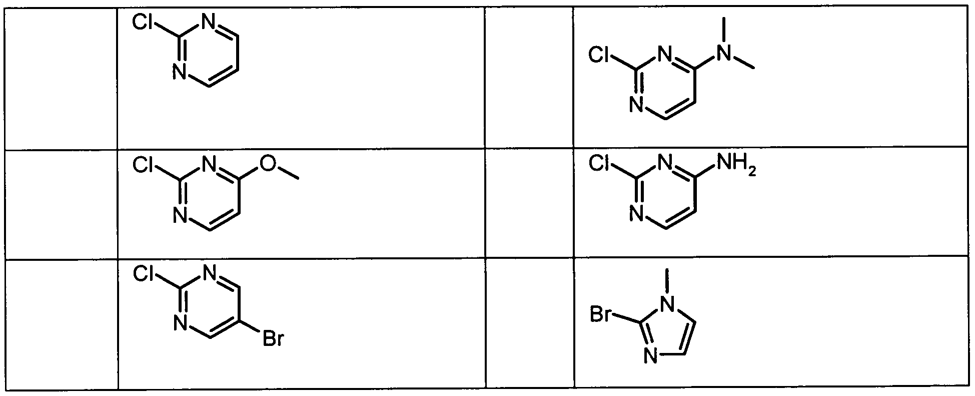

### Synthese von Intermediaten der Formel VII:

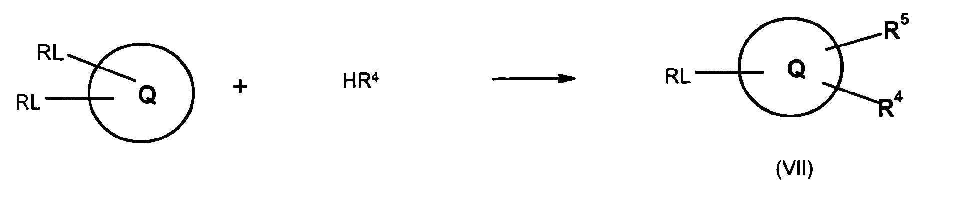

Beispielsweise können 4-Dichlor-pyrimidine der Formel (X) können mit Nucleophilen der Formel (IX) zu Verbindungen der Formel (II) umgesetzt werden. Verbindungen der Formel (II) sind eine Untergruppe der Intermediate der Formel VII mit Q gleich Pyrimidin, RL gleich Cl, R⁴ gleich XR² und R⁵ gleich R¹.

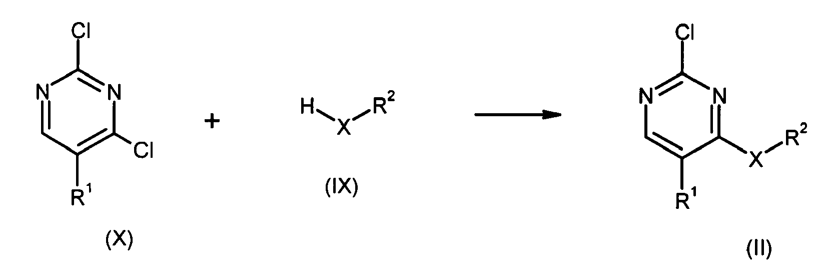

### Schema 6: Umsetzung von Dichloranilinen der Formel (X) mit Nucleophilen der Formel (IX). Die Substituenten X, R¹ und R haben die in der allgemeinen Formel (I) angegebenen Bedeutungen.

### Intermediat VII.1:

### 2-(2-Chloro-pyrimidin-4-ylamino)-ethanol

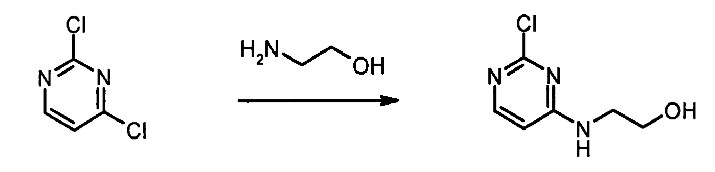

Zu einer Suspension von 1 g (6,7 mmol) 2,4-Dichlorpyrimidin in 15 ml Acetonitril/DMF 2:1 werden unter Eisbadkühlung 0,93 ml (6,7 mmol) Triethylamin hinzufügt und eine Lösung von 0,4 ml (6,7 mmol) 2-Aminoethanol in 1 ml Acetonitril hinzugetropft. Danach werden 12 h bei Raumtemperatur nachgerührt. Das Reaktionsgemisch wird abfiltirert, gut mit Dichlormethan gewaschen und die Mutterlauge einengt. Nach Chromatographie (Hexan/Essigester 1:1-> DCM/MeOH 4:1) ergaben sich 148 mg (13% d.Th.) des Intermediates VII.1.
¹H-NMR (DMSO):7,92 (d, 1H), 7,83 (d, 1H), 6,44 (m, 1H), 4,76 (d, 1H), 3,46 (m, 2H), 3,31 (m, 2H)
MS: 174 (ES).

In Analogie zu Intermediat VII.1 werden folgende Intermediate hergestellt:

| **Intermediat** | Struktur | Analytik |
|---|---|---|
| **VII.2** | 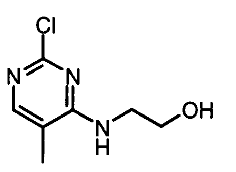 | MS: 188 (ES+) |
| **VII.3** | 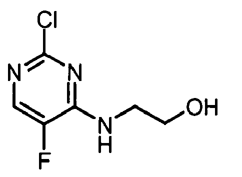 | MS : 192 (ES+) |
| **VII.4** | 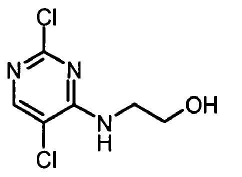 | MS : 208 (ES+) |
| **VII.5** | 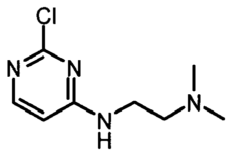 | MS : 201 (ES+) |

### Intermediat VII.6:

### 2-(2-Chloro-pyrimidin-4-yloxy)-ethanol

### und

### Intermediat VII.7:

### 2-(4-Chloro-pyrimidin-2-yloxy)-ethanol

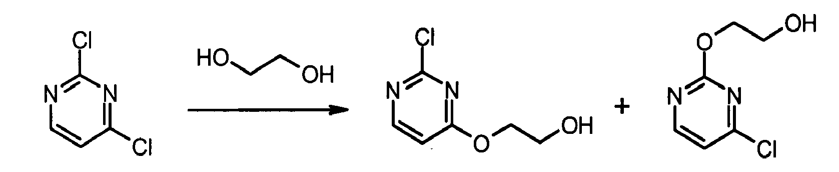

Eine Lösung von 0,42 ml (7,5 mmol) Ethylenglykol in 10 ml Acetonitril/NMP 1:1 wird portionsweise mit 327 mg (7,5 mmol) Natriumhydrid (55-60%ig in Öl) bei Raumtemperatur versetzt und dann werden weiter 15 min bei RT nachgerührt .Die entstandene Aufschlemmung wird unter Eisbadkühlung portionsweise zu einer Lösung von 1,34 g (9 mmol) 2,4-Dichlorpyrimidin in 10 ml Acetonitril addiert . Danach lässt man langsam auf Raumtemperatur kommen und 12 h rühren. Dann wird der Ansatz auf Eiswasser gegossen, mit Essigester extrahiert, mit Natriumsulfat getrocknet, eingeengt und chromatographisch gereinigt (Kieselgel; Gradient Hexan/Essigester 2:1 -> reiner Essigester). Man erhält so 275 mg (21% d.Th.) eines 4:1 Regioisomerengemisches (laut H-NMR) von 2-(2-Chloro-pyrimidin-4-yloxy)-ethanol und 2-(4-Chloro-pyrimidin-2-yloxy)-ethanol
MS : 175 (ES+)

In Analogie zu Intermediat VII.7 wird folgendes Intermediat hergestellt:

| Intermediat | Struktur | Analytik | Bemerkungen |
|---|---|---|---|
| **VII.8** | 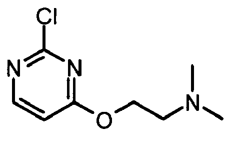 | MS : 202 (ES+) | Regioselektive Reaktion |

### f) Intermediate der Formel (VIII):

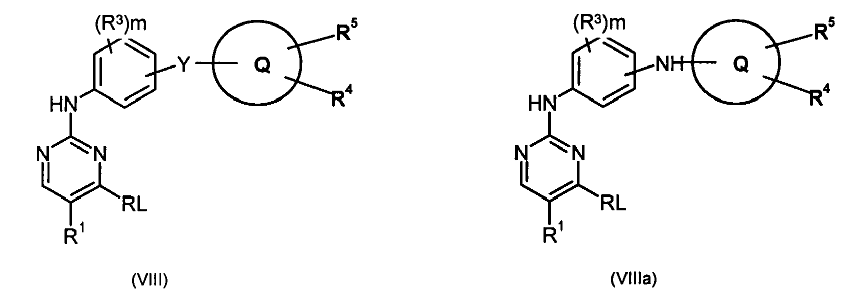

Substituierte 2-Chlor-pyrimdine der Formel (XI) können mit Nucleophilen der Formel (V) zu Verbindungen der Formel (VIII) umgesetzt werden.

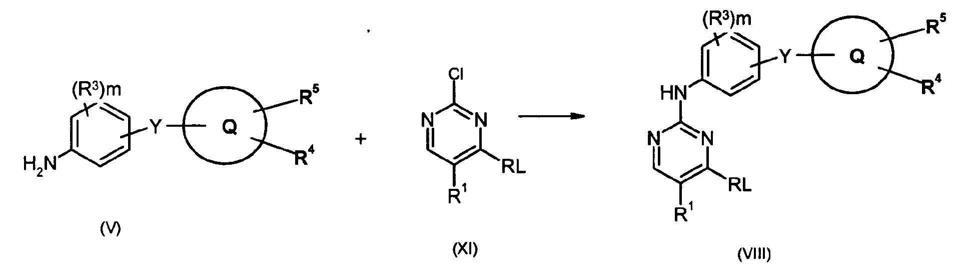

### Schema 7: Die Substituenten Q, R¹, R², R³, R⁴, R⁵, X, Y und m haben die in der allgemeinen Formel (I) angegebenen Bedeutungen. RL als maskierte Fluchtgruppe ist insbesondere SMe und S-Alkyl.

### Intermediat VIII.1

### N-(5-Bromo-4-methylsulfanyl-pyrimidin-2-yl)-N'-pyrimidin-2-yl-benzene-1,4-diamine

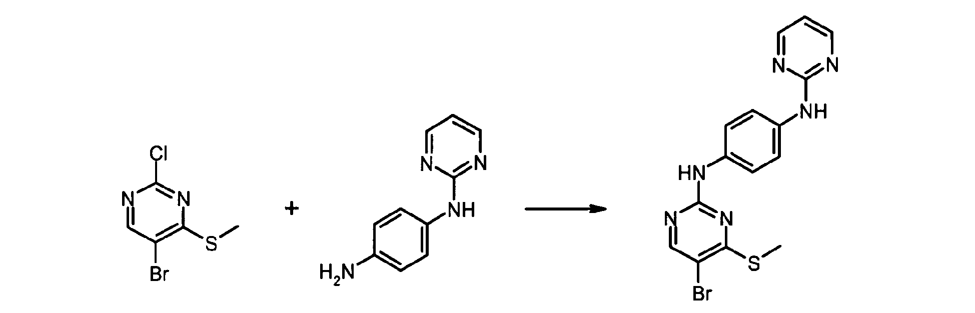

Eine Lösung von 782 mg (3,26 mmol) .) N-Pyrimidin-2-yl-benzene-1,4-diamin e und 608 mg (3,26 mmol) 5-Bromo-2-chloro-4-methylsulfanyl-pyrimidine in 13,3 ml n-Butanol/MeOH 10:1 1 wird 40 Stunden bei +60°C gerührt. Danach wird Eingeengt, mit Acetonitril versetzt und die Kristalle abgesaugt. Die Kristalle werden mit Acetonitril und Wasser gewaschen und dann bei +40°C im Vaccum getrocknet. Man erhält so 320 mg (25% d.Th.) des Intermediates VIII.1.
¹H-NMR (DMSO): 9,59 (s, 1H), 9,46(s, 1H), 8,40(d, 2H), 8,21 (s, 1H), 7,58 (m, 4H),6,75 (m, 1H), 2,51 (s, 3H)
ES+ : 391

In Analogie zu Intermediat VIII.1 wird folgendes Intermediat hergestellt:

| **Intermediat** | Struktur | Analytik |
|---|---|---|
| **VIII.2** | 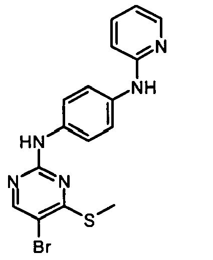 | MS : 390 (ES+) |

Die Intermediate der Formel VIII können je nach maskierter Fluchtgruppe bereits Verbindungen sein, die unter die allgemeine Formel (I) fallen und als Proteinkinaseinhibitoren Aktivität zeigen, so auch die Intermediate VIII.1 und
Intermediat VIII.2 (Verbindungen 23 und 29).

### B. Herstellung der erfindungsgemäßen Verbindungen

### Verfahrensvariante B 1:

2-Chlor-pyrimidine der Formel (II) können mit Phenylen-diaminen der Formel (IIIa) zu den gewünschten symmetrischen Zielverbindungen der Formel (IV) umgesetzt werden.

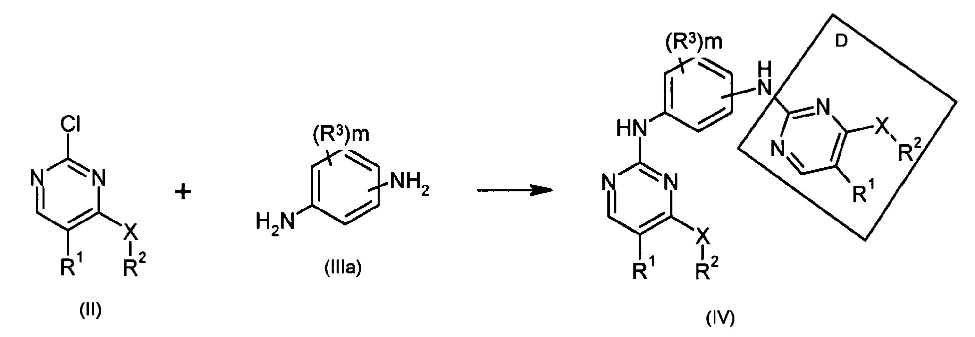

### Schema 8: Genereller Zugang zu symmetrischen Zielverbindungen Die Substituenten R¹, R², R³, X und m haben die in der allgemeinen Formel (I) angegebenen Bedeutungen wobei in Baustein D der Formel (IV) X-R² die Bedeutung von R⁴ und R¹ die Bedeutung von R⁵ hat.

### Beispiel 1:

### (2R,3R)-3-(5-Bromo-2-{4-[5-bromo-4-((1 R,2R)-2-hydroxy-1-methyl-propylamino)-pyrimidin-2-ylamino]-phenylamino}-pyrimidin-4-ylamino)-butan-2-ol

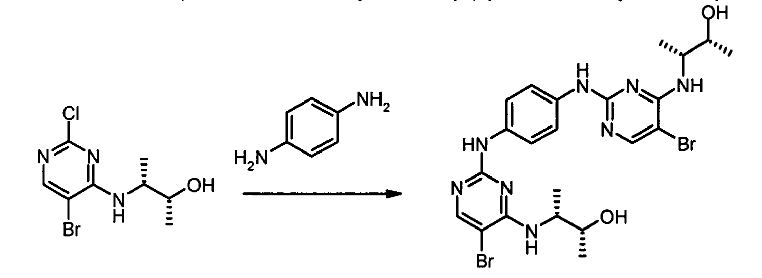

Eine Lösung von 281 mg (1 mmol) (2R,3R)-3-(5-Brom-2-chlor-pyrimidin-4-ylamino)-butan-2-ol und 216 mg (2 mmol) 1,4-Phenylendiamin in 4 ml Acetonitril und 0,6 ml Wasser wird mit 2 Äquivalenten 4 molarer Salzsäure in Dioxan versetzt und 12 h bei +95°C gerührt. Nach dem Abkühlen werden die Kristalle abgesaugt, mit Acetonitril gewaschen und bei +60°C im Vakuum getrocknet. Man erhält 38 mg (6 % d.Th.) der Verbindung 1.
¹H-NMR (DMSO): 9.03 (s, 2H), 7.96 (s, 2H), 7.52 (m, 4H), 5,91 (d, 2H), 4,96 (d, 2H), 4,01 (m, 2H), 3,71 (m, 2H), 1,15 (d, 6H), 1,04 (d, 6H),
MS: 597 (ES).

Die Mutterlage enthält Intermediat 9 (siehe Kapitel B. IV Herstellung von Intermediate der Formel VI).

### Beispiel 2:

### R)-3-(5-Bromo-2-{4-[5-bromo-4-((R)-2-hydroxy-1,2-dimethyl-propylamino)-pyrimidin-2-ylamino]-phenylamino}-pyrimidin-4-ylamino)-2-methyl-butan-2-ol

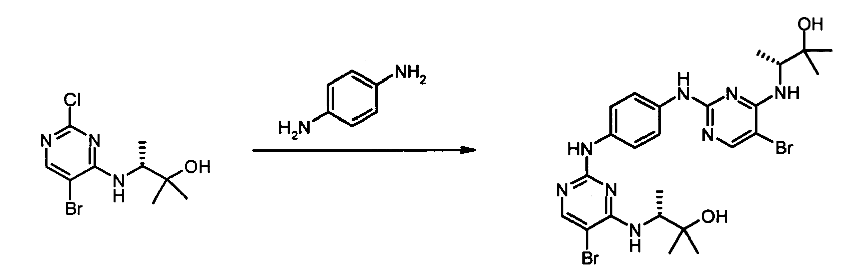

Eine Lösung von 884 mg (3 mmol) (R)-3-(5-Brom-2-chlor-pyrimidin-4-ylamino)-2-methyl-butan-2-ol und 649 mg (6 mmol) 1,4-Phenylendiamin in 50 ml Acetonitril wird mit 0,75 ml (3 mmol) 4 molarer Salzsäure in Dioxan versetzt und 16 h bei +95°C gerührt. Nach dem Abkühlen werden die Kristalle abgesaugt, mit Acetonitril gewaschen und bei +60°C im Vaccum getrocknet. Man erhält so 95 mg (5 % d.Th.) der Verbindung 2.
¹H-NMR (DMSO): 9,07 (s, 2H), 8,01(s, 2H), 7,56(s, 4H), 5,95 (s, 2H), 4,82 (s, 2H),4,1 (m, 2H), 1,18(d, 9H), 1,13 (d, 9H)
MS : 625 (ES).

### Beispiel 3:

### (R)-3-(5-Bromo-2-{3-[5-bromo-4-((R)-2-hydroxy-1,2-dimethyl-propylamino)-pyrirnidin-2-ylamino]-phenylamino}-pyrimidin-4-ylamino)-2-methyl-butan-2-ol

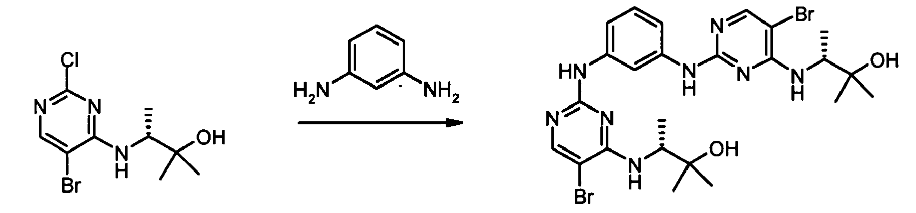

Eine Lösung von 295 mg (1 mmol) (R)-3-(5-Brom-2-chlor-pyrimidin-4-ylamino)-2-methyl-butan-2-ol und 216 mg (2 mmol) 1,3-Phenylendiaminhydrochlorid in 7,5 ml Acetonitril und 0,6 ml Wasser wird mit 4 molarer Salzsäure in Dioxan versetzt und 16 h bei +95°C gerührt. Nach dem Abkühlen werden die Kristalle abgesaugt, mit Acetonitril gewaschen und bei +60°C im Vaccum getrocknet. Man erhält so 157 mg (43 % d.Th.) der Verbindung 3.
¹H-NMR (DMSO): 10,12 (s, 2H) 8,21 (s, 2H), 8,1 (s, 1H), 7.28 (m, 3H), 6,82 (s, 2H), 4,0 (m, 1 H), 1,03 (m, 18H)
MS: 625 (ES)

### Verfahrensvariante B II:

2-Chlor-pyrimidine der Formel (II) können mit verschiedenartig substituierten Anilinen der Formel (V) zu den gewünschten Zielverbindungen der Formel (I) umgesetzt werden.

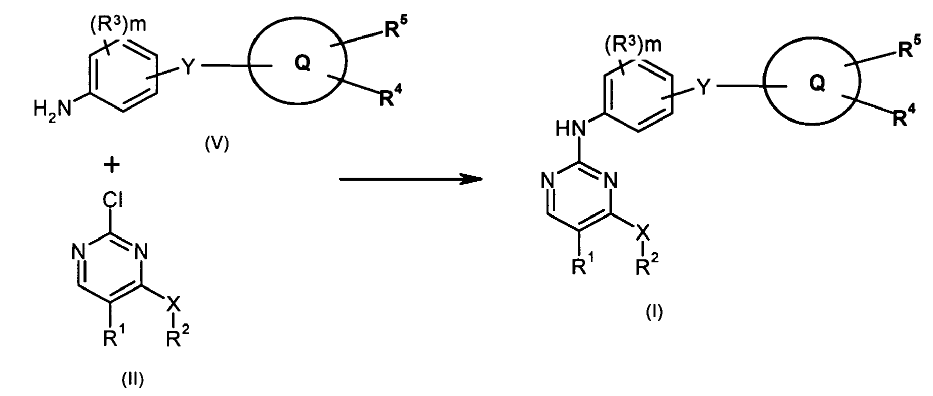

### Schema 9: Genereller Zugang zu unsymmetrischen Zielverbindungen; Die Substituenten Q, R¹, R², R³, R⁴, R⁵, X, Y und m haben die in der allgemeinen Formel (I) angegebenen Bedeutungen.

### Beispiel 4:

### (2R,3R)-3-{2-[4-(Pyrimidin-2-yloxy)-phenylamino]-5-trifluoromethyl-pyhmidin-4-ylamino}-butan-2-ol

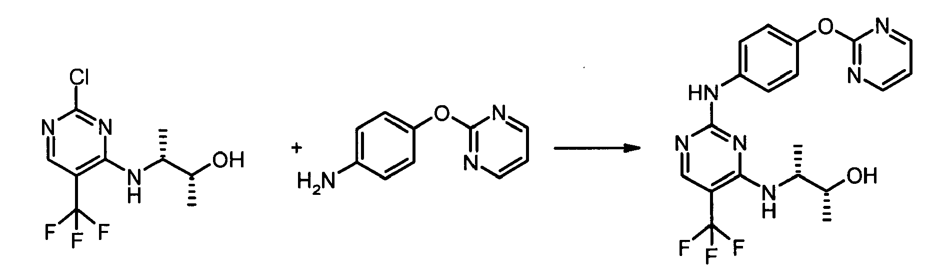

Eine Lösung von 37 mg (0,2 mmol) 4-(2- pyrimidinyloxy) phenylamin und 54 mg (0,2 mmol) (2R,3R)-3-(2-Chloro-5-trifluoromethyl-pyrimidin-4-ylamino)-butan-2-ol in 4,4 ml n-Butanol/MeOH 10:1 1 wird mit 0,3 Äquivalenten 4 molarer Salzsäure in Dioxan versetzt und 24 Stunden bei +60°C gerührt. Nach Abkühlen wird mit 1,3 Äquivalenten (0,036 ml) Triethylamin versetzt, eingeengt und dann mit DCM/MeOH 9:1 über Kieselgel geflasht. Man erhält so 45 mg (53% d.Th.) der Verbindung 4.
¹H-NMR (DMSO): 9,67 (s, 1H), 8,59 (d,2H), 8,19 (s, 1H), 7,74 (d, 2H), 7,20 (m, 1H), 7,06 (m, 2H), 5,94 (d, 1H), 5,04 (d, 1H), 4,1 (m, 1H), 3,73 (m, 1H), 1,16 (d, 3H), 1,04 (d, 3H)
MS : 421 (ES).

### Verfahrensvariante B III

Substituierte Anilinopyrimdine der Formel (VI) können mit Elektrophilen der Formel (VII) zu Verbindungen der Formel (I) umgesetzt werden. Diese Umsetzung kann sowohl durch Säuren, Basen oder Metalle (z.B. Palladium- oder Kupfer-Komplexe) katalysiert werden.

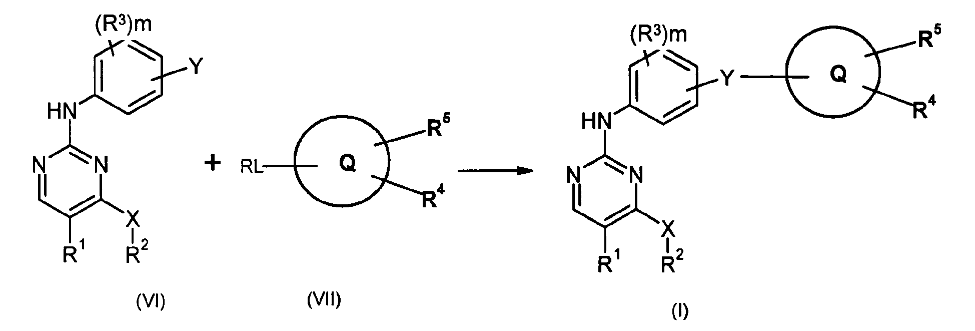

### Schema 10: Herstellung der Zielverbindungen durch direkte Addition von Heteroaromaten an Anilininopyrimdine. Die Substituenten Q, R¹, R², R³, R⁴, R⁵, X, Y und m haben die in der allgemeinen Formel (I) angegebenen Bedeutungen. RL steht für eine Fluchtgruppe wie beispielsweise Cl, F, Br, l, OTf.

### Beispiel 5:

### (2R,3R)-3-{5-Bromo-2-[4-(pyrimidin-2-ylamino)-phenylamino]-pyrimidin-4-ylamino}-butan-2-ol

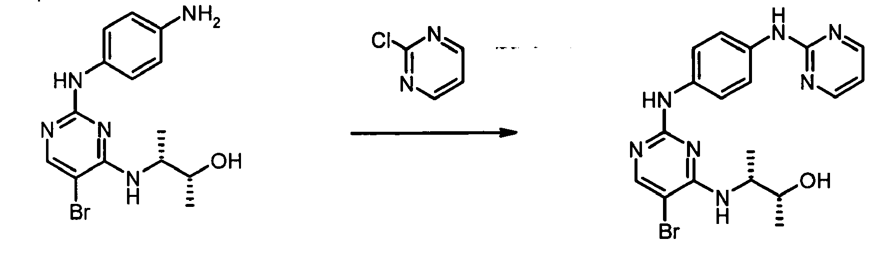

Eine Lösung von 71 mg (0,2 mmol) (2R,3R)-3-[2-(4-Amino-phenylamino)-5-bromo-pyrimidin-4-ylamino]-butan-2-ol und 23 mg (0,2 mmol) 2-Chlorpyrimidin in 3,3 ml n-Butanol/MeOH 10:1 1 wird mit 1,5 Äquivalenten 4 molarer Salzsäure in Dioxan versetzt und 16 Stunden bei +60°C gerührt. Nach Abkühlen werden 2 Äquivalente Triethylamin addiert, eingeengt und chromatographisch (DCM/ MeOH 9:1) gereinigt. Man erhält so 15 mg (18 % d.Th.) der Verbindung 5.
¹H-NMR (DMSO): 9,37 (s, 1H), 9,04 (s, 1H), 8,38 (m, 2H), 7,96 (s1H), 7,54 (s, 4H), 6,71 (m, 1H), 5,91 (d, 1H), 4,95 (d, 1H), 3,74 (m, 1H), 3,72 (m, 1H), 1,15 (d, 3H), 1,05 (d, 3H)
MS: 430 (ES).

### Beispiel 6:

### (2R,3R)-3-(5-Bromo-2-{4-[4-(2-hydroxy-ethylamino)-pyrimidin-2-ylamino]-phenylamino}-pyrimidin-4-ylamino)-butan-2-ol

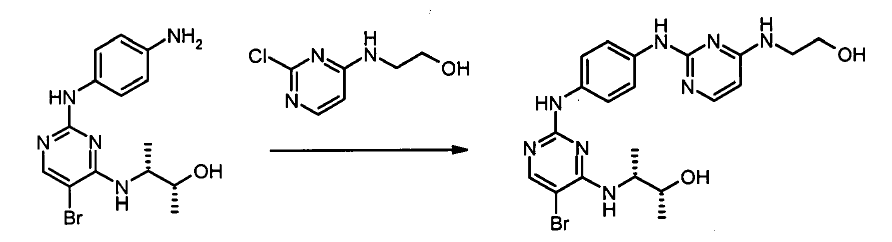

Eine Lösung von 71 mg (0,2 mmol) (2R,3R)-3-[2-(4-Amino-phenylamino)-5-bromo-pyrimidin-4-ylamino]-butan-2-ol und 35 mg (0,2 mmol) 2-(2-Chloro-pyrimidin-4-ylamino)-ethanol in 3,3 ml n-Butanol/MeOH 10:1 wird mit 0,3 Äquivalenten 4 molarer Salzsäure in Dioxan versetzt und 16 Stunden bei +60°C gerührt . Nach Abkühlen werden 1,3 Äquivalenten (0,036 ml) Triethylamin addiert und eingeengt. Danach wird in Acetonitril aufgenommen, die Kristalle absaugt und mit etwas Acetonitril und Wasser waschen. Nach Trocknen bei +40°C im Vakuum erhält man 73 mg (75% d.Th.) der Verbindung 6.
¹H-NMR (DMSO): 10,16( s, 1H) ,9,31 (s, 1H),8,94 (s, 1H),8,01 (s, 1H),7,70 (m, 3H), 7,36(m, 2H), 6,18(d, 1H), 6,02 (d, 1H), 4,97 (m, 2H), 4,04 (m, 2H), 3,42 (m, 2H) 1,16 (d, 3H), 1,04 (d, 3H)
MS : 489 (ES).

### Beispiel 7:

### (2R,3R)-3-(5-Bromo-2-{4-[4-(2-hydroxy-ethylamino)-pyrimidin-2-ylamino]-phenylamino}-pyrimidin-4-yloxy)-butan-2-ol

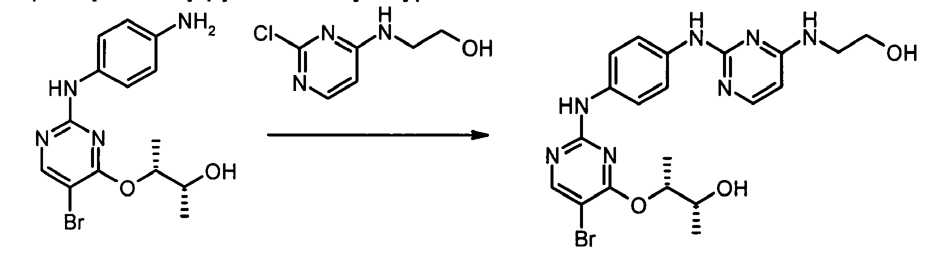

Eine Lösung von 71 mg (0,2 mmol).) (2R,3R)-3-[2-(4-Amino-phenylamino)-5-bromo-pyrimidin-4-yloxy]-butan-2-ol und 35 mg (0,2 mmol) 2-(2-Chloro-pyrimidin-4-ylamino)-ethanol in 3,3 ml n-Butanol/MeOH 10:1 1 wird mit 0,3 Äquivalenten 4 molarer Salzsäure in Dioxan versetzt und 16 Stunden bei +60°C gerührt . Nach Abkühlen wird mit 1,3 Äquivalenten (0,036 ml) Triethylamin versetzt, eingeengt, dann mit Acetonitril versetzt und die Kristalle abgesaugt. Die Kristalle werden Acetonitril und Wasser gewaschen und dann bei +40°C im Vaccum getrocknet. Man erhält so 60 mg (61 % d.Th.) der Verbindung 7.
¹H-NMR (DMSO): 10,3 (s, 1H), 9,72(s, 1H), 9,02(s, 1H), 8,32 (s, 1H), 7,68 (m, 3H),7,41 (m, 2H), 6,20(d, 1H), 5,15 (m, 1H), 4,87 (m, 2H), 3,78 (m, 1H), 3,54 (m, 2H), 3,42 (m, 2H),1,23 (d, 3H), 1,08 (d, 3H)
MS : 490 (ES).

### Beispiele 8 und 9

### Beispiel 8:

### (2R,3R)-3-(5-Bromo-2-{4-[4-(2-hydroxy-ethoxy)-pyrimidin-2-ylamino]-phenylamino}-pyrimidin-4-yloxy)-butan-2-ol

### und

### Beispiel 9:

### (2R,3R)-3-(5-Bromo-2-{4-[2-(2-hydroxy-ethoxy)-pyrimidin-4-ylamino]-phenylamino}-pyrimidin-4-yloxy)-butan-2-ol

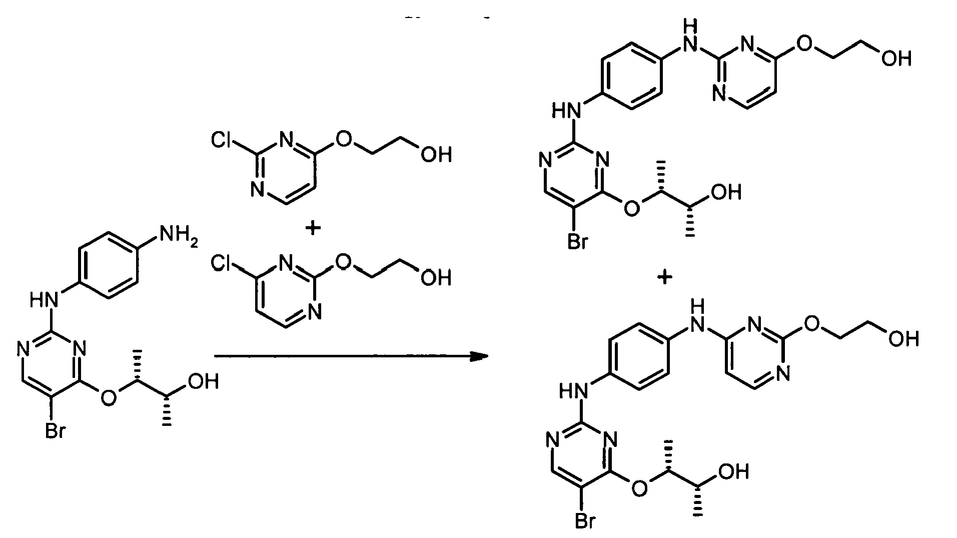

Eine Lösung des Regioisomerengemischs von 2-(2-Chloro-pyrimidin-4-yloxy)-ethanol und 2-(4-Chloro-pyrimidin-2-yloxy)-ethanol (35 mg, 0,2 mmol) und 71 mg (0,2 mMol) (2R,3R)-3-[2-(4-Amino-phenylamino)-5-bromo-pyrimidin-4-yloxy]-butan-2-ol in 4,4 ml n-Butanol/MeOH 10:1 wird mit 0,3 Äquivalenten 4 molarer Salzsäure in Dioxan versetzt und 24 Stunden bei +60°C gerührt . Nach Abkühlen wird mit 1,3 Äquivalenten (0,036 ml) Triethylamin versetzt, eingeengt und dann mit DCM/MeOH 9:1 über Kieselgel geflasht. Man erhält so 22 mg (22% d.Th.) der Verbindung 8 und 15 mg (15% d.Th.) der Verbindung 9.

### Verbindung 8:

¹H-NMR (DMSO): 9,56(s, 1H), 9,45(s, 1H), 8,28(s, 1H), 7,97 (d, 1H), 7,58 (m, 2H),7,52 (m, 2H), 6,33(d, 1H), 5,15 (m, 1H), 4,82 (m, 2H), 4,20 (m, 2H), 3,77 (m 1H), 3,66 (m, 2H), 1,22(d, 3H), 1,08(d, 3H)
MS : 493 (ES).

### Verbindung 9:

¹H-NMR (DMSO): 9,49( s, 1H), 9,34(s, 1H), 8,26(s, 1H), 8,13 (d, 1H), 7,59 (m, 2H),7,54 (m, 2H), 6,18(d, 1H), 5,13 (m, 1H), 4,83 (m, 2H), 4,28 (m, 2H), 3,77 (m 1H), 3,68 (m, 2H), 1,20(d, 3H), 1,08(d, 3H)
MS : 493 (ES).

### Beispiele 10 und 11

### Beispiel 10:

### (2R,3R)-3-(5-Bromo-2-{4-[4-(2-dimethylamino-ethoxy)-pyrimidin-2-ylamino]-phenylamino}-pyrimidin-4-yloxy)-butan-2-ol

### und

### Beispiel 11:

### 2-{4-[5-Bromo-4-((1 R,2R)-2-hydroxy-1-methyl-propoxy)-pyrimidin-2-ylamino]-phenylamino}-pyrimidin-4-ol

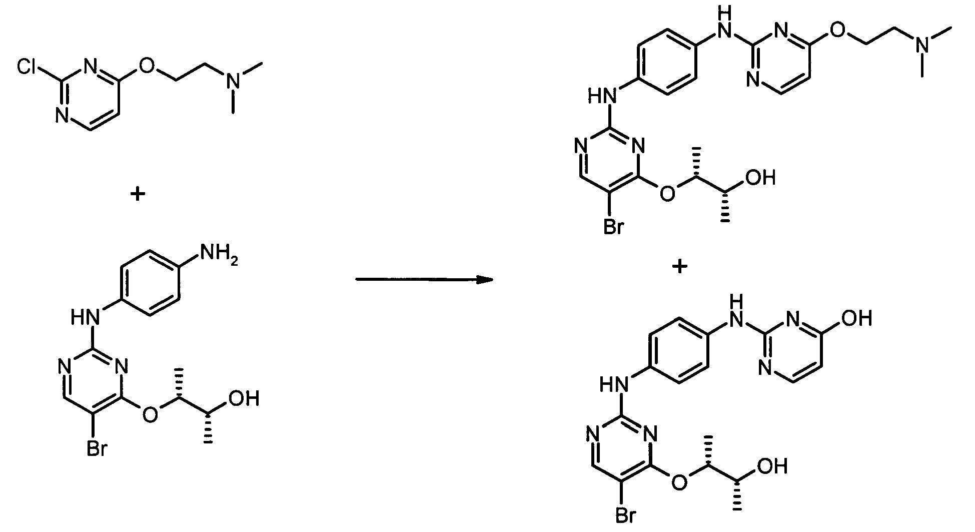

Eine Lösung von -[2-(2-Chlorpyrimidin)-4-yloxy)-ethyl]-dimethylamin (100 mg, 0,5 mmol) und 175,2 mg (0,5 mmol) (2R,3R)-3-[2-(4-Amino-phenylamino)-5-bromo-pyrimidin-4-yloxy]-butan-2-ol in 8,3 ml n-Butanol/MeOH 10:1 wird mit 1,5 Äquivalenten 4 molarer Salzsäure in Dioxan versetzt und 24 Stunden bei +60°C gerührt. Nach Abkühlen wird mit 3 Äquivalenten (0,21 ml) Triethylamin versetzt, eingeengt und dann mit DCM/MeOH 9:1 über Kieselgel geflasht. Man erhält so 50 mg eines Gemisches ,das durch HPLC aufgetrennt wird in 19 mg 7,4%d.Th. (2R,3R)-3-(5-Bromo-2-{4-[4-(2-dimethylamino-ethoxy)-pyrimidin-2-ylamino]-phenylamino}-pyrimidin-4-yloxy)-butan-2-ol und 26 mg 11,7%d.Th. 2-{4-[5-Bromo-4-((1R,2R)-2-hydroxy-1-methyl-propoxy)-pyrimidin-2-ylamino]-phenylamino}-pyrimidin-4-ol.

### Verbindung 10

¹H-NMR (DMSO): 9,52(s, 1H), 9,45(s, 1H), 8,27(s, 1H), 8,19 (d, 1H), 7,55 (m, 4H),6,23 (d, 1H), 5,16(m, 1H), 4,87 (s, 1H), 4,74 (s, 1H), 3,76 (m, 3H), 3,12 (s 6H ),1,21 (d, 3H), 1,07(d, 3H)
MS: 520 (Cl).

### Verbindung 11

¹H-NMR (DMSO): 9,66(s, 1H), 8,30(s, 1H), 7,66(m, 3H), 7,41 (d, 2H), 5,84 (d, 1H),5,15 (m, 2H), 3,78(m, 1H), 1,23 (d, 3H), 1,07 (d, 3H)
MS: 447 (ES).

### Verfahrensvariante B IV:

Substituierte Anilinopyrimdine der Formel (VIII) können mit Nucleophilen der Formel (IX) zu Verbindungen der Formel (I) umgesetzt werden.

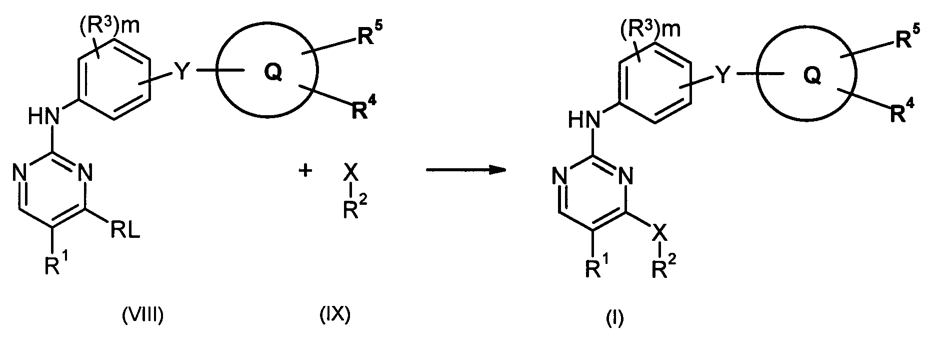

### Schema 11: Herstellung der Zielverbindungen durch nucleophlie Substitution in 4-Position des Pyrimidins. Die Substituenten Q, R¹, R², R³, R⁴, R⁵, X, Y und m haben die in der allgemeinen Formel (I) angegebenen Bedeutungen. RL steht für eine Fluchtgruppe wie -F, -Cl, -Br, -I, -OCF₃, -S-CH₃,-SOCH₃ oder -SO₂-CH₃.

### Beispiele 12-14

### Beispiel 12:

### (2R,3R)-3-{5-Bromo-2-[4-(pyridin-2-ylamino)-phenylamino]-pyrimidin-4-ylamino}-butan-2-ol

### und

### Beispiel 13:

### (2R,3R)-3-{5-Bromo-2-[4-(1-oxy-pyridin-2-ylamino)-phenylamino]-pyrimidin-4-ylamino}-butan-2-ol

### und

### Beispiel 14:

### N-(5-Bromo-4-methanesulfinyl-pyrimidin-2-yl)-N'-pyridin-2-yl-benzene-1,4-diamine

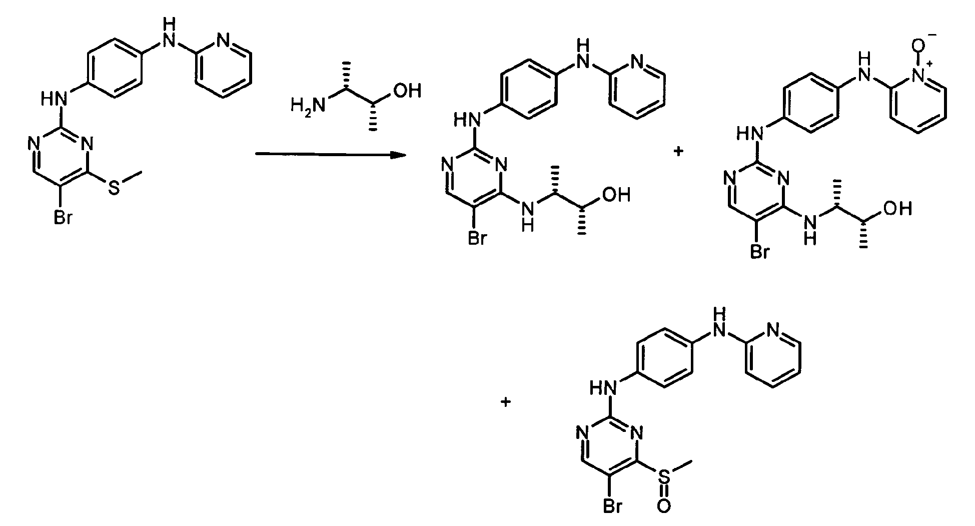

Eine Lösung von 87 mg (0,22 mmol) N-(5-Bromo-4-methylsulfanyl-pyrimidin-2-yl)-N'-pyridin-2-yl-benzene-1,4-diamine in 2 ml N-Methyl-2-pyrrolidinon wird portionsweise bei RT mit 1,5 Äquivalenten (75,3 mg, 0,34 mmol) m-CPBA versetzt. Nach 2 h Rühren bei RT werden 4 Äquivalenten Triethylamin (0,12 ml, 0,9mmol) und 56 mg (0,45 mmol) (2R,3R)-3-Amino-butan-2-ol addiert. Nach 5 h bei +60°C wird das Reaktionsgemisch auf Eiswasser gegossen, 3 x mit Essigester extrahiert, mit Natriumsulfat getrocknet und eingeengt. Flashchromatographie über Kieselgel .CH2CL2/MeOH 9:1 ergab 14 mg (15 % d.Th.) der Verbindung 12, sowie 25 mg (25% d.Th.) der Verbindung 13 und 19 mg (21 % d.Th.) der Verbindung 14.

### Verbindung 12

¹H-NMR (DMSO): 9,75 (m, 1H), 9,60(s, 1H), 8,05(s, 1H), 7,96 (d, 1H), 7,75 (m, 1H),7,63 (d, 2H), 7,42(d, 2H), 6,59 (d, 1H), 6,82 (m, 1H), 6,51 (m, 1H), 4,03 (m, 1H), 3,73 (m, 1H),1,14 (d, 3H), 1,03 (d, 3H)
MS : 429 (ES).

### Verbindung 13

¹H-NMR (DMSO): 9,28 ( s, 1H), 9,02(s, 1H), 8,21 (d, 1 H), 8,05 (s, 1 H), 7,74 (d, 2H),7,23 (m, 3H), 7,04(d, 1H), 6,74 (m, 1H), 6,03 (d, 1H), 5,0 (d, 1H), 4,09 (m, 1H ) , 3,78 (m, 1H) ,1,22 (d, 3H), 1,10 (d, 3H)
MS : 445 (ES).

### Verbindung 14

¹H-NMR (DMSO): 9,82 (s, 1H), 9,09(s, 1H), 8,30(s, 1H), 8,20 (d, 1H), 7,75 (m, 2H),7,26 (m, 3H), 7,09(d, 1H), 6,76 (m, 1H), 2,57(s, 3H)
MS : 406(ES).

Die folgenden Verbindungen werden in Analogie zu den oben genannten Beispielen hergestellt:

| **Beispiel** | Struktur | Status / Bemerkungen | Herstellung |
|---|---|---|---|
| 15 | 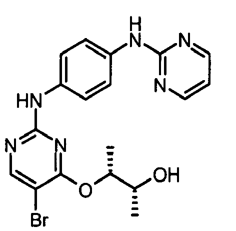 | MS : 430 (ES) | Variante B III |
| 16 | 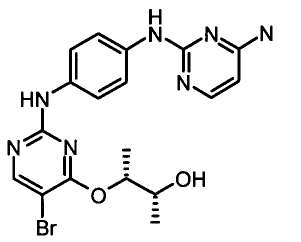 | MS: 446 (ES) | Variante B III |
| 17 | 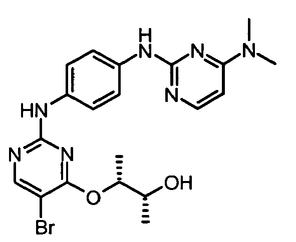 | MS : 474 (ES) | Variante B III |
| 18 | 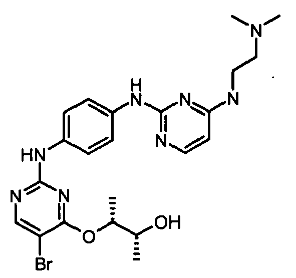 | MS : 519 (ES) | Variante B III |
| 19 | 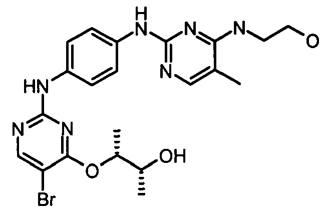 | MS: 504 (ES) | Variante B III |
| 20 | 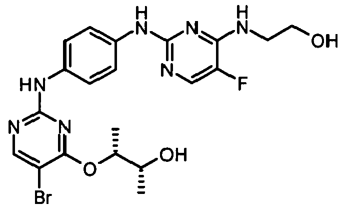 | MS: 508 (ES) | Variante B III |
| 21 | 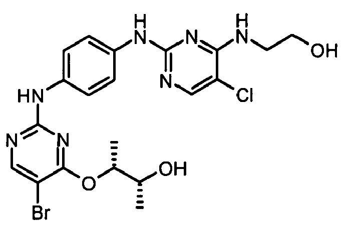 | MS: 524 (ES) | Variante B III |
| 22 | 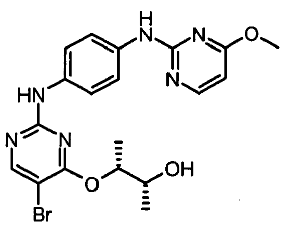 | MS: 461 (ES) | Variante B III |
| 23 | 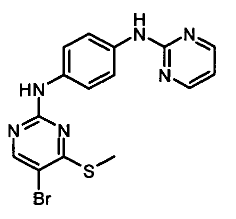 | | Intermediat VIII.1 |
| 24 | 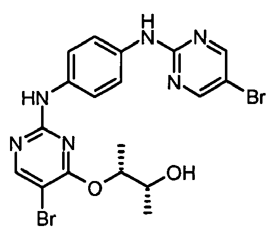 | MS :509/511/513 (ES) | Variante B III |
| 25 | 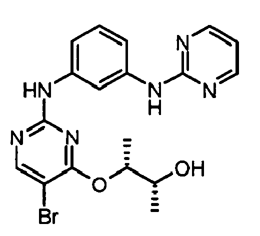 | MS : 431 (ES) | Variante B III |
| 26 | 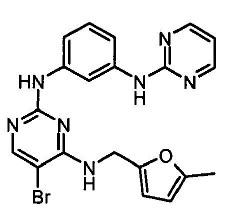 | MS: 452 (ES+) | Variante B III |
| 27 | 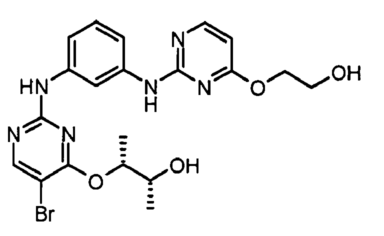 | MS: 491 (ES) | Variante B III |
| 28 | 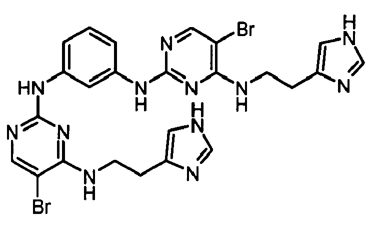 | | Variante B I |
| 29 | 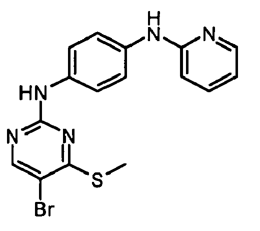 | | Intermediat VIII.2 |
| 30 | 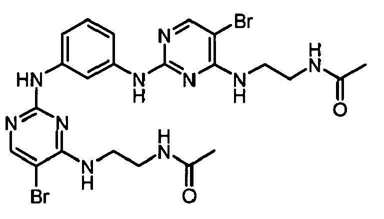 | | Variante B I |
| 31 | 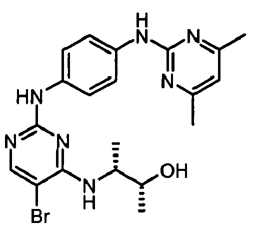 | MS: 458 (ES+) | Variante B II |
| 32 | 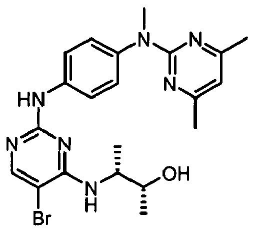 | MS: 472 (ES+) | Variante B II |
| 33 | 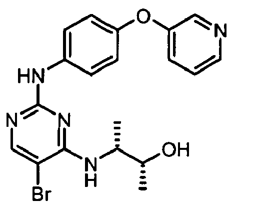 | MS: 430 (ES+) | Variante B II |
| 34 | 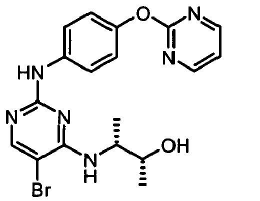 | MS: 431 (ES+) | Variante B II |
| 35 | 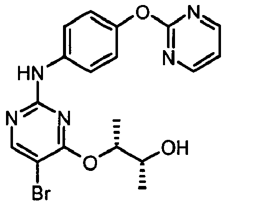 | MS: 432 (ES+) | Variante B II |
| 36 | 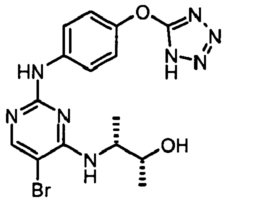 | MS: 421 (ES+) | Variante B II |
| 37 | 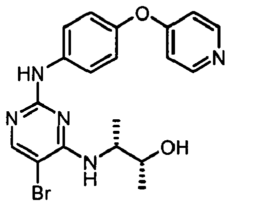 | MS: 430 (ES+) | Variante B II |
| 38 | 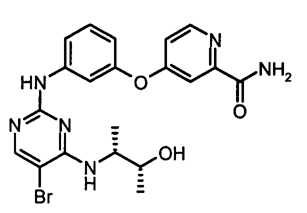 | MS: 473 (ES+) | Variante B II |
| 39 | 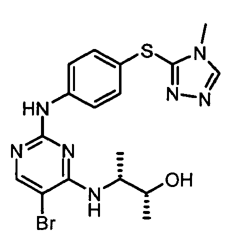 | MS: 450 (ES+) | Variante B II |
| 40 | 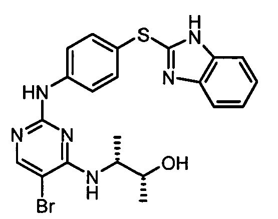 | MS: 485 (ES+) | Variante B II |
| 41 | 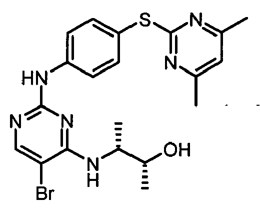 | MS: 475 (ES+) | Variante B II |
| 42 | 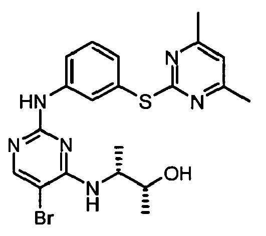 | MS: 475 (ES+) | Variante B II |
| 43 | 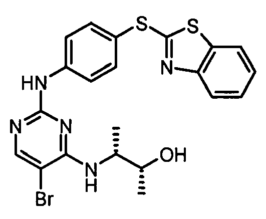 | MS: 502 (ES+) | Variante B II |

Die folgenden Verbindungen können in Analogie zu den oben genannten Beispielen hergestellt werden:

| | | | |
|---|---|---|---|
| | | | |
| 44 | 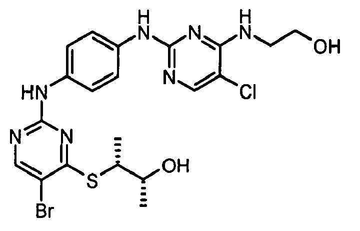 | 45 | 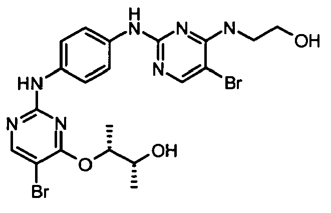 |
| | | | |
| 46 | 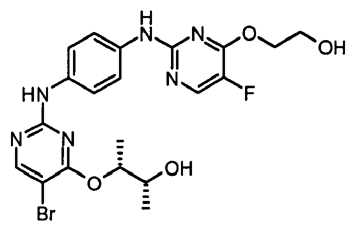 | 47 | 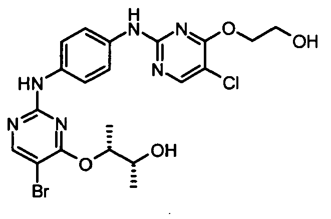 |
| | | | |
| 48 | 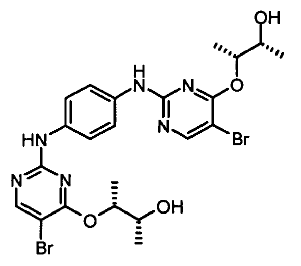 | 49 | 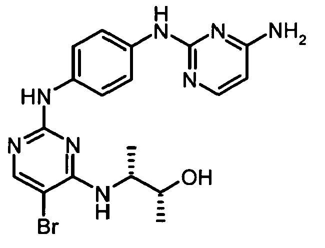 |
| | | | |
| 50 | 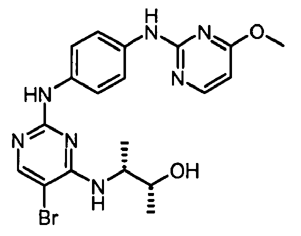 | 51 | 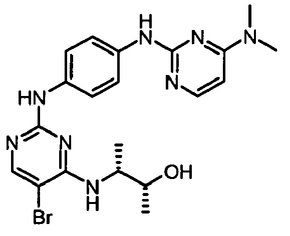 |
| | | | |
| 52 | 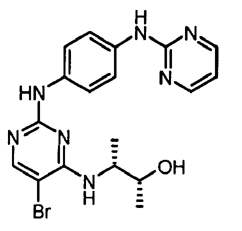 | 53 | 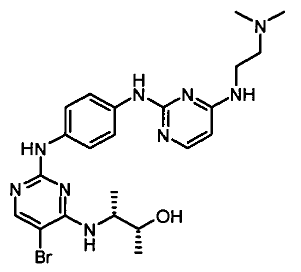 |
| | | | |
| 54 | 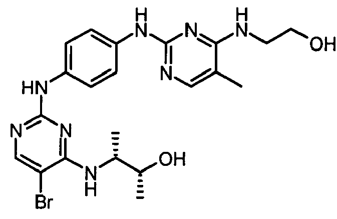 | 55 | 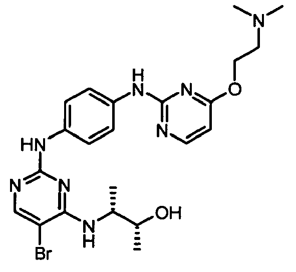 |
| | | | |
| 56 | 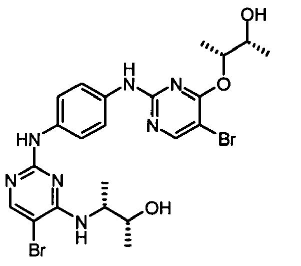 | 57 | 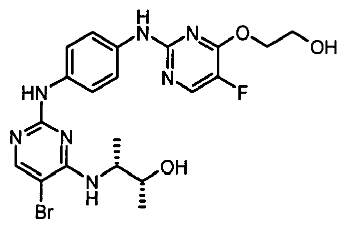 |
| | | | |
| 58 | 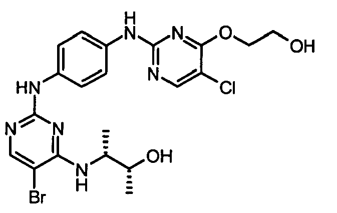 | 59 | 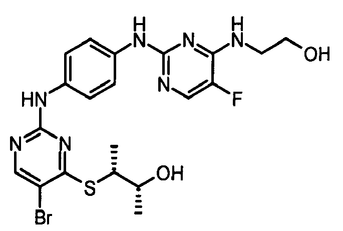 |
| | | | |
| 60 | 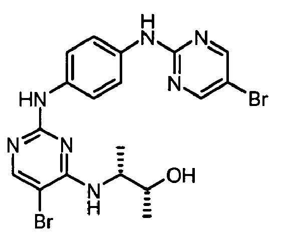 | 61 | 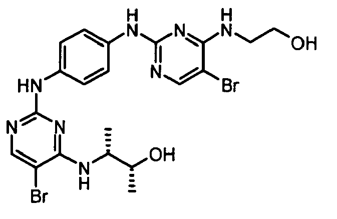 |
| | | | |
| 62 | 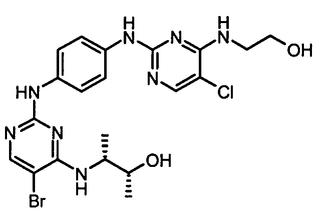 | 63 | 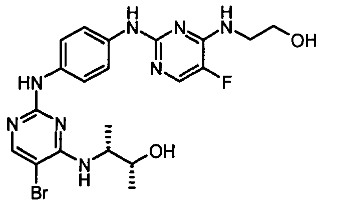 |
| | | | |
| 64 | 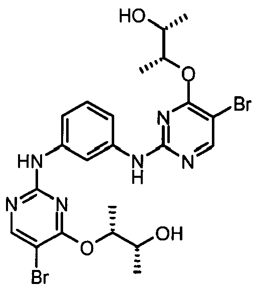 | 65 | 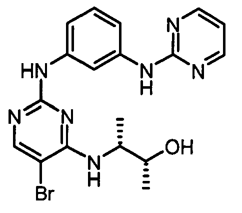 |
| | | | |
| 66 | 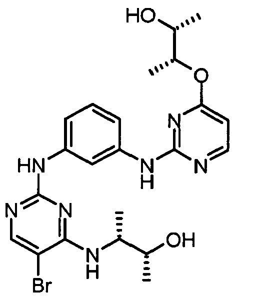 | 67 | 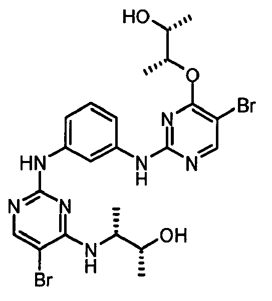 |
| | | | |
| 68 | 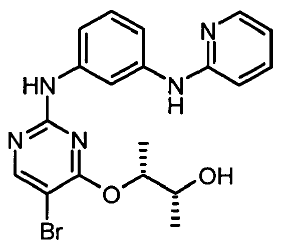 | 69 | 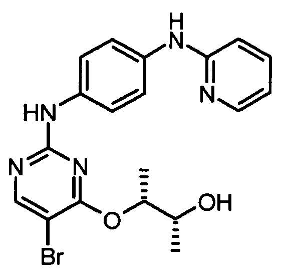 |
| | | | |
| 70 | 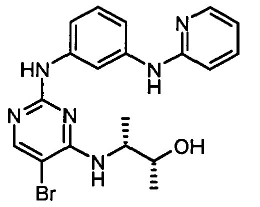 | 71 | 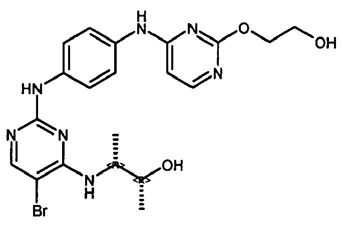 |
| | | | |
| 72 | 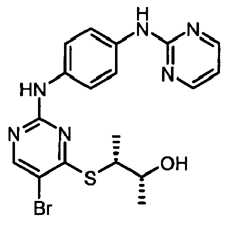 | 73 | 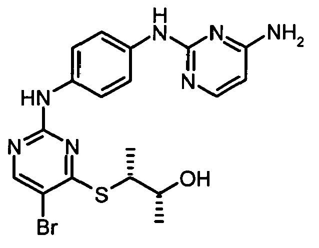 |
| | | | |
| 74 | 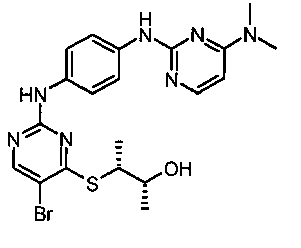 | 75 | 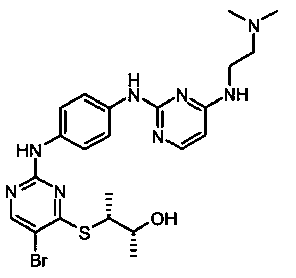 |
| | | | |
| 76 | 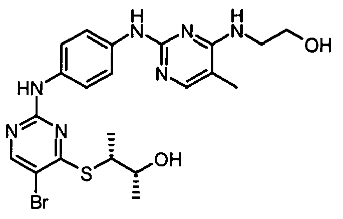 | | |
| | | | |

Der Anmeldung liegt folgende Gruppierung von Proteinkinasen zugrunde:
A. Zellzykluskinasen: a) CDKs, b) Plk, c) Aurora
B. angiogene Rezeptortyrosinkinasen: a) VEGF-R, b) Tie, c) FGF-R, d) EphB4
C. proliferative Rezeptortyrosinkinasen: a) PDGF-R, Flt-3, c-Kit
D. Kontrollpunktkinasen: a) AMT/ATR, b) Chk 1/2, c) TTK/hMps1, BubR1, Bub1
E. anti-apoptotische Kinasen a) AKT/PKB b) IKK c) PIM1, d) ILK
F. Migratorische Kinasen a) FAK, b) ROCK

### A. Zellzykluskinasen a) CDKs, b) Plk, c) Aurora

Der eukaryote Zellteilungszyklus stellt die Duplikation des Genoms und seine Verteilung auf die Tochterzellen sicher, indem er eine koordinierte und regulierte Abfolge von Ereignissen durchläuft. Der Zellzyklus wird in vier aufeinanderfolgende Phasen eingeteilt: die G1 Phase repräsentiert die Zeit vor der DNA-Replikation, in der die Zelle wächst und für externe Stimuli empfänglich ist. In der S Phase repliziert die Zelle ihre DNA, und in der G2 Phase bereitet sie sich auf den Eintritt in die Mitose vor. In der Mitose (M Phase) wird die replizierte DNA getrennt und die Zellteilung vollzogen.

Die Zyklin-abhängigen Kinasen (CDKs), eine Familie von Serin/Threonin-Kinasen, deren Mitglieder die Bindung eines Zyklins (Cyc) als regulatorische Untereinheit zu ihrer Aktivierung benötigen, treiben die Zelle durch den Zellzyklus. Unterschiedliche CDK/Cyc Paare sind in den verschiedenen Phasen des Zellzyklus aktiv. Für die grundlegende Funktion des Zellzyklus bedeutende CDK/Cyc Paare sind beispielsweise CDK4(6)/CycD, CDK2/CycE, CDK2/CycA, CDK1/CycA und CDK1/CycB.

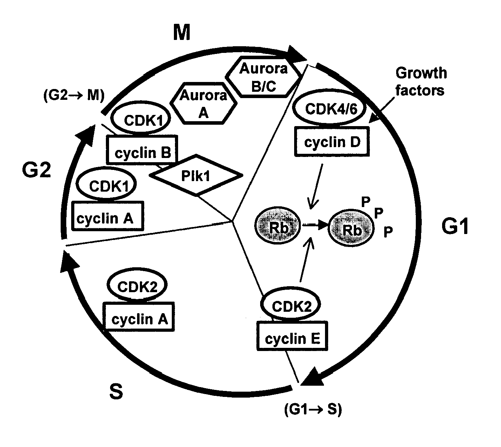

Der Eintritt in den Zellzyklus und das Durchlaufen des "Restriction Point", der die Unabhängigkeit einer Zelle von weiteren Wachstumssignalen für den Abschluss der begonnenen Zellteilung markiert, werden durch die Aktivität der CDK4(6)/CycD und CDK2/CycE Komplexe kontrolliert. Das wesentliche Substrat dieser CDK-Komplexe ist das Retinoblastoma-Protein (Rb), das Produkt des Retinoblastoma Tumorsuppressor Gens. Rb ist ein transkriptionelles Ko-Repressor Protein. Neben anderen noch weitgehend unverstandenen Mechanismen, bindet und inaktiviert Rb Transkriptionsfaktoren vom E2F-Typ, und bildet transkriptionelle Repressorkomplexe mit Histon-Deacetylasen (HDAC) (Zhang H.S. et al. (2000). Exit from G1 and S phase of the cell cycle is regulated by repressor complexes containing HDAC-Rb-hSWI/SNF and Rb-hSWI/SNF. Cell 101, 79-89). Durch die Phosphorylierung des Rb durch CDKs werden gebundene E2F Transkriptionsfaktoren freigesetzt und führen zu transkriptioneller Aktivierung von Genen, deren Produkte für die DNA Synthese und die Progression durch die S-Phase benötigt werden. Zusätzlich bewirkt die Rb-Phosphorylierung die Auflösung der Rb-HDAC Komplexe, wodurch weitere Gene aktiviert werden. Die Phosphorylierung von Rb durch CDKs ist mit dem Überschreiten des "Restriction Point" gleichzusetzen. Für die Progression durch die S-Phase und deren Abschluss ist die Aktivität der CDK2/CycE und CDK2/CycA Komplexe notwendig. Nach vollständiger Replikation der DNA steuert die CDK1 im Komplex mit CycA oder CycB das Durchlaufen der Phase G2 und den Eintritt der Zelle in die Mitose (Abb. 1). Beim Übergang von der G2 Phase in die Mitose trägt die Polo-like Kinase Ptk1 zur Aktivierung der CDK1 bei. Während des Ablaufs der Mitose ist PIk1 weiterhin an der Maturierung der Zentrosomen, dem Aufbau des Spindelapparates, der Separierung der Chromosomen und der Trennung der Tochterzellen beteiligt.

Die Familie der Aurora Kinasen besteht im humanen Organismus aus drei Mitgliedern: Aurora-A, Aurora-B und Aurora-C. Die Aurora Kinasen regulieren wichtige Prozesse während der Zellteilung (Mitose).
Aurora-A ist an den Zentrosomen und den Spindelmikrotubuli lokalisiert, wo es verschiedene Substratproteine phosphoryliert, unter anderem das Kinesin Eg5, TACC, PP1. Die genauen Mechanismen der Genese des Spindelapparates und der Rolle von Aurora-A dabei sind allerdings noch weitgehend unklar.
Aurora-B ist Teil eines Multiprotein Komplexes, der an der Zentrosomenstruktur der Chromosomen lokalisiert ist und neben Aurora-B u.a. INCENP, Survivin und Borealin/Dasra B enthält (zusammenfassende Übersicht in: Vagnarelli & Earnshaw, Chromosomal passengers: the four-dimensional regulation of mitotic events. Chromosoma. 2004 Nov;113(5):211-22. Epub 2004 Sep 4). Die Kinaseaktivität von Aurora-B stellt sicher, dass vor der Teilung der Chromosomenpaare alle Verbindungen zum Mikrotubulin-Spindelapparat korrekt sind (sogenannter Spindel Checkpoint). Substrate von Aurora-B sind hier unter anderem Histon H3 und MCAK. Nach Trennung der Chromosomen ändert Aurora-B seine Lokalisation und kann während der letzten Mitosephase (Zytokinese) an der noch verbliebenen Verbindungsbrücke zwischen den beiden Tochterzellen gefunden werden. Durch Phosphorylierung seiner Substrate MgcRacGAP, Vimentin, Desmin, der leichten regulatorischen Kette von Myosin, und anderen, reguliert Aurora-B die Abschnürung der Tochterzellen.
Aurora-C ist von seiner Aminosäuresequenz, Lokalisation, Substratspezifität und Funktion Aurora-B sehr ähnlich (Li X et al. Direct association with inner centromere protein (INCENP) activates the novel chromosomal passenger protein, Aurora-C. J Biol Chem. 2004 Nov 5;279(45):47201-11. Epub 2004 Aug 16;, Chen et al. Overexpression of an Aurora-C kinase-deficient mutant disrupts the Aurora-B/INCENP complex and induces polyploidy. J Biomed Sci. 2005;12(2):297-310; Yan X et al. Aurora-C is directly associated with Survivin and required for cytokinesis. Genes to ells 2005 10, 617-626). Der Hauptunterschied zwischen Aurora-B und
Aurora-C ist die starke Überexpression von Aurora-C im Hoden (Tseng TC et al. Protein kinase profile of sperm and eggs: cloning and characterization of two novel testis-specific protein kinases (AIE1, AIE2) related to yeast and fly chromosome segregation regulators. DNA Cell Biol. 1998 Oct;17(10):823-33.).

Die essentielle Funktion der Aurora Kinasen bei der Mitose macht sie zu interessanten Zielproteinen für die Entwicklung kleiner inhibitorischer Moleküle zur Behandlung von Krebs oder anderen Erkrankungen, die Störungen der Zellproliferation zur Ursache haben. Überzeugende experimentelle Daten deuten darauf hin, dass eine Inhibition der Aurora-Kinasen in vitro und in vivo das Fortschreiten zellulärer Proliferation verhindert und den programmierten Zelltod (Apoptose) auslöst. Dies konnte mittels (1) siRNA Technologie (Du & Hannon. Suppression of p160ROCK bypasses cell cycle arrest after Aurora-A/STK15 depletion. Proc Natl Acad Sci USA. 2004 Jun 15;101(24):8975-80. Epub 2004 Jun 3; Sasai K et al. Aurora-C kinase is a novel chromosomal passenger protein that can complement Aurora-B kinase function in mitotic cells. Cell Motil Cytoskeleton. 2004 Dec;59(4):249-63) oder (2) Überexpression einer dominant negativen Aurora Kinase (Honda et al. Exploring the functional interactions between Aurora B, INCENP, and survivin in mitosis. Mol Biol Cell. 2003 Aug;14(8):3325-41. Epub 2003 May 29), sowie (3) mit kleinen chemischen Molekülen gezeigt werden, die spezifisch Aurora Kinasen inhibieren (Hauf S et al. The small molecule Hesperadin reveals a role for Aurora B in correcting kinetochore-microtubule attachment and in maintaining the spindle assembly checkpoint. J Cell Biol. 2003 Apr 28;161(2):281-94. Epub 2003 Apr 21.; Ditchfield C et al. Aurora B couples chromosome alignment with anaphase by targeting BubR1, Mad2, and Cenp-E to kinetochores. J Cell Biol. 2003 Apr 28;161 (2):267-80.).
Die Inaktivierung von Aurora Kinasen führt dazu, dass (1) der mitotische Spindelapparat nicht oder fehlerhaft ausgebildet wird (vorwiegend bei Aurora-A Inhibition) und /oder dass (2) durch Blockierung des Spindel Checkpoints keine oder eine fehlerhafte Trennung der Schwesterchromatiden statt findet (vorwiegend bei Aurora-B/-C Inhibition) und / oder dass (3) die Trennung der Tochterzellen nicht vollzogen wird (vorwiegend bei Aurora-B/-C Inhibition). Diese Folgen (1-3) der Inaktivierung von Aurora Kinasen im einzelnen oder als Kombinationen führen schließlich zu Aneuploidie und / oder Polyploidie und letzlich sofort oder nach wiederholten Mitosen zu einem nicht lebensfähigen Zustand bzw. zum programmierten Zelltod der proliferierenden Zellen (mitotische Katastrophe).

Spezifische Kinaseinhibitoren können den Zellzyklus in unterschiedlichen Stadien beeinflussen. So ist beispielsweise von einem CDK4 oder einem CDK2 Inhibitor eine Blockade des Zellzyklus in der G1 Phase bzw. im Übergang von der G1 Phase zu der S-Phase zu erwarten.

### B. Angiogene Rezeptortyrosinkinasen

Rezeptortyrosinkinasen und deren Liganden sind in entscheidender Weise bei einer Vielzahl von zellulären Prozessen beteiligt, die in der Regulation des Wachstums und der Differenzierung von Zellen involviert sind. Von besonderem Interesse sind hier das Vascular Endothelial Growth Factor (VEGF) / VEGF-Rezeptor System, das Fibroblast Growth Factor (FGF) / FGF Rezeptor System, das Eph-Ligand / Eph-Rezeptor System, und das Tie-Ligand / Tie-Rezeptor System. In pathologischen Situationen, die mit einer verstärkten Neubildung von Blutgefäßen (Neovaskularisierung) einher gehen, wie z.B. Tumorerkrankungen, wurde eine erhöhte Expression von angiogenen Wachstumsfaktoren und ihrer Rezeptoren gefunden. Inhibitoren des VEGF / VEGF-Rezeptor Systems, FGF / FGF-Rezeptor Systems (Rousseau et al., The tyrp1-Tag/tyrp1-FGFR1-DN bigenic mouse: a model for selective inhibition of tumor development, angiogenesis, and invasion into the neural tissue by blockade of fibroblast growth factor receptor activity. Cancer Res. 64, :2490, 2004), des EphB4 Systems (Kertesz et al., The soluble extracellular domain of EphB4 (sEphB4) antagonizes EphB4-EphrinB2 interaction, modulates angiogenesis and inhibits tumor growth. Blood. 2005 Dec 1; [Epub ahead of print]), sowie des Tie-Ligand / Tie Systems (Siemeister et al., Two independent mechanisms essential for tumor angiogenesis: inhibition of human melanoma xenograft growth by interfering with either the vascular endothelial growth factor receptor pathway or the Tie-2 pathway. Cancer Res. 59, 3185, 1999) können die Ausbildung eines Blutgefäßsystems in Tumoren inhibieren, damit den Tumor von der Sauerstoff- und Nährstoffversorgung abschneiden und somit das Tumorwachstum inhibieren.

### C. Proliferative Rezeptortyrosinkinasen

Rezeptortyrosinkinasen und deren Liganden sind in entscheidender Weise bei der Proliferation von Zellen beteiligt. Von besonderem Interesse sind hier das Platelet-Derived Growth Factor (PDGF) Ligand / PDGF-Rezeptor System, c-Kit Ligand / c-Kit Rezeptor System und das FMS-like Tyrosinkinase 3 (Flt-3) Ligand / Flt-3 System. In pathologischen Situationen, die mit einem verstärkten Wachstum von Zellen einher gehen, wie z.B. Tumorerkrankungen, wurde eine erhöhte Expression von proliferativen Wachstumsfaktoren und ihrer Rezeptoren oder die Kinase aktivierende Mutationen gefunden. Die Inhibition der Enzymaktivität dieser Rezeptortyrosinkinasen führt zu einer Reduktion des Tumorwachstums. Dies konnte z.B. durch Studien mit dem kleinen chemischen Molekül ST1571/Glivec gezeigt werden, welches unter anderem PDGF-R und c-Kit inhibiert (zusammenfassende Übersichten in: Oestmann A., PDGF receptors - mediators of autocrine tumor growth and regulators of tumor vasculature and stroma, Cytokine Growth Factor Rev. 2004 Aug;15(4):275-86; Roskoski R., Signaling by Kit protein-tyrosine kinase - the stem cell factor receptor. Biochem Biophys Res Commun. 2005 Nov 11;337(1):1-13.; Markovic A. et al., FLT-3: a new focus in the understanding of acute leukemia. Int J Biochem Cell Biol. 2005 Jun;37(6):1168-72: Epub 2005 Jan 26.).

### E. Kontrollpunktkinasen

Unter Kontrollpunktkinasen (= Checkpoint-Kinasen) im Sinne der vorliegenden Anmeldung sind Zellzykluskinasen zu verstehen, die den geordneten Ablauf der Zellteilung überwachen, wie beispielsweise ATM und ATR, Chk1 und Chk2, Mps1, Bub1 und BubR1. Von besonderer Bedeutung sind der DNA-Schädigungs Checkpoint in der G2 Phase und der Spindel-Checkpoint während der Mitose.

Die Aktivierung der ATM, ATR, Chk1 und Chk2 Kinasen erfolgt nach DNA-Schädigung einer Zelle und führt zu einem Arrest des Zellzyklus in der G2 Phase durch Inaktivierung der CDK1. (Chen & Sanchez, Chk1 in the DNA damage response: conserved roles from yeasts to mammals. DNA Repair 3, 1025, 2004). Inaktivierung von Chk1 verursacht den Verlust des durch DNA Schädigung induzierten G2 Arrest, zur Zellzyklusprogression der Zelle in Anwesenheit geschädigter DNA, und führt schließlich zum Zelltod (Takai et al. Aberrant cell cycle checkpoint function and early embryonic death in Chk1 (-/-) mice.Genes Dev. 2000 Jun 15;14(12):1439-47; Koniaras et al. Inhibition of Chk1-dependent G2 DNA damage checkpoint radiosensitizes p53 mutant human cells. Oncogene. 2001 Nov 8;20(51):7453-63.; Liu et al. Chk1 is an essential kinase that is regulated by Atr and required for the G(2)/M DNA damage checkpoint. Genes Dev. 2000 Jun 15;14(12):1448-59.). Die Inaktivierung von Chk1, Chk2 oder Chk1 und Chk2 verhindert den durch DNA-Schädigung verursachten G2 Arrest und macht proliferierende Krebszellen empfindlicher gegenüber DNA-schädigende Therapien wie z.B. Chemotherapie oder Radiotherapie. Chemotherapien, die zur DNA Schädigung führen, sind z.B. DNA-Strangbruch induzierende Substanzen, DNAalkylierende Substanzen, Topoisomerase Inhibitoren, Aurora Kinase Inhibitoren, Substanzen, die den Aufbau der mitotischen Spindel beeinflussen, hypoxischer Stress aufgrund limitierter Sauerstoffversorgung eines Tumors (z.B. induziert durch anti-angiogene Medikamente wie VEGF Kinase Inhibitoren).

Ein zweiter wesentlicher Checkpoint innerhalb der Zellzyklus kontrolliert den korrekten Aufbau und Anhaftung des Spindelapparates an die Chromosomen während der Mitose. An diesem sogenannten Spindel-Checkpoint sind die Kinasen TTK/hMps1, Bub1, und BubR1 beteiligt (zusammenfassende Übersicht in: Kops et al. On the road to cancer: aneuploidy and the mitotic checkpoint. Nat Rev Cancer. 2005 Oct; 5(10):773-85). Diese sind an Kinetochoren von noch nicht an den Spindelapparat angehefteter kondensierter Chromosomen lokalisiert und inhibieren den sogenannten anaphase-promoting complex/cyclosome (APC/C). Erst nach vollständiger und korrekter Anheftung des Spindelapparates an die Kinetochoren werden die Spindel-Checkpoint Kinasen Mps-1, Bub1, und BubR1 inaktiviert, wodurch APC/C aktiviert wird und es zur Trennung der gepaarten Chromosomen kommt. Eine Inhibition der Spindel-Checkpointkinasen führt zur der Trennung der gepaarten Chromosomen bevor alle Kinetochoren an den Spindelapparat angeheftet sind und in Folge zu chromosomalen Fehlverteilungen, die von den Zellen nicht toleriert werden und schließlich zum Zellzyklusstillstand oder zum Zelltod führen.

### F. Anti-apoptotische Kinasen

Verschiedene Mechanismen schützen eine Zelle gegenüber dem Zelltod während nicht optimaler Lebensbedingungen. In Tumorzellen führen diese Mechanismen zu einem Überlebensvorteil der Zellen in der wachsenden Tumormasse, die durch den Mangel an Sauerstoff, Glukose und weiteren Nährstoffen gekennzeichnet ist, ermöglichen ein Überleben von Tumorzellen ohne Anheftung an die extrazelluäre Matrix was zur Metastasierung führen kann, oder führen zu Resistenzen gegenüber Therapeutika. Wesentliche anti-apoptotische Signalwege umfassen den PDK1-AKT/PKB Signalweg (Altomare & Testa. Perturbations of the AKT signaling pathway in human cancer. Oncogene. 24, 7455, 2005), den NFkappaB Signalweg (Viatour et al. Phosphorylation of NFkB and IkB proteins: implications in cancer and inflammation), den Pim1 Signalweg (Hammerman et al. Pim and Akt oncogenes are independent regulators of hematopoietic cell growth and survival. Blood. 2005 105, 4477, 2005) und den integrin-linked kinase (ILK) Signalweg (Persad & Dedhar. The role of integrin-linked kinase (ILK) in cancer progression. Cancer Met. Rev. 22, 375, 2003). Durch die Inhibition der anti-apoptotischen Kinasen, wie beispielsweise AKT/PBK, PDK1, IkappaB Kinase (IKK), Pim1, oder ILK, werden die Tumorzellen gegenüber der Wirkung von Therapeutika oder gegenüber ungünstigen Lebensbedingungen in der Tumorumgebung sensitiviert. Tumorzellen werden nach Inhibition der anti-apoptotischen Kinasen empfindlicher auf durch Aurora-Inhibition verursachte Störungen der Mitose reagieren und vermehrt dem Zelltod unterliegen.

### G. Migratorische Kinasen

Invasives, Gewebe-infiltrierendes Tumorwachstum und Metastasierung setzt voraus, daß die Tumorzellen den Gewebeverband durch Migration verlassen können. Verschiedene zelluläre Mechanismen sind in die Regulation der Zellmigration involviert: Integrin-vermittelte Adhäsion an Proteine der extrazellulären Matrix reguliert über die Aktivität der focal adhesion kinase (FAK); Kontrolle des Zusammenbaus der kontraktilen Aktin-Filamente über den RhoA / Rho-Kinase (ROCK) Signalweg (zusammenfassende Übersicht in M.C. Frame, Newest findings on the oldest oncogene; how activated src does it. J. Cell Sci. 117, 989, 2004).

Die erfindungsgemäßen Verbindungen wirken zum Beispiel
- gegen Krebs, wie solide Tumore, Tumor- oder Metastasenwachstum, insbesondere: Ataxia-telangiectasia, Basalzellkarzinom, Blasenkarzinom, Gehirntumor, Brustkrebs, Cervix Karzinom, Tumoren des Zentralnervensystems, Kolorektalkarzinom, Endometriales Karzinom, Magenkarzinom, Gastrointestinales Karzinom, Kopf- und Halstumore, Akute lymphozytische Leukämie, Akute myelogene Leukämie, Chronische lymphozytische Leukämie, Chronische myelogene Leukämie, Haarzell Leukämie, Leberkarzinom, Lungentumor, Nicht-kleinzelliges Lungenkarzinom, Kleinzelliges Lungenkarzinom, B-Zell Lymphom, Hodgkin's Lymphom, Non-Hodgkin's Lymphom, T-Zell Lymphom, Melanom, Mesotheliom, Myelom, Myom, Tumore des Oesophagus, orale Tumore, Ovarialkarzinom, Pankreastumore, Prostatatumore, Nierenkarzinom, Sarkom, Kaposi's Sarkom, Leiomyosarkom, Hautkrebs, Plattenzellkarzinom, Hodenkrebs, Schilddrüsenkrebs, Bindegewebstumor des Magen-Darmgewebes, Bindegewebssarkom der Haut, Hypereosinophiles Syndrom, Mastzellenkrebs,
- bei kardiovaskulären Erkrankungen wie Stenosen, Arteriosklerosen und Restenosen, Stent-induzierte Restenose,
- bei Angiofibrom, Crohn-Krankheit, Endometriose, Hämangioma.

Die Formulierung der erfindungsgemäßen Verbindungen zu pharmazeutischen Präparaten erfolgt in an sich bekannter Weise, indem man den oder die Wirkstoffe mit den in der Galenik gebräuchlichen Hilfsstoffen in die gewünschte Applikationsform überführt.

Als Hilfsstoffe können dabei beispielsweise Trägersubstanzen, Füllstoffe, Sprengmittel, Bindemittel, Feuchthaltemittel, Gleitmittel, Ab- und Adsorptionsmittel, Verdünnungsmittel, Lösungsmittel, Cosolventien, Emulgatoren, Lösungsvermittler, Geschmackskorrigentien, Färbemittel, Konservierungs-, Stabilisierungs-, Netzmittel, Salze zur Veränderung des osmotischen Drucks oder Puffer zum Einsatz kommen.

Dabei ist auf Remington's Pharmaceutical Science, 15th ed. Mack Publishing Company, East Pennsylvania (1980) hinzuweisen.

Die pharmazeutischen Formulierungen können
in fester Form, zum Beispiel als Tabletten, Dragees, Pillen, Suppositorien, Kapseln, transdermale Systeme oder
in halbfester Form , zum Beispiel als Salben, Cremes, Gele, Suppositiorien, Emulsionen oder
in flüssiger Form, zum Beispiel als Lösungen, Tinkturen, Suspensionen oder Emulsionen vorliegen.

Hilfsstoffe im Sinne der Erfindung können beispielsweise Salze, Saccharide (Mono-, Di-, Tri-, Oligo-, und/oder Polysaccharide), Proteine, Aminosäuren, Peptide, Fette, Wachse, Öle, Kohlenwasserstoffe sowie deren Derivate sein, wobei die Hilfsstoffe natürlichen Ursprungs sein können oder synthetisch bzw. partial synthetisch gewonnen werden können.

Für die orale oder perorale Applikation kommen insbesondere Tabletten, Dragees, Kapseln, Pillen, Pulver, Granulate, Pastillen, Suspensionen, Emulsionen oder Lösungen in Frage.
Für die parenterale Applikation kommen insbesondere Suspensionen, Emulsionen und vor allem Lösungen in Frage.

### Beschreibung der Assays

### Assay 1

### Aurora-C-Kinaseassay

Die Aurora-C-inhibitorische Aktivität der Substanzen dieser Erfindung wurde in dem in den folgenden Absätzen beschriebenen Aurora-C-HTRF-Assay gemessen (HTRF *= Homogeneous Time Resolved Fluorescence).*
Rekombinantes Fusionsprotein aus GST und humanem Aurora-C wurde in transient transfizierten HEK293-Zellen exprimiert und durch Affinitätschromatographie an Glutathion-Sepharose gereinigt. Als Substrat für die Kinasereaktion wurde das biotinylierte Peptid Biotin-Ttds-FMRLRRLSTKYRT (C-Terminus in Amid-Form) verwendet, das z.B. bei der Firma JERINI Peptide Technologies (Berlin) gekauft werden kann.
Aurora-C wurde für 60 min bei 22°C in Anwesenheit verschiedener Konzentrationen an Testsubstanzen in 5 µL Assaypuffer [25 mM Hepes/NaOH pH 7.4, 0.5 mM MnCl₂, 2.0 mM Dithiothreitol, 0.1 mM Natriumorthovanadat, 10 µM Adenosine-tri-phosphate (ATP), 0.5 µM/ml Substrat, 0.01% (v/v) TritonX-100 (Sigma), 0.05 % (w/v) Bovines Serumalbumin (BSA), 1 % (v/v) Dimethylsulfoxid] inkubiert. Die Konzentration des Aurora-C wurde an die jeweilige Aktivität des Enzyms angepasst und so eingestellt, dass der Assay im linearen Bereich arbeitete. Typische Konzentrationen lagen im Bereich von 0.3 nM. Die Reaktion wurde gestoppt durch Zugabe von 5 µl einer Lösung von HTRF-Detektionsreagentien (0.2 µM Streptavidin-XLent and 1.4 nM Anti-Phospho-(Ser/Thr)-Akt-Substrat-Eu-Kryptat (Cis biointernational, Frankreich, Produkt Nr. 61 P02KAE), einem mit Europium-Kryptat-markiertem Phospho-(Ser/Thr)-Akt-Substrat-Antikörper [Produkt #9611 B, Cell Signaling Technology, Danvers, MA, USA]) in wässriger EDTA-Lösung (40 mM EDTA, 400 mM KF, 0.05 % (w/v) Bovines Serumalbumin (BSA) in 25 mM HEPES/NaOH pH 7.0).

Die resultierende Mischung wurde 1 h bei 22°C inkubiert, um die Bildung eines Komplexes aus dem biotinylierten phosphorylierten Substrat und den Detektionsreagentien zu ermöglichen. Anschließend wurde die Menge des phosphorylierten Substrates ausgewertet durch eine Messung des Resonanz-Energietransfers vom Anti-Phospho-(Ser/Thr)-Akt-Substrat-Eu-Kryptat zum Streptavidin-XLent. Hierzu wurden in einem HTRF-Meßgerät, z.B. einem Rubystar (BMG Labtechnologies, Offenburg, Germany) oder einem Viewlux (Perkin-Elmer), die Fluoreszenz-Emissionen bei 620 nm and 665 nm nach Anregung bei 350 nm gemessen. Das Verhältnis der Emissionen bei 665 nm und at 622 nm wurde als Maß für die Menge des phosphorylierten Substrates genommen. Die Daten wurden normalisiert (Enzymreaktion ohne Inhibitor = 0 % Inhibition, alle anderen Assaykomponente aber kein Enzym = 100 % Inhibition) und 1C50-Werte wurden kalkuliert mit einem 4-Parameter-Fit, wofür eine inhouse-Software verwendet wurde.

### Assay 2

### CDK1/CycB Kinase Assay

Rekombinante CDK1- und CycB-GST-Fusionsproteine, gereinigt aus Bakulovirus-infizierten Insektenzellen (Sf9), wurden von ProQinase GmbH, Freiburg, gekauft. Das als Kinase-Substrat verwendet Histon IIIIS ist über die Fa. Sigma käuflich zu erwerben.
CDK1/CycB (5 ng/µL) wurde für 10 min bei 22°C in Anwesenheit verschiedener Konzentrationen an Testsubstanzen (0 µM, sowie innerhalb des Bereiches 0,01 - 100 µM) in 40 µL Assaypuffer [50 mM Tris/HCI pH8,0, 10 mM M₉Cl₂, 0,1 mM Na ortho-Vanadat, 1,0 mM Dithiothreitol, 0.025% PEG 20000, 0,5 µM ATP, 1 µM Histon IIIIS, 0,2 µCi/Messpunkt ³³P-gamma ATP, 0,05% NP40, 1,25% Dimethylsulfoxid] inkubiert. Die Reaktion wurde durch Zugabe von EDTA-Lösung (250 mM, pH8,0, 15 µl/Messpunkt) gestoppt.
Von jedem Reaktionsansatz wurden 15 µl auf P30 Filterstreifen (Fa. Wallac) aufgetragen, und nicht-eingebautes ³³P-ATP wurde durch dreimaliges Waschen der Filterstreifen für je 10 min in 0,5%iger Phosphorsäure entfernt. Nach dem Trocknen der Filterstreifen für 1 Stunde bei 70°C wurden die Filterstreifen mit Szintillator-Streifen (MeltiLex™ A, Fa. Wallac) bedeckt und für 1 Stunde bei 90°C eingebrannt. Die Menge an eingebautem ³³P (Substratphosphorylierung) wurde durch Szintillationsmessung in einem Gamma-Strahlungsmessgerät (Wallac) bestimmt. Die Messdaten wurden normalisiert auf 0% Inhibition (Enzymreaktion ohne Inhibitor) und 100% Inhibition (alle Assaykomponenten außer Enzym). Die Bestimmung der IC50 Werte erfolgte mittels eines 4-Parameter Fits unter Benutzung firmeneigener Software.

### Assay 3

### CDK2/CycE-Kinaseassay

Die CDK2/CycE-inhibitorische Aktivität der Substanzen dieser Erfindung wurde in dem in den folgenden Absätzen beschriebenen CDK2/CycE-HTRF-Assay gemessen (HTRF = *Homogeneous Time Resolved Fluorescence).*
Rekombinante CDK2-GST und CycE-GST-Fusionsproteine, gereinigt aus Bakulovirus-infizierten Insektenzellen (Sf9), wurde von ProQinase GmbH, Freiburg, gekauft. Als Substrat für die Kinasereaktion wurde das biotinylierte Peptid Biotin-Ttds-YISPLKSPYKISEG (C-Terminus in Amid-Form) verwendet, das z.B. bei der Firma JERINI Peptide Technologies (Berlin) gekauft werden kann.
CDK2/CycE wurde für 60 min bei 22°C in Anwesenheit verschiedener Konzentrationen an Testsubstanzen in 5 µL Assaypuffer [50 mM Tris/HCl pH 8.0, 10 mM MgCl₂, 1.0 mM Dithiothreitol, 0.1 mM Natriumorthovanadate, 10 µM Adenosine-tri-phosphat (ATP), 0.75 µM Substrat, 0.01% (v/v) Nonidet-P40 (Sigma), 1 % (v/v) Dimethylsulfoxid] inkubiert. Die Konzentration des CDK2/CycE wurde an die jeweilige Aktivität des Enzyms angepasst und so eingestellt, dass der Assay im linearen Bereich arbeitete. Typische Konzentrationen lagen im Bereich von 1 ng/ml. Die Reaktion wurde gestoppt durch Zugabe von 5 µl einer Lösung von HTRF-Detektionsreagentien (0.2 µM Streptavidin-XLent and 3.4 nM Phospho-(Ser) CDKs Substrate Antibody [Produkt #2324B, Cell Signaling Technology, Danvers, MA, USA] und 4 nM Prot-A-EuK [Protein A markiert mit Europium-Kryptat von Cis biointernational, Frankreich, Produkt-Nr. 61PRAKLB]) in wässriger EDTA-Lösung (100 mM EDTA, 800 mM KF, 0.2 % (w/v) Bovines Serumalbumin (BSA) in 100 mM HEPES/NaOH pH 7.0).

Die resultierende Mischung wurde 1 h bei 22°C inkubiert, um die Bildung eines Komplexes aus dem biotinylierten phosphorylierten Substrat und den Detektionsreagentien zu ermöglichen. Anschließend wurde die Menge des phosphorylierten Substrates ausgewertet durch eine Messung des Resonanz-Energietransfers vom Prot-A-EuK zum Streptavidin-XLent. Hierzu wurden in einem HTRF-Meßgerät, z.B. einem Rubystar (BMG Labtechnologies, Offenburg, Germany) oder einem Viewlux (Perkin-Elmer), die Fluoreszenz-Emissionen bei 620 nm and 665 nm nach Anregung bei 350 nm gemessen. Das Verhältnis der Emissionen bei 665 nm und at 622 nm wurde als Maß für die Menge des phosphorylierten Substrates genommen. Die Daten wurden normalisiert (Enzymreaktion ohne Inhibitor = 0 % Inhibition, alle anderen Assaykomponente aber kein Enzym = 100 % Inhibition) und IC50-Werte wurden kalkuliert mit einem 4-Parameter-Fit, wofür eine inhouse-Software verwendet wurde.

### Assay 4

### KDR kinase assay

Die KDR-inhibitorische Aktivität der Substanzen dieser Erfindung wurde in dem in folgenden Absätzen beschriebenen KDR-HTRF-Assay gemessen (HTRF = *Homogeneous Time Resolved Fluorescence).*

Rekombinantes KDR-Kinase-GST-Fusionsprotein, gereinigt aus Bakulovirus-infizierten Insektenzellen (Sf9), wurde von ProQinase GmbH, Freiburg, gekauft. Als Substrat für die Kinasereaktion wurde das biotinylierte Peptid Biotin-Ahx-DFGLARDMYDKEYYSVG (C-Terminus in Säureform) verwendet, das z.B. bei der Firma Biosynthan GmbH (Berlin-Buch, Germany) gekauft werden kann.

KDR-Kinase wurde für 45 min bei 22°C in Anwesenheit verschiedener Konzentrationen an Testsubstanzen in 5 µL Assaypuffer [50 mM Hepes/NaOH pH 7.0, 25 mM MgCl₂, 5 mM MnCl₂, 1.0 mM Dithiothreitol, 0.1 mM Natriumorthovanadat, 10 µM Adenosine-tri-phosphate (ATP), 0.5 µM Substrat, 0.001 % (v/v) Nonidet-P40 (Sigma), 1 % (v/v) Dimethylsulfoxid] inkubiert. Die Konzentration des KDR wurde an die jeweilige Aktivität des Enzyms angepasst und so eingestellt, dass der Assay im linearen Bereich arbeitete. Die Reaktion wurde gestoppt durch Zugabe von 5 µl einer Lösung von HTRF-Detektionsreagentien (0.1 µM Streptavidin-XLent und 2 nM PT66-Eu-Chelat, ein Europiumchelat-markierter anti-Phospho-Tyrosin Antikörper von Perkin Elmer) in wässriger EDTA-Lösung (125 mM EDTA, 0.2 % (w/v) Bovines Serumalbumin (BSA) in 50 mM HEPES/NaOH pH 7.0).

Die resultierende Mischung wurde 1 h bei 22°C inkubiert, um eine Bindung des biotinylierten phosphorylierten Substrates an das Streptavidin-XLent und das PT66-Eu-Chelat zu ermöglichen. Anschließend wurde die Menge des phosphorylierten Substrates ausgewertet durch eine Messung des Resonanz-Energietransfers vom PT66-Eu-Chelat zum Streptavidin-XLent. Hierzu wurden in einem HTRF-Meßgerät, z.B. einem Rubystar (BMG Labtechnologies, Offenburg, Germany) oder einem Viewlux (Perkin-Elmer), die Fluoreszenz-Emissionen bei 620 nm and 665 nm nach Anregung bei 350 nm gemessen. Das Verhältnis der Emissionen bei 665 nm und at 622 nm wurde als Maß für die Menge des phosphorylierten Substrates genommen. Die Daten wurden normalisiert (Enzymreaktion ohne Inhibitor = 0 % Inhibition, alle anderen Assaykomponente aber kein Enzym = 100 % Inhibition) und IC50-Werte wurden kalkuliert mit einem 4-Parameter-Fit, wofür eine inhouse-Software verwendet wurde.

### Assay 5

### MCF7 Proliferationsassay

Kultivierte humane MCF7 Brusttumorzellen (ATCC HTB-22) wurden in einer Dichte von 5000 Zellen/Meßpunkt in einer 96-well Multititerplatte in 200 µl Wachstumsmediums (RPM11640, 10% Fötales Kälberserum, 2 mU/mL Insulin, 0,1 nM Östradiol) ausplattiert. Nach 24 Stunden wurden die Zellen einer Platte (Nullpunkt-Platte) mit Kristallviolett gefärbt (s.u.), während das Medium der anderen Platten durch frisches Kulturmedium (200 µl), dem die Testsubstanzen in verschiedenen Konzentrationen (0 µM, sowie im Bereich 0,01 - 30 µM; die finale Konzentration des Lösungsmittels Dimethylsulfoxid betrug 0,5%) zugesetzt waren, ersetzt. Die Zellen wurden für 4 Tage in Anwesenheit der Testsubstanzen inkubiert. Die Zellproliferation wurde durch Färbung der Zellen mit Kristallviolett bestimmt: Die Zellen wurden durch Zugabe von 20 µl/Meßpunkt einer 11 %igen GlutaraldehydLösung 15 min bei Raumtemperatur fixiert. Nach dreimaligem Waschen der fixierten Zellen mit Wasser wurden die Platten bei Raumtemperatur getrocknet. Die Zellen wurden durch Zugabe von 100 µl/Meßpunkt einer 0,1 %igen Kristallviolett-Lösung (pH durch Zugabe von Essigsäure auf pH3 eingestellt) gefärbt. Nach dreimaligem Waschen der gefärbten Zellen mit Wasser wurden die Platten bei Raumtemperatur getrocknet. Der Farbstoff wurde durch Zugabe von 100 µl/Meßpunkt einer 10%igen Essigsäure-Lösung gelöst, und die Extinktion wurde photometrisch bei einer Wellenlänge von 595 nm bestimmt. Die prozentuale Änderung des Zellwachstums wurde durch Normalisierung der Meßwerte auf die Extinktionwerte der Nullpunktplatte (=0%) und die Extinktion der unbehandelten (0 µM) Zellen (=100%) berechnet. Die Bestimmung der IC50 Werte erfolgte mittels eines 4-Parameter Fits unter Benutzung firmeneigener Software.

### Inhibitorische Wirkung der erfindungsgemäßen Verbindungen

Die Verbindungen folgender Beispiele wurden in den verschiedenen Kinase-Assays hinsichtlich ihrer inhibitorischen Wirkung getestet, sowie in der MCF7-Krebszelllinie bezüglich ihrer wachstumshemmenden Wirkung (Tab. 1)

**Tabelle 1:**

| Beispiel Nr | CDK1 [M] | CDK2 [M] | Aurora C [M] | KDR [M] | MCF7 [M] |
|---|---|---|---|---|---|
| 8 | 2,8E-08 | 2,8E-09 | 2E-07 | 4,4E-08 | 3E-08 |
| 15 | 2,7E-08 | 2,9E-09 | 1,4E-07 | 7,4E-08 | 8,9E-08 |
| 16 | 2,3E-08 | 2E-09 | 4,2E-08 | 8,3E-08 | 4,2E-07 |
| 17 | 7,1 E-08 | 4,4E-09 | 1 E-07 | 6,2E-08 | 9,8E-08 |
| 18 | 4E-08 | 5,9E-09 | 7,5E-08 | 1,4E-07 | 1,7E-07 |
| 19 | 5,1 E-08 | 2,2E-09 | 9,1 E-08 | 1 E-07 | 2,9E-07 |
| 21 | 5,4E-08 | 2,2E-09 | 1,8E-07 | 7,3E-08 | 9,1 E-08 |
| 22 | 1,3E-07 | 4,8E-09 | 2,7E-07 | 1,2E-07 | 1,3E-07 |
| 24 | 1,1E-07 | 6,2E-09 | 1,3E-07 | 2,4E-07 | 1 E-07 |
| 25 | 1,4E-08 | ..,2E-09 | 3,4E-07 | 7,5E-08 | 2,7E-07 |
| 27 | 1,6E-08 | 2,4E-09 | 4,4E-07 | 3,2E-08 | 3,3E-08 |
| 28 | | 6,7E-07 | 1,2E-07 | 2,3E-08 | |
| 29 | >1 E-06 | 2,6E-07 | 8,1 E-06 | > 0,00002 | > 3E-06 |
| 30 | | 5,8E-06 | 5,3E-08 | 2,1 E-08 | 2E-06 |
| 33 | 7,8E-08 | 6,6E-09 | 3,8E-07 | 5E-08 | 2,7E-07 |
| 34 | 3, 5 E-08 | 9,1 E-09 | 4, 9 E-07 | 1 E-07 | |
| 39 | 1,1E-08 | 1,3E-09 | 3,5E-07 | 4,1E-08 | |
| 41 | 9,8E-08 | 6E-08 | | 1,4E-06 | 8E-07 |
| 44 | > 1 E-06 | 3,7E-07 | 1 E-06 | 7,3E-06 | > 3 E-06 |

## Patentansprüche

1. Verbindungen der allgemeinen Formel (I) mit den Bausteinen A, B, C und D, 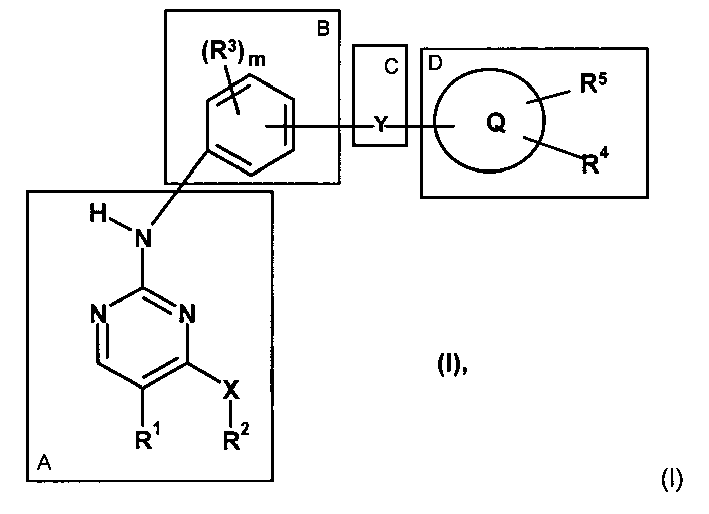 in der
R¹ für
(i) Wasserstoff, Halogen, Cyano, Nitro, -NR⁸R⁹, -NR⁷-C(O)-R¹², -NR⁷-C(O)-OR¹², -NR⁷-C(O)-NR⁸R⁹, -NR⁷-SO₂-, -CF₃ oder -OCF₃, oder
(ii) einen gegebenenfalls mit Hydroxy, -NR⁸R⁹, -NR⁷-C(O)-R¹², -NR⁷-C(O)-OR¹², -NR⁷-C(O)-NR⁸R⁹, -NR⁷-SO₂-R¹², Cyano, Halogen, C₁-C₆-Alkoxy, -CF₃ und/oder -OCF₃ ein- oder mehrfach, gleich oder verschieden substituierten C₁-C₆-Alkyl-, C₂-C₆-Alkenyl-, C₁-C₆-Alkoxy- oder C₂-C₆-Alkinylrest, oder
(iii) einen gegebenenfalls mit Hydroxy, -NR⁸R⁹, -NR⁷-C(O)-R¹², -NR⁷-C(O)-OR¹², -NR⁷-C(O)-NR⁸R⁹, -NR⁷-SO₂-R¹², Cyano, Halogen, -CF₃, C₁-C₆-Alkoxy, -OCF₃ und/oder C₁-C₆-Alkyl ein- oder mehrfach, gleich oder verschieden substituierten Phenyl- oder monocyclischen Heteroarylring steht,
R² für
(i) Wasserstoff oder
(ii) einen C₁-C₁₀-Alkyl-, C₂-C₁₀-Alkenyl- oder C₂-C₁₀-Alkinylrest, einen C₃-C₇-Cycloalkyl- oder einen Heterocyclylring mit 3 bis 8 Ringatomen steht,
jeweils gegebenenfalls ein- oder mehrfach, gleich oder verschieden substituiert mit
a) Halogen, Hydroxy, -NR⁸R⁹, -NR⁷-C(O)-R¹², -NR⁷-C(O)-OR¹², -NR⁷-C(O)-NR⁸R⁷, -NR⁷-SO₂-R¹², Cyano, -C(O)R⁶, -O(CO)-R¹², -SO₂NR⁸R⁹, -SO₂-R¹², -S(O)(NR⁸)R¹², -(N)S(O)R¹³R¹⁴, -CF₃, -OCF₃, -N[(CO)-(C₁-C₆-Alkyl)]₂ und/oder
b) C₁-C₆-Alkoxy, C₁-C₆-Alkyl, C₂-C₆-Alkenyl, C₂-C₆-Alkinyl, C₃-Cₛ-Cycloalkyl, Heterocyclyl mit 3 bis 8 Ringatomen und/oder einem monocyclischen oder bicyclischen Heteroaryl, jeweils gegebenenfalls selbst ein- oder mehrfach, gleich oder verschieden mit Halogen, Hydroxy, einem C₁-C₆-Alkyl, C₁-C₆-Alkoxy, -NR⁸R⁹, -C(O)OR¹⁶, -SO₂NR⁸R⁹, -CF₃ oder -OCF₃ substituiert,
R³ für
(i) Hydroxy, Halogen, Cyano, Nitro, -CF₃, -OCF₃, -C(O)NR⁸R⁹, -C(S)NR⁸R⁹, -NR⁸R⁹, -NR⁷-C(O)-R¹², -NR⁷-C(O)-OR¹², -NR⁷-C(O)-NR⁸R⁹, -NR⁷-SO₂-R¹², und/oder
(ii) einen gegebenenfalls mit Halogen, Hydroxy, C₁-C₆-Alkoxy, -CF₃, -OCF₃ oder -NR⁸R⁹ ein- oder mehrfach, gleich oder verschieden substituierten C₁-C₆-Alkyl- und/oder C₁-C₆-Alkoxyrest, und/oder
(iii) einen gegebenenfalls mit Halogen, Hydroxy, C₁-C₆-Alkoxy, -CF₃, -OCF₃, -NR⁸R⁹ und/oder C₁-C₆-Alkyl ein- oder mehrfach, gleich oder verschieden substituierten C₃-C₇-Cycloalkylring
steht,
m für 0-3 steht,
R⁴ und R⁵ unabhängig voneinander für
(i) Wasserstoff, -NR⁸R⁹, -OR⁸, Halogen, -(CO)-NR⁸R⁹ und/oder
(ii) einen C₁-C₆-Alkyl-, C₂-C₆-Alkenyl-, C₂-C₆-Alkinylrest, einen C₃-C₇-Cycloalkyl- oder Phenylring, einen Heterocyclylring mit 3 bis 8 Ringatomen oder einen monocyclischen Heteroarylring stehen, jeweils gegebenenfalls selbst mit Hydroxy, -NR⁸R⁹, Cyano, Halogen, -CF₃, C₁-C₆-Alkoxy, -OCF₃ und/oder C₁-C₆-Alkyl ein- oder mehrfach, gleich oder verschieden substituiert,
X und Y unabhängig voneinander für -O-, -S-, -S(O)-, -S(O)₂- oder-NR¹⁵- stehen,
wobei,
R¹⁵ für
(i) Wasserstoff oder
(ii) einen C₁-C₆-Alkylrest, C₃-C₈-Cycloalkyl- oder einen Heterocyclylring mit 3 bis 8 Ringatomen oder
(iii) -C(O)-(C₁-C₆)-Alkyl, -C(O)-Phenyl, oder -C(O)-Benzyl steht,
und (ii) und (iii) gegebenenfalls mit Hydroxy,
-NR¹⁰R¹¹, Cyano, Halogen, -CF₃, C₁-C₆-Alkoxy und/oder
-OCF₃ ein- oder mehrfach, gleich oder verschieden substituiert sind, oder
wenn X für -NR¹⁵- steht, alternativ
-NR¹⁵- und R² gemeinsam einen 3 bis 8 gliedrigen Ring bilden, der gegebenenfalls zusätzlich zum Stickstoffatom ein oder mehrere weitere Heteroatome enthält, gegebenenfalls ein- oder mehrfach, gleich oder verschieden mit Hydroxy, C₁-C₆-Alkyl, C₁-C₆-Alkoxy, -C(O)R¹², -SO₂R¹², Halogen oder der Gruppe-NR⁸R⁹ substituiert ist, gegebenenfalls 1 bis 3 Doppelbindungen enthält und/oder gegebenenfalls durch eine oder mehrere -C(O)-Gruppen unterbrochen ist,
Q für einen monocyclischen oder bicyclischen Heteroarylring steht,
R⁶ für
(i) Wasserstoff oder Hydroxy, oder
(ii) einen C₁-C₆-Alkyl-, C₃-C₆-Alkenyl-, C₃-C₆-Alkinyl- oder C₁-C₆-Alkoxyrest, einen C₃-C₇-Cycloalkyl- oder Phenylring, einen Heterocyclylring mit 3 bis 8 Ringatomen oder einen monocyclischen Heteroarylring steht, jeweils gegebenenfalls selbst mit Hydroxy, -NR⁸R⁹, Cyano, Halogen, -CF₃, C₁-C₆-Alkoxy und/oder -OCF₃ ein- oder mehrfach, gleich oder verschieden substituiert,
R⁷ für Wasserstoff oder einen C₁-C₆-Alkylrest steht,
R⁸ und R⁹ unabhängig voneinander für
(i) Wasserstoff und/oder
(ii) einen C₁-C₆-Alkylrest, C₂-C₆-Alkenyl-, C₃-C₈-Cycloalkyl- und/oder Phenylring, einen Heterocyclylring mit 3 bis 8 Ringatomen und/oder einen monocyclischen Heteroarylring, gegebenenfalls mit Hydroxy; -NR¹⁰R¹¹, -NR⁷-C(O)-R¹², Cyano, Halogen, -CF₃, C₁-C₆-Alkoxy und/oder -OCF₃ ein- oder mehrfach, gleich oder verschieden substituiert sind,
oder
R⁸ und R⁹ gemeinsam mit dem Stickstoffatom einen 3- bis 8-gliedrigen Ring bilden, der gegebenenfalls zusätzlich zum Stickstoffatom 1 oder 2 weitere Heteroatome enthält und der mit Hydroxy, -NR¹⁰R¹¹, Cyano, Halogen, -CF₃, C₁-C₆-Alkoxy und/oder -OCF₃ ein- oder mehrfach, gleich oder verschieden substituiert sein kann,
R¹⁰und R¹¹ unabhängig voneinander für Wasserstoff oder einen C₁-C₆-Alkylrest stehen, der gegebenenfalls mit Hydroxy, Cyano, Halogen, -CF₃, C₁-C₆-Alkoxy und/oder -OCF₃ ein- oder mehrfach, gleich oder verschieden substituiert ist.
R¹² , R¹³ R¹⁴ unabhängig voneinander für einen C₁-C₆-Alkyl, C₂-C₆-Alkenyl- und/oder C₂-C₆-Alkinylrest, einen C₃-C₇-Cycloalkyl- oder Phenylring , einen Heterocyclylring mit 3 bis 8 Ringatomen oder einen monocyclischen Heteroarylring steht, gegebenenfalls jeweils selbst mit Hydroxy, Nitro, -NR⁸R⁹, Cyano, Halogen, -CF₃, C₁-C₆-Alkyl, C₁-C₆-Alkoxy und/oder -OCF₃ ein- oder mehrfach, gleich oder verschieden substituiert,
R¹⁶ für
(i) Wasserstoff oder
(ii) einen C₁-C₆-Alkyl-, C₃-C₆-Alkenyl-, C₃-C₆-Alkinylrest, einen C₃-C₇-Cycloalkyl- oder Phenylring, einen Heterocyclylring mit 3 bis 8 Ringatomen oder einen monocyclischen Heteroarylring steht, jeweils gegebenenfalls selbst mit Hydroxy, -NR⁸R⁹, Cyano, Halogen, -CF₃, C₁-C₆-Alkoxy und/oder -OCF₃ ein- oder mehrfach, gleich oder verschieden substituiert,
sowie deren Salze, Diastereomere und Enantiomere.

2. Verbindungen gemäß Anspruch 1 der allgemeinen Formel (I), in der
R¹ für Halogen, -CF₃, -OCF₃, C₁-C₄-Alkyl oder Nitro steht,
R² für einen C₁-C₁₀-Alkyl-, C₂-C₁₀-Alkenyl- oder C₂-C₁₀-Alkinylrest, einen C₃-C₇-Cycloalkyl- oder einen Heterocyclylring mit 3 bis 7 Ringatomen steht, jeweils gegebenenfalls ein oder mehrfach, gleich oder verschieden substituiert mit Hydroxy, -NR⁸R⁹, -NR⁷-C(O)-R¹², -NR⁷-C(O)-OR¹², -NR⁷-C(O)-NR⁸R⁹oder einem monocyclischen oder bicyclischen Heteroaryl, der gegebenenfalls selbst ein- oder mehrfach substituiert ist mit Hydroxy oder einem C₁-C₆-Alkylrest.
R³ für
(i) Hydroxy, Halogen, Cyano, Nitro, -CF₃, -OCF₃, -C(O)NR⁸R⁹, -C(S)NR⁸R⁹, -NR⁸R⁹, -NR⁷-C(O)-R¹², -NR⁷-C(O)-OR¹², -NR⁷-C(O)-NR⁸R⁹oder -NR⁷ -SO₂-R¹² und/oder
(ii) einen gegebenenfalls mit Halogen, Hydroxy, C₁-C₆-Alkoxy, -CF₃, -OCF₃ oder -NR⁸R⁹ ein- oder mehrfach, gleich oder verschieden substituierten C₁-C₅-Alkyl- und/oder C₁-C₅-Alkoxyrest, steht
m für 0-3 steht,
R⁴ und R⁵ unabhängig voneinander stehen für
(i) Wasserstoff, -NHR⁸, -OR⁸, Halogen, -(CO)-NR⁸R⁹ und/oder
(ii) einen C₁-C₄-Alkyl-, C₃-C₅-Alkenyl-, C₃-C₅-Alkinylrest oder einen C₃-C₆-Cycloalkylring, jeweils gegebenenfalls selbst mit Hydroxy, -NR⁸R⁹, Cyano, Halogen, -CF₃, C₁-C₆-Alkoxy, -OCF₃ und/oder C₁-C₆-Alkyl ein- oder mehrfach, gleich oder verschieden substituiert
X und Y unabhängig voneinander stehen für
-O-, -S-, -S(O)- , -S(O)₂- oder -NR¹⁵-, wobei
R¹⁵ für Wasserstoff oder einen C₁-C₆-Alkylrest, C₃-C₈-Cycloalkyl- oder einen Heterocyclylring mit 3 bis 8 Ringatomen steht, jeweils gegebenenfalls ein- oder mehrfach, gleich oder verschieden substituiert mit Hydroxy, -NR¹⁰R¹¹, Cyano, Halogen, -CF₃, C₁-C₆-Alkoxy und/oder-OCF₃, oder
wenn X für -NR¹⁵- steht,
-NR¹⁵- und R² bevorzugt alternativ gemeinsam einen 3 bis 6 gliedrigen Ring bilden, der gegebenenfalls zusätzlich zum Stickstoffatom ein weiteres Heteroatom enthält, gegebenenfalls ein- oder mehrfach, gleich oder verschieden mit Hydroxy, C₁-C₆-Alkyl, C₁-C₆-Alkoxy, -C(O)R¹², -SO₂R¹², Halogen oder der Gruppe-NR⁸R⁹ substituiert ist, gegebenenfalls 1 oder 2 Doppelbindungen enthält und/oder durch eine -C(O)-Gruppe unterbrochen ist.
Q für einen monocyclischen oder bicyclischen Heteroarylring steht,
R⁶ für
(i) Wasserstoff oder
(ii) einen C₁-C₄-Alkyl-, C₃-C₅-Alkenyl-, C₃-C₅-Alkinyl- oder C₁-C₅-Alkoxyrest, einen C₃-C₆-Cycloalkyl- oder Phenylring, einen Heterocyclylring mit 3 bis 6 Ringatomen oder einen monocyclischen Heteroarylring steht, jeweils gegebenenfalls selbst mit Hydroxy,-NR⁸R⁹, Cyano, Halogen, -CF₃, C₁-C₆-Alkoxy und/oder -OCF₃ ein- oder mehrfach, gleich oder verschieden substituiert.
R⁷ für Wasserstoff steht,
R⁸ und R⁹ unabhängig voneinander stehen für
Wasserstoff und/oder einen C₁-C₅-Alkyl-, C₂-C₅-Alkenylrest, einen C₃-C₇-Cycloalkyl- und/oder Phenylring und/oder einen monocyclischen Heteroarylring, jeweils gegebenenfalls mit Hydroxy, -NR¹⁰R¹¹, -NR⁷-C(O)-R¹² und/oder C₁-C₆-Alkoxy ein- oder mehrfach, gleich oder verschieden substituiert, oder
R⁸ und R⁹ bilden gemeinsam mit dem Stickstoffatom einen 5- bis 7-gliedrigen Ring, der gegebenenfalls zusätzlich zum Stickstoffatom 1 weiteres Heteroatom enthält und der mit Hydroxy, -NR¹⁰R¹¹und/oder C₁-C₆-Alkoxy ein- oder mehrfach, gleich oder verschieden substituiert sein kann.
R¹⁰und R¹¹ unabhängig voneinander stehen für Wasserstoff oder einen C₁-C₆-Alkylrest, der gegebenenfalls mit Hydroxy, Halogen oder C₁-C₆-Alkoxy ein- oder mehrfach, gleich oder verschieden substituiert ist,.
R¹² für einen C₁-C₆-Alkyl, C₂-C₆-Atkenyl- oder C₂-C₆-Alkinylrest, einen C₃-C₇-Cycloalkyl- oder Phenylring, einen Heterocyclylring mit 3 bis 8 Ringatomen oder einen monocyclischen Heteroarylring steht jeweils gegebenenfalls selbst mit Hydroxy, Halogen, Nitro, -NR⁸R⁹, C₁-C₆-Alkyl und/oder C₁-C₆-Alkoxy ein- oder mehrfach, gleich oder verschieden substituiert.
R¹³ und R¹⁴ unabhängig voneinander stehen für einen C₁-C₆-Alkyl, C₂-C₆-Alkenyl- und/oder C₂-C₆-Alkinylrest, einen C₃-C₇-Cycloalkyl- und/oder Phenylring, einen Heterocyclylring mit 3 bis 8 Ringatomen und/oder einen monocyclischen Heteroarylring, jeweils gegebenenfalls selbst mit Hydroxy,-NR⁸R⁹ und/oder C₁-C₆-Alkoxy ein- oder mehrfach, gleich oder verschieden substituiert.
R¹⁶ für einen C₁-C₆-Alkyl-, C₃-C₆-Alkenyl-, C₃-C₆-Alkinylrest, einen C₃-C₇-Cycloalkyl- oder Phenylring, einen Heterocyclylring mit 3 bis 8 Ringatomen oder einen monocyclischen Heteroarylring steht, jeweils gegebenenfalls selbst mit Hydroxy, -NR⁸R⁹, Cyano, Halogen, -CF₃, C₁-C₆-Alkoxy und/oder -OCF₃ ein- oder mehrfach, gleich oder verschieden substituiert,
sowie deren Salze, Diastereomere und Enantiomere.

3. Verbindungen der Formel (I) gemäß einem der Ansprüche 1 oder 2, in der R¹ für Halogen, -CF₃ oder C₁-C₂-Alkyl steht,
sowie deren Salze, Diastereomere und Enantiomere.

4. Verbindungen der Formel (I) gemäß einem der Ansprüche 1 bis 3, in der
R² für einen C₁-C₈-Alkyl-, C₂-C₈-Alkenyl- oder C₂-C₈-Alkinylrest, einen C₃-C₆-Cycloalkyl- oder einen Heterocyclylring mit 3 bis 5 Ringatomen steht, jeweils gegebenenfalls ein oder mehrfach, gleich oder verschieden substituiert mit Hydroxy, -NR⁸R⁹, -NR⁷-C(O)-R¹², -NR⁷-C(O)-OR¹², -NR⁷-C(O)-NR⁸R⁹ oder einem monocyclischen Heteroaryl, der gegebenenfalls selbst ein- oder mehrfach substituiert ist mit Hydroxy oder einem C₁-C₅-Alkylrest,
sowie deren Salze, Diastereomere und Enantiomere.

5. Verbindungen der Formel (I) gemäß einem der Ansprüche 1 bis 4, in der
X für -O- oder -NR¹⁵- steht, wobei,
R¹⁵ für Wasserstoff oder einen C₃-C₆-Alkylrest, C₃-C₇-Cycloalkyl- oder einen Heterocyclylring mit 3 bis 6 Ringatomen steht, jeweils gegebenenfalls ein- oder mehrfach, gleich oder verschieden substituiert mit Hydroxy, -NR¹⁰R¹¹, Cyano, Halogen, -CF₃, C₁-C₆-Alkoxy und/oder -OCF₃,
oder
wenn X für -NR¹⁵- steht,
-NR¹⁵- und R² alternativ gemeinsam einen 5 oder 6 gliedrigen Ring bilden, der gegebenenfalls zusätzlich zum Stickstoffatom ein weiteres Heteroatom enthält und der gegebenenfalls ein- oder mehrfach, gleich oder verschieden mit Hydroxy, C₁-C₆-Alkyl, C₁-C₆-Alkoxy,-C(O)R¹², -SO₂R¹², Halogen oder der Gruppe -NR⁸R⁹ substituiert ist,
sowie deren Salze, Diastereomere und Enantiomere.

6. Verbindungen der Formel (I) gemäß einem der Ansprüche 1 bis 5, in der
R⁴ und R⁵ unabhängig voneinander stehen für Wasserstoff, einen C₁-C₃-Alkylrest, -NR⁸R⁹, -OR⁸ oder Halogen, wobei R⁸ und R⁹ unabhängig voneinander für Wasserstoff, einem monocyclischen Heteroarylring oder einen C₁-C₆-Alkylrest stehen, die gegebenenfalls ein- oder mehrfach, gleich oder verschieden substituiert sind mit Hydroxy, - NR¹⁰R¹¹ oder -NR⁷-C(O)-R¹²,
sowie deren Salze, Diastereomere und Enantiomere.

7. Verbindungen der Formel (I) gemäß einem der Ansprüche 1 bis 6, in der
R⁶ für einen C₂-C₅-Alkyl-, C₄-C₆-Alkenyl-, C₄-C₆-Alkinyl- oder C₂-C₅-Alkoxyrest, einen C₄-C₆-Cycloalkyl- oder Phenylring, einen Heterocyclylring mit 3 bis 5 Ringatomen oder einen monocyclischen Heteroarylring steht, jeweils gegebenenfalls selbst mit Hydroxy, -NR⁸R⁹, Cyano, Halogen, -CF₃, C₁-C₆-Alkoxy und/oder -OCF₃ ein- oder mehrfach, gleich oder verschieden substituiert,
sowie deren Salze, Diastereomere und Enantiomere.

8. Verbindungen der Formel (I) gemäß einem der Ansprüche 1 bis 7, in der
Y für -O- oder -NR¹⁵- steht, wobei
R¹⁵ für Wasserstoff oder einen C₁-C₃-Alkylrest, C₃-C₇-Cycloalkyl- oder einen Heterocyclylring mit 3 bis 6 Ringatomen steht, jeweils gegebenenfalls ein- oder mehrfach, gleich oder verschieden substituiert mit Hydroxy, - NR¹⁰R¹¹, Cyano, Halogen, -CF₃, C₁-C₆-Alkoxy und/oder -OCF₃,
sowie deren Salze, Diastereomere und Enantiomere.

9. Verbindungen der Formel (I) gemäß einem der Ansprüche 1 bis 8, in der
R⁸ und R⁹ unabhängig voneinander stehen für Wasserstoff, einen monocyclischen Heteroarylring oder einen C₁-C₆-Alkylrest, die gegebenenfalls ein- oder mehrfach, gleich oder verschieden substituiert sind mit Hydroxy, -NR¹⁰R¹¹ oder -NR⁷-C(O)-R¹²,
sowie deren Salze, Diastereomere und Enantiomere.

10. Verbindungen der Formel (I) gemäß einem der Ansprüche 1 bis 9, in der
R¹⁰und R¹¹ unabhängig voneinander stehen für Wasserstoff oder einen C₁-C₄-Alkylrest, der gegebenenfalls mit Hydroxy ein- oder mehrfach, gleich oder verschieden substituiert ist,
sowie deren Salze, Diastereomere und Enantiomere.

11. Verbindungen der Formel (I) gemäß einem der Ansprüche 1 bis 10, in der
R¹² für einen C₁-C₅-Alkyl, C₂-C₅-Alkenyl-, einen C₃-C₆-Cycloalkyl- oder Phenylring, einen Heterocyclylring mit 3 bis 6 Ringatomen oder einen monocyclischen Heteroarylring steht, jeweils gegebenenfalls selbst mit Hydroxy, Halogen, Nitro, -NR⁸R⁹, C₁-C₆-Alkyl und/oder C₁-C₆-Alkoxy ein- oder mehrfach, gleich oder verschieden substituiert,
sowie deren Salze, Diastereomere und Enantiomere.

12. Verbindungen der Formel (I) gemäß einem der Ansprüche 1 bis 11, in der
R¹³ und R¹⁴ unabhängig voneinander stehen für einen C₁-C₅-Alkyl, C₂-C₅-Alkenyl- und/oder C₂-C₅-Alkinylrest, einen C₃-C₆-Cycloalkyl- und/oder Phenylring, einen Heterocyclylring mit 3 bis 6 Ringatomen und/oder einen monocyclischen Heteroarylring,
sowie deren Salze, Diastereomere und Enantiomere.

13. Verbindungen der Formel (I) gemäß einem der Ansprüche 1 bis 12, in der
m für 0 oder 1 steht,
sowie deren Salze, Diastereomere und Enantiomere.

14. Verbindungen der Formel (I) gemäß einem der Ansprüche 1 bis 13, in der R⁷ für Wasserstoff steht,
sowie deren Salze, Diastereomere und Enantiomere.

15. Verbindungen der Formel (I) gemäß einem der Ansprüche 1 bis 14, in der
R¹⁶ für einen C₁-C₆-Alkylrest, einen C₃-C₇-Cycloalkyl- oder Phenylring, einen Heterocyclylring mit 3 bis 8 Ringatomen oder einen monocyclischen Heteroarylring steht,
sowie deren Salze, Diastereomere und Enantiomere.

16. Verbindungen gemäß Anspruch 1 der allgemeinen Formel (I), in der
R¹ für Halogen oder -CF₃ steht,
R² für einen C₁-C₁₀-Alkylrest steht, der gegebenenfalls ein- oder mehrfach, gleich oder verschieden substituiert ist mit, Hydroxy, - NR⁸R⁹, -NR⁷-C(O)-R¹² und/oder einem monocyclischen Heteroaryl, gegebenenfalls selbst ein- oder mehrfach, mit einem C₁-C₆-Alkyl, substituiert,
m für 0 steht,
R⁴ und R⁵ unabhängig voneinander stehen für Wasserstoff, -NR⁸R⁹, -OR⁸, Halogen, -(CO)-NR⁸R⁹ und/oder einen C₁-C₆-Alkylrest,
X und Y unabhängig voneinander stehen für -O-, -S-, -S(O)- oder -NR¹⁵-, wobei R¹⁵ für Wasserstoff oder einen C₁-C₆-Alkylrest steht,
Q für einen monocyclischen oder bicyclischen Heteroarylring steht,
R⁷ für Wasserstoff steht,
R⁸ und R⁹ unabhängig voneinander für Wasserstoff , einen C₁-C₆-Alkylrest und/oder einen monocyclischen Heteroarylring stehen, die gegebenenfalls mit Hydroxy, -NR¹⁰R¹¹, -NR⁷-C(O)-R¹² substituiert sind,
R¹⁰ und R¹¹ unabhängig voneinander für Wasserstoff oder einen C₁-C₆-Alkylrest stehen,
R¹² für einen C₁-C₆-Alkylrest steht,
sowie deren Salze, Diastereomere und Enantiomere.

17. Verbindungen gemäß Anspruch 1 der allgemeinen Formel (I), in der
R¹ für Halogen steht,
R² für einen C₁-C₁₀-Alkylrest steht, der gegebenenfalls ein- oder mehrfach, gleich oder verschieden substituiert ist mit Hydroxy, - NR⁸R⁹, oder -NR⁷-C(O)-R¹².
m für 0 steht,
R⁴ und R⁵ unabhängig voneinander stehen für Wasserstoff, -NR⁸R⁹, -OR⁸, Halogen, -(CO)-NR⁸R⁹ und/oder einen C₁-C₆-Alkylrest,
X für -O-, oder -NR¹⁵- steht, wobei R¹⁵ für Wasserstoff oder einen C₁-C₆-Alkylrest steht,
Y für -NR¹⁵- steht, wobei R¹⁵ für Wasserstoff oder einen C₁-C₆-Alkylrest steht,
Q für einen monocyclischen oder bicyclischen Heteroarylring steht,
R⁷ für Wasserstoff steht,
R⁸ und R⁹ unabhängig voneinander für Wasserstoff , einen C₁-C₆-Alkylrest und/oder einen monocyclischen Heteroarylring stehen, die gegebenenfalls mit Hydroxy, -NR¹⁰R¹¹, -NR⁷-C(O)-R¹² substituiert sind,
R¹⁰und R¹¹ unabhängig voneinander für Wasserstoff oder einen C₁-C₆-Alkylrest stehen,
R¹² für einen C₁-C₆-Alkylrest steht,
sowie deren Salze, Diastereomere und Enantiomere.

18. Verbindungen der allgemeinen Formel I gemäß einem der vorstehenden Ansprüche 1 bis 17 zur Verwendung als Arzneimittel.

19. Verwendung von Verbindungen der allgemeinen Formel I gemäß einem der vorstehenden Ansprüche 1 bis 17 zur Herstellung eines Arzneimittels zur Behandlung von Krebs.

20. Verfahren zur Herstellung einer Verbindung gemäß einem der Ansprüche 1 bis 17, **gekennzeichnet durch** die Schritte:
a) 2,4-Dichlor-pyrimidine der Formel (X) werden mit Nucleophilen der Formel (IX) zu Verbindungen der Formel (II) umgesetzt 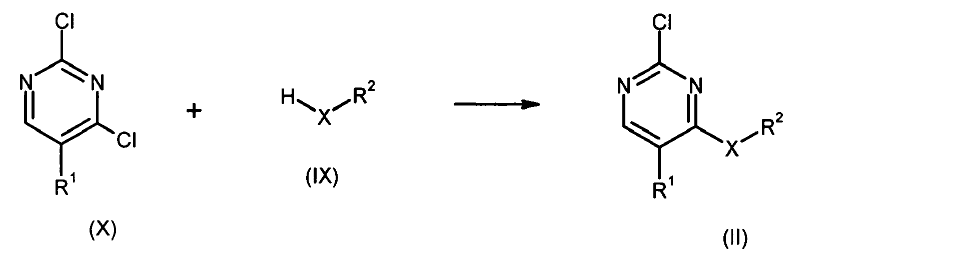
b) 2-Chlor-pyrimidine der Formel (II) werden mit Phenylen-diaminen der Formel (IIIa) zu Verbindungen der Formel (IV) umgesetzt 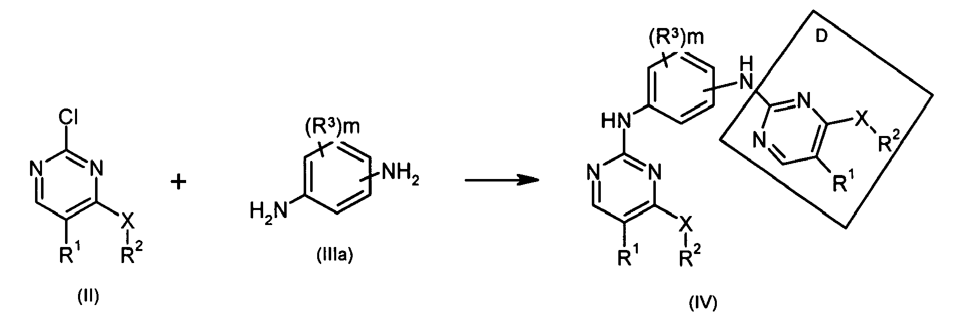
wobei die Substituenten R¹, R², R³, X und m die in der allgemeinen Formel (I) angegebenen Bedeutungen besitzen und in Baustein D der Formel (IV) X-R²die Bedeutung von R⁴ und R¹ die Bedeutung von R⁵ hat.

21. Verfahren zur Herstellung einer Verbindung gemäß einem der Ansprüche 1 bis 17, **gekennzeichnet durch** die Schritte:
a) 2,4-Dichlor-pyrimidine der Formel (X) werden mit Nucleophilen der Formel (IX) zu Verbindungen der Formel (II) umgesetzt 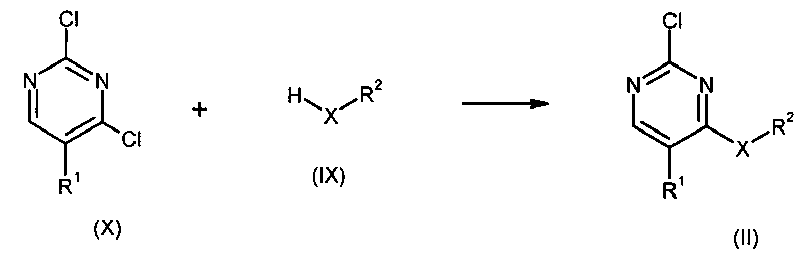
b₁) Anilinderivate der Formel III werden mit Elektrophilen der Formel VII umgesetzt 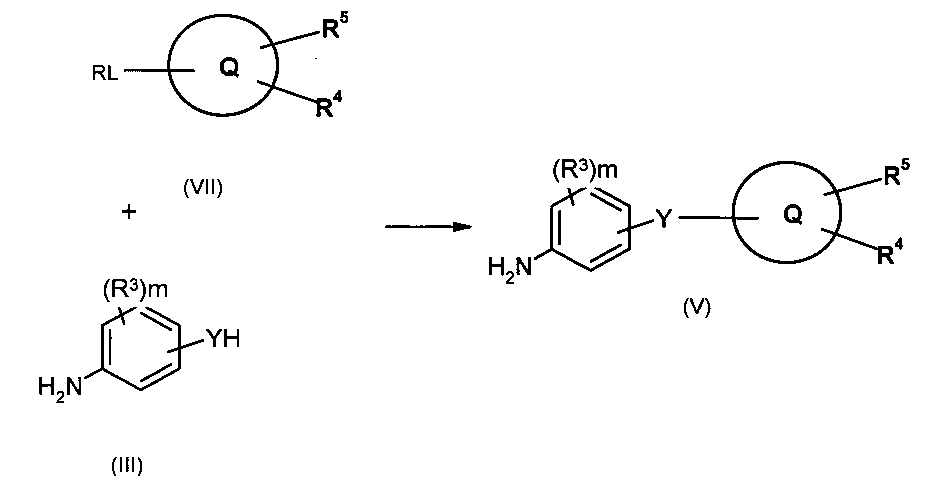
b₂) oder alternativ zu b₁) bei Verbindungen mit Y = NH werden Nitroaniline der Formel (IIIc) mit Elektrophilen der Formel (VII) umgesetzt und nachfolgend die Nitrogruppe reduziert; 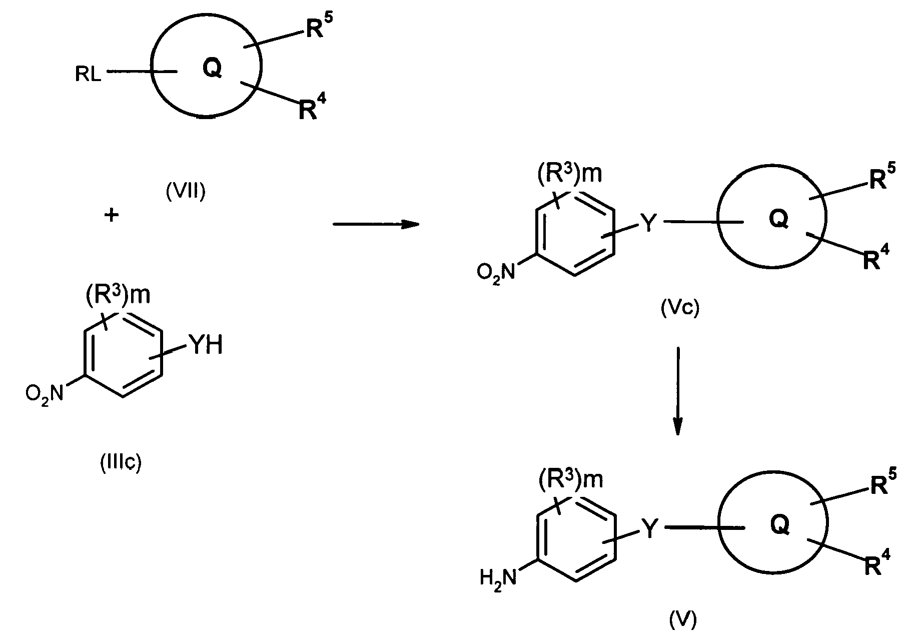
c) 2-Chlor-pyrimidine der Formel (II) aus Verfahrensschritt a) werden mit substituierten Anilinen der Formel (V) aus Verfahrensschritt b₁ oder b₂ zu Verbindungen der Formel (I) umgesetzt werden. 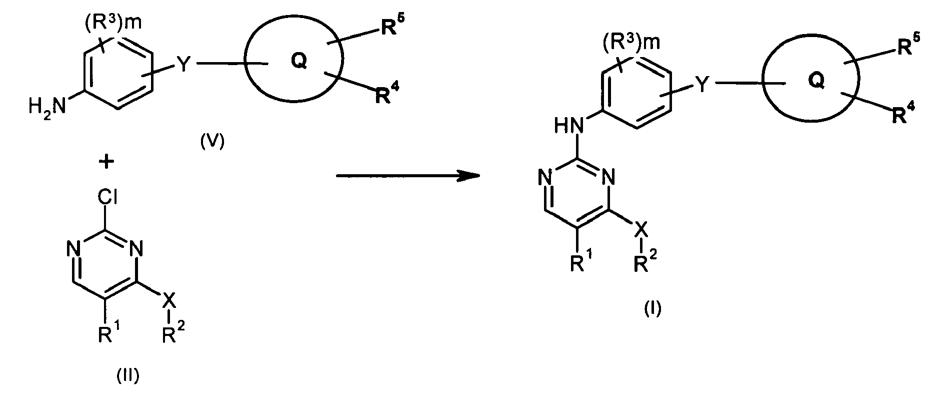
wobei die Substituenten Q, R¹, R², R³, R⁴, R⁵, X, Y und m die in der allgemeinen Formel (I) angegebenen Bedeutungen besitzen.

22. Verfahren zur Herstellung einer Verbindung gemäß einem der Ansprüche 1 bis 17, **gekennzeichnet durch** die Schritte:
a)2,4-Dichlor-pyrimidine der Formel (X) werden mit Nucleophilen der Formel (IX) zu Verbindungen der Formel (II) umgesetzt 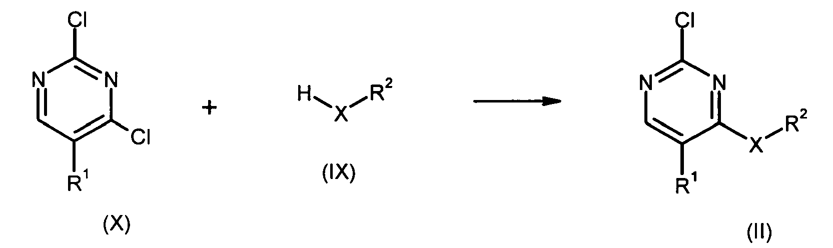
b₁) 2-Chlor-pyrimidine der Formel (II) werden mit Nucleophilen der Formel (IIId) zu Verbindungen der Formel (VI) umgesetzt werden. 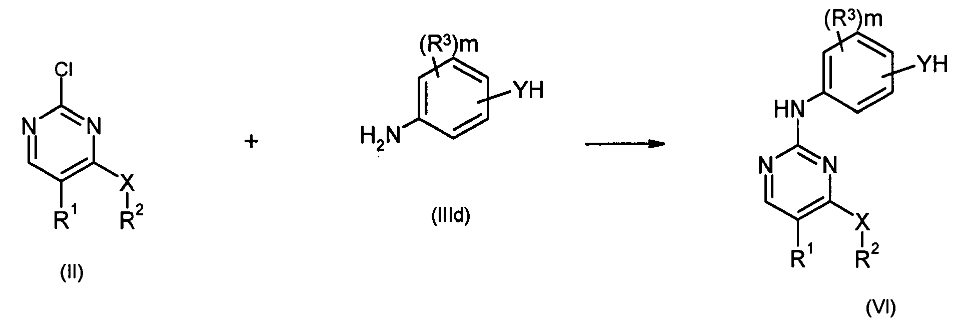
b₂) oder alternativ zu b₁) bei Verbindungen mit Y = NH werden 2-Chlor-Pyrimidine der Formel (II) mit Nitro-Anilinen der Formel (IIIc) zu Verbindungen (VIb) umgesetzt und anschließend die Nitrogruppe reduziert 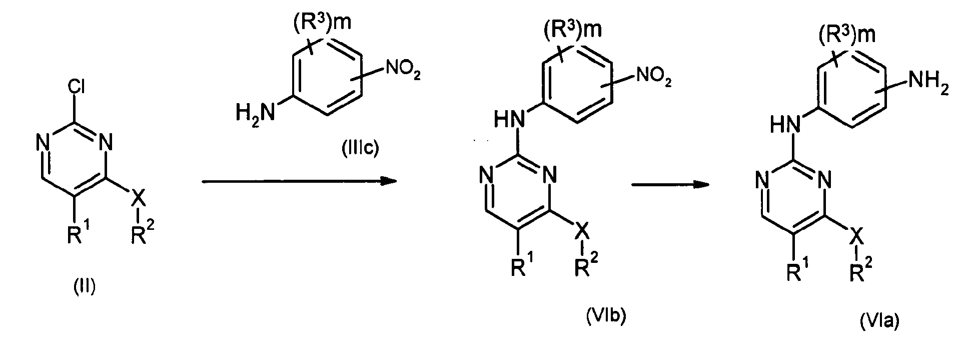
c) Substituierte Anilinopyrimdine der Formel (VI) oder (VIa) aus den Verfahrensschritten b₁ oder b₂ werden mit Elektrophilen der Formel (VII) zu Verbindungen der Formel (I) umgesetzt 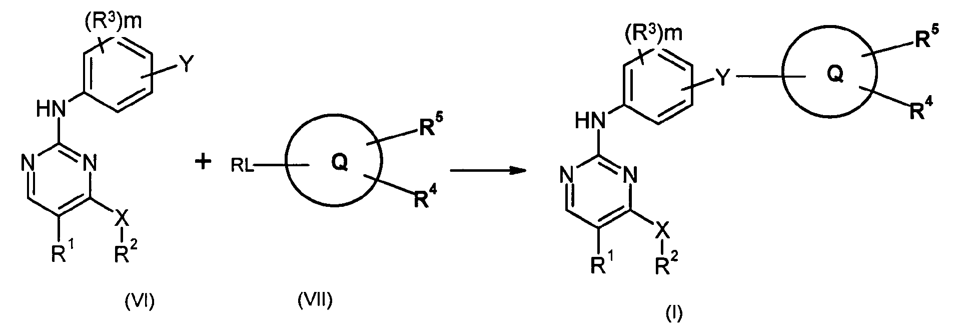
wobei die Substituenten Q, R¹, R², R³, R⁴, R⁵, X, Y und m die in der allgemeinen Formel (I) angegebenen Bedeutungen besitzen und RL für eine Fluchtgruppe steht.

23. Verfahren zur Herstellung einer Verbindung gemäß einem der Ansprüche 1 bis 17, **gekennzeichnet durch** die Schritte:
a₁) Anilinderivate der Formel III werden mit Elektrophilen der Formel VII umgesetzt 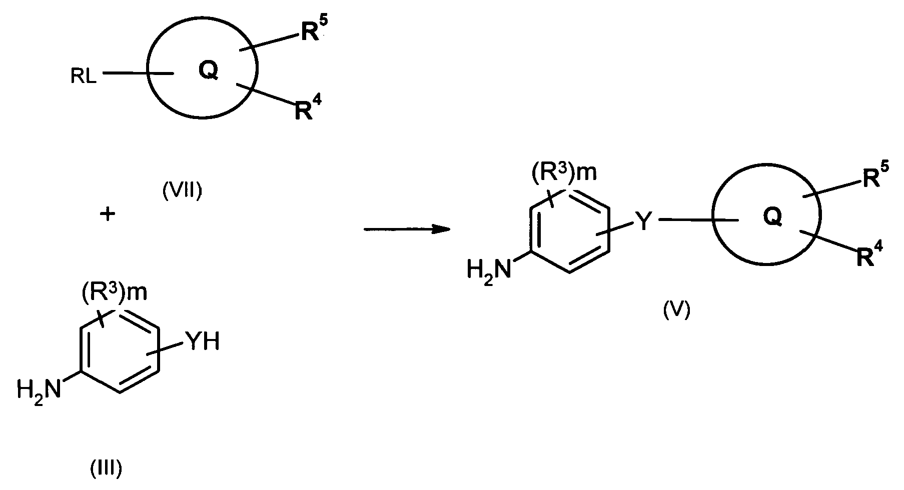 oder
a₂) oder alternativ werden Nitroaniline der Formel (IIIc) mit Elektrophilen der Formel (VII) umgesetzt und nachfolgend die Nitrogruppe reduziert, 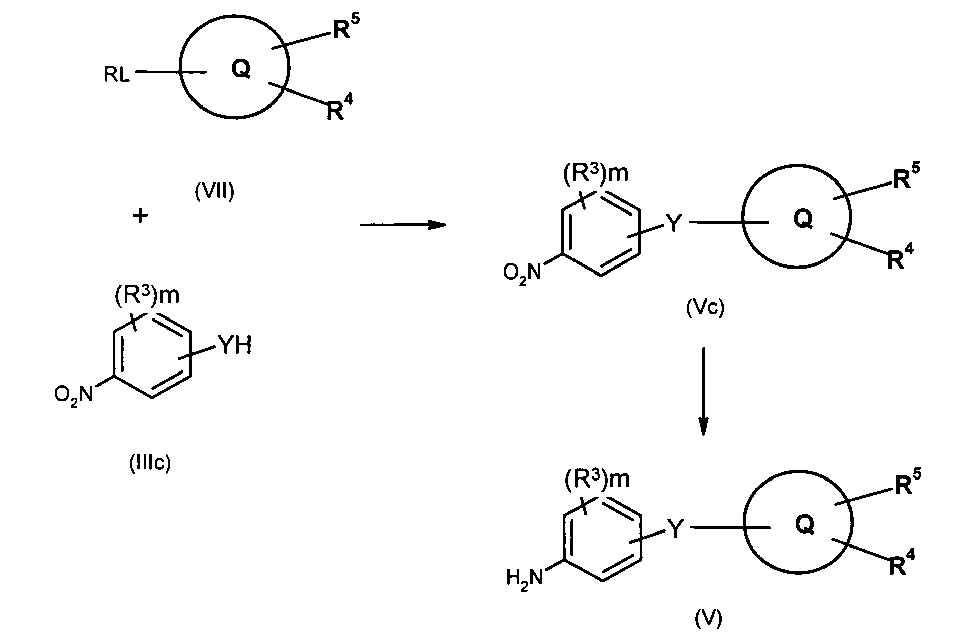
b) substituierte 2-Chlor-pyrimdine der Formel (XI) werden mit Nucleophilen der Formel (V) aus den Verfahrensschritten a₁ oder a₂ zu Verbindungen der Formel (VIII) umgesetzt werden. 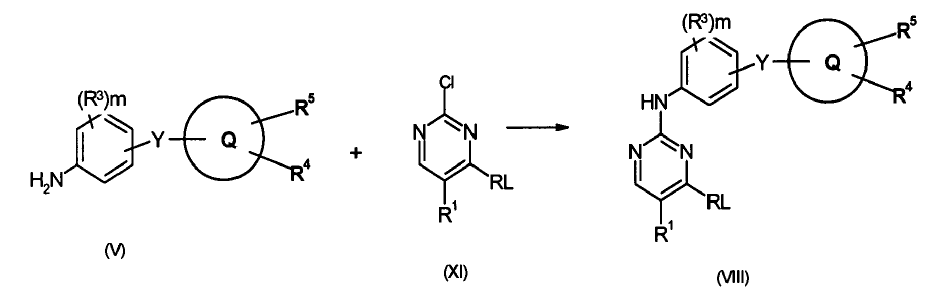
c) substituierte Anilinopyrimdine der Formel (VIII) werden mit Nucleophilen der Formel (IX) zu Verbindungen der Formel (I) umgesetzt werden. 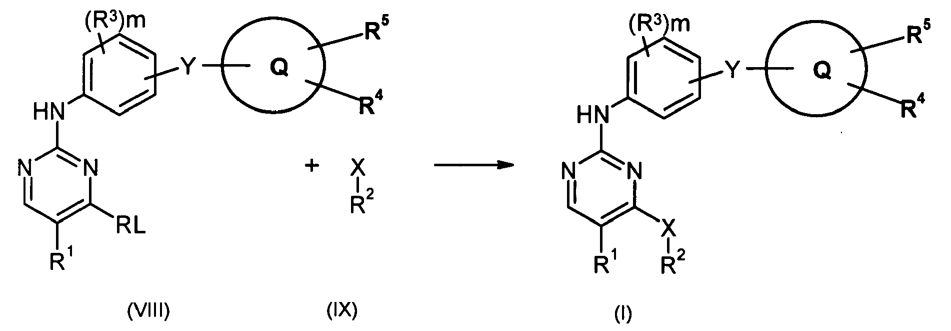
wobei die Substituenten Q, R¹, R², R³, R⁴, R⁵, X, Y und m die in der allgemeinen Formel (I) angegebenen Bedeutungen besitzen und RL für eine Fluchtgruppe steht.

24. Pharmazeutische Formulierung enthaltend eine oder mehrere Verbindungen gemäß einem der Ansprüche 1 bis 17.
